# EUROPEAN PATENT SPECIFICATION

(11) **EP 2 961 388 B1**
(45) Date of publication and mention of the grant of the patent: **24.04.2019**
(21) Application number: 14710728.8
(22) Date of filing: 27.02.2014
(51) Int. Cl.: A61K 9/19, A61K 45/06, A61K 31/675, A61K 31/69, A61P 35/00, A61P 37/02

(54) **DRUG COMBINATIONS**
ARZNEIMITTELKOMBINATIONEN
COMBINAISONS DE MÉDICAMENTS

(30) Priority: 01.03.2013 US 201361771525 P; 04.10.2013 US 201361887165 P
(43) Date of publication of application: 06.01.2016
(60) Divisional application: 19163282.7
(73) Proprietor: Astex Pharmaceuticals, Inc., Pleasanton, CA 94588 (US)
(72) Inventor: AZAB, Mohammad, Pleasanton, CA 94588 (US); TAVERNA, Pietro, Pleasanton, CA 94588 (US); COVRE, Alessia, I-33170 Pordenone (IT); CORAL, Sandra, I-33170 Pordenone (IT)
(74) Representative: D Young & Co LLP
(86) International application number: PCT/US2014/019137
(87) International publication number: WO 2014/134355

(56) References cited:
- US-A1- 2005 266 012
- US-B2- 7 700 567
- STEVEN J. O'DAY ET AL: "Targeting cytotoxic T-lymphocyte antigen-4 (CTLA-4)", CANCER, vol. 110, no. 12, 15 December 2007 (2007-12-15), pages 2614-2627, XP055097404, ISSN: 0008-543X, DOI: 10.1002/cncr.23086 cited in the application
- SANDRA CORAL ET AL: "Immunomodulatory activity of SGI-110, a 5-aza-2'-deoxycytidine-containing demethylating dinucleotide", CANCER IMMUNOLOGY, IMMUNOTHERAPY, vol. 62, no. 3, 9 November 2012 (2012-11-09), pages 605-614, XP055131495, ISSN: 0340-7004, DOI: 10.1007/s00262-012-1365-7 cited in the application
- H. K. KOBLISH ET AL: "Hydroxyamidine Inhibitors of Indoleamine-2,3-dioxygenase Potently Suppress Systemic Tryptophan Catabolism and the Growth of IDO-Expressing Tumors", MOLECULAR CANCER THERAPEUTICS, vol. 9, no. 2, 2 February 2010 (2010-02-02), pages 489-498, XP055131665, ISSN: 1535-7163, DOI: 10.1158/1535-7163.MCT-09-0628
- FOLKERT STEINHAGEN ET AL: "TLR-based immune adjuvants", VACCINE, ELSEVIER LTD, GB, vol. 29, no. 17, 14 August 2010 (2010-08-14), pages 3341-3355, XP028380413, ISSN: 0264-410X, DOI: 10.1016/J.VACCINE.2010.08.002 [retrieved on 2010-08-07] cited in the application

## Description

### BACKGROUND

Epigenetic modification of the genome, and in particular DNA methylation, plays a major role in human malignancies by influencing crucial cellular pathways in cancer initiation and progression (including cell cycle control, apoptosis, invasive and metastatic potential and angiogenesis). DNA methylation is mediated by the enzyme DNA methyltransferase, and results in the addition of a methyl group to a cytosine when the cytosine occurs in the context of a CpG dinucleotide.

DNA methylation of promoter-associated CpG islands results in silencing of the corresponding gene - in general, promoter-associated CpG islands are unmethylated in nonmalignant cells. Aberrant DNA hypermethylation in tumour cells is therefore a functional equivalent to inactivation of tumour suppressor genes by mutation, and so promotes tumour escape from host immune recognition *via* the down-regulation of various components of the tumour recognition complex in neoplastic cells (including HLA class I antigens, CTA antigens and accessory/co-stimulatory molecules). This results in a reduction in clinical efficacy of immunotherapeutic approaches for cancer treatment.

DNA hypomethylating agents (DHAs) induce global and gene-specific DNA hypomethylation. This promotes re-expression of tumour-associated antigens and thereby boosts immune recognition. Examples include 5-azacytidine, 5-aza-2'-deoxycytidine (decitabine) and Zebularine: 5-azacytidine and 5-aza-2'-deoxycytidine are currently approved by the US Food and Drug Administration for the treatment of patients with myelodysplastic syndromes, and decitabine is currently being developed as a pharmaceutical for the treatment of chronic myelogenous leukemia (CML), myelodysplastic syndrome (MDS), non-small cell lung cancer (NSCLC), sickle-cell anaemia and acute myelogenous leukemia (AML).

### SUMMARY OF THE INVENTION

In some embodiments, the invention provides a combination comprising the following separate components:
(i) a compound of Formula I or a pharmaceutically acceptable salt thereof:

   (5-azacytosine group)-L-(guanine group) (I)

   wherein L is a phosphorous-containing linker of Formula (II): 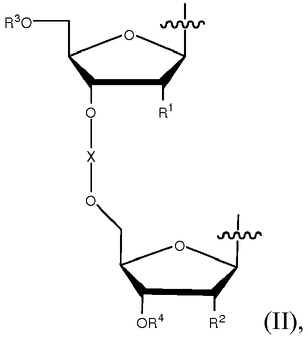 wherein, R¹ and R² are independently H, OH, an alkoxy group, an alkoxyalkoxy group, an acyloxy group, a carbonate group, a carbamate group, or a halogen; R³ is H, or R³ together with the oxygen atom to which R³ is bound forms an ether, an ester, a carbonate, or a carbamate; R⁴ is H, or R⁴ together with the oxygen atom to which R⁴ is bound forms an ether, an ester, a carbonate, or a carbamate; and X together with the oxygen atoms to which X is bound forms a phosphodiester, a phosphorothioate diester, a boranophosphate diester, or a methylphosphonate diester; and
(ii) one or more ancillary therapeutic component(s), wherein said ancillary therapeutic component is a T-cell activating agent;
   wherein said compound of Formula I, or pharmaceutically-acceptable salt thereof, is for administration before said ancillary therapeutic component.
In some embodiments, R¹ and R² are independently H, OH, OMe, OEt, OCH₂CH₂OMe, OBn, or F, and X together with the oxygen atoms to which X is bound form a phosphodiester. In some embodiments, R¹ and R² are H.

In some embodiments, the compound of Formula I is any one of I-(1-44). In some embodiments, the compound of Formula I is:

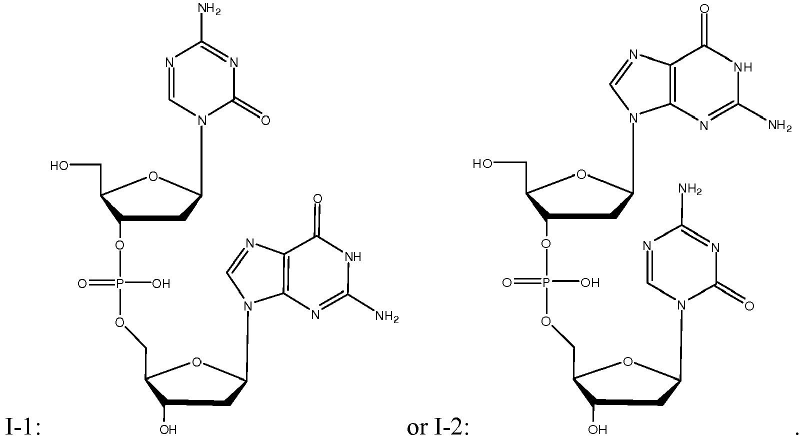

In some embodiments, the compound of formula I is of the formula: 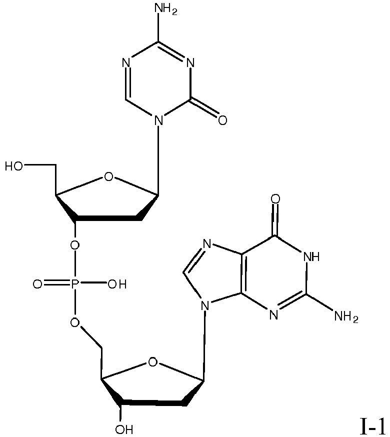 or a pharmaceutically-acceptable salt thereof. In some embodiments, the salt is a sodium salt.

The compound or salt thereof can be in the form of a formulation, for example being dissolved in a substantially anhydrous solvent comprising 45% to 85% propylene glycol; 5% to 45% glycerin; and 0% to 30% ethanol. In such embodiments, said solvent can comprise 65% to 70% propylene glycol; 25% to 30% glycerin, and 0% to 10% ethanol, for example: (a) 65% to 70% propylene glycol and 25% to 30% glycerin, any balance being ethanol; (b) 65% propylene glycol; 25% glycerin; and 10% ethanol; (c) 65% propylene glycol; 25% glycerin; and 10% ethanol; (d) 70% propylene glycol and 30% glycerin, ethanol being absent; (e) 45% to 85% propylene glycol; 5% to 45% glycerin; and 0% to 30% ethanol; (f) 65% to 70% propylene glycol; 25% to 30% glycerin, and 0% to 10% ethanol. The formulation can further comprise DMSO, optionally at a DMSO:compound ratio of 2:1; 1:1; 0.5:1; 0.3:1 or 0.2-0.3:1. The combination can be suitable for administration by subcutaneous injection.

When present as part of a formulation, the compound can be present at a concentration of 80 mg/mL to 110 mg/mL, optionally 100 mg/mL.

In some embodiments, the invention provides a kit comprising:
(a) a first vessel containing the compound or salt thereof as described herein;
(b) a second vessel containing a substantially anhydrous solvent as described herein; and
(c) one or more ancillary therapeutic component(s) as described herein.

The compound can be present in the kit in the form of a substantially anhydrous powder, for example being lyophilized. In some embodiments, the first vessel can contain 80 mg to 110 mg of said compound, for example 100 mg of said compound, and can further comprise instructions for administration by subcutaneous injection.

Also described herein is a process for preparing a pharmaceutical composition, the process comprising dissolving a compound or salt thereof as defined above in a substantially anhydrous solvent as also defined above, and then combining the dissolved compound with one or more ancillary therapeutic component(s) as also defined above. Also described herein is a process which further comprises the preliminary steps of:
(a) dissolving said compound in DMSO to produce a solution of said compound in DMSO; and
(b) lyophilizing said solution of step (a) to provide said compound as a substantially anhydrous powder.

Also described herein is a process for producing a pharmaceutical composition comprising a compound or salt thereof as defined above in the form of a substantially anhydrous powder, the process comprising dissolving said compound in DMSO to produce a solution in DMSO, lyophilizing said solution to provide said compound as a substantially anhydrous powder and then combining the powder with one or more ancillary therapeutic component(s). Described herein, said substantially anhydrous powder comprises residual DMSO, for example: (a) present in an amount of ≤2000, or about 0.1 to about 2000 mg/g of said compound; or (b) present in an amount of ≤1000, or about 0.1 to about 1000 mg/g; ≤600, or about 0.1 to about 600mg/g; ≤500, or about 0.1 to about 500 mg/g; ≤400, or about 0.1 to about 400 mg/g; ≤300, or about 0.1 to about 300 mg/g; or about 200 - about 300mg/g of said compound; or (c) present in an amount of 200-300 mg/g of said compound.

Also described herein is a substantially anhydrous powder consisting essentially of a compound or salt thereof as defined above and DMSO, the DMSO being present in an amount of ≤200, or about 0.1% to about 200% w/w, in combination with one or more ancillary therapeutic component(s) as defined above. Described herein, the DMSO is present in an amount of ≤100%, or about 0.1% to about 100%, ≤60%, or about 0.1% to about 60%, ≤50%, or about 0.1% to about 50%, ≤40%, or about 0.1% to about 40%, or ≤30%, or about 0.1% to about 30% w/w DMSO/compound, for example in an amount of about 20 - about 30% w/w DMSO/compound.

Also described is a pharmaceutical composition obtainable by, or obtained by, the processes of the invention.

In some embodiments, the ancillary therapeutic component comprises a T-cell activating agent.

In some embodiments, the ancillary therapeutic component comprises a cancer vaccine.

In some embodiments, the ancillary therapeutic component comprises an adjuvant.

In some embodiments, the ancillary therapeutic component comprises a T-cell activating agent and a cancer vaccine.

In some embodiments, the ancillary therapeutic component comprises a T-cell activating agent, for example being selected from agonists or antibodies for: ICOS, GITR, MHC, CD80, CD86, Galectin 9 and LAG-3.

In other embodiments, the T-cell activating agent is an antibody, for example being selected from: (a) a CD137 agonist; (b) a CD40 agonist; (c) an OX40 agonist; (d) a PD-1 mAb; (e) a PD-L1 mAb; (f) a PD-L2 mAb; (g) a CTLA-4 mAb; and (h) combinations of (a)-(g).

In some embodiments, the ancillary therapeutic component is Tremelimumab or Ipilimumab.

In some embodiments, the ancillary therapeutic component comprises a CTA cancer vaccine, for example being based on a CTA antigen selected from: NY-ESO-1, LAGE-1, MAGE-A1, -A2, -A3, -A4, -A6, -A10, -A12, CT7, CT10, GAGE1-6, GAGE 1-2, BAGE, SSX1-5, SSX 2, HAGE, PRAME, RAGE-1, XAGE-1, MUC2, MUC5B, B7.1/2, CD28, B7-H1, HLA, CD40L and HMW-MAA, for example based on MAGE-A3 (for example recMAGE-A3), NY-ESO-1 and PRAME.

In some embodiments, the ancillary therapeutic component comprises an IDO inhibitor, for example selected from INCB24360, 1 methyl tryptophan and NLG919.

In some embodiments, the invention provides a combination or kit as described herein for use in treating a disease selected from:
(a) a myelodysplastic syndrome (MDS);
(b) a cancer;
(c) a haematological disorder; or
(d) a disease associated with abnormal haemoglobin synthesis,
wherein the compound of Formula I or salt thereof is administered first (as a priming therapy), followed by administration of the ancillary therapeutic component(s).

The MDS can be selected from low-, intermediate- and high-risk MDS and myloproliferative neoplasms.

The haematological disorder can be leukemia, for example, selected from: acute myeloid leukemia (AML), acute promyelocyte leukemia, acute lymphoblastic leukemia, and chronic myelogenous leukemia. In some embodiments, the AML can be selected from elderly AML, first relapse AML and second relapse AML.

The cancer can be selected from breast cancer, skin cancer, bone cancer, prostate cancer, liver cancer, lung cancer, non-small cell lung cancer, squamous non-small cell lung adenocarcinoma, brain cancer, cancer of the larynx, gall bladder, pancreas, rectum, parathyroid, thyroid, adrenal, neural tissue, head and neck, colon, stomach, bronchi, and kidney cancer, basal cell carcinoma, squamous cell carcinoma of both ulcerating and papillary type, metastatic skin carcinoma, osteo sarcoma, Ewing's sarcoma, reticulum cell sarcoma, myeloma, giant cell tumour, small-cell lung tumour, islet cell tumour, primary brain tumour, acute and chronic lymphocytic and granulocytic tumours, hairy-cell tumour, adenoma, hyperplasia, medullary carcinoma, pheochromocytoma, mucosal neuroma, intestinal ganglioneuromas, hyperplastic corneal nerve tumour, marfanoid habitus tumour, Wilm's tumour, seminoma, ovarian tumour, platinum resistant ovarian cancer, leiomyomater tumour, cervical dysplasia and in situ carcinoma, neuroblastoma, retinoblastoma, soft tissue sarcoma, malignant carcinoid, mycosis fungoide, rhabdomyosarcoma, Kaposi's sarcoma, osteogenic sarcoma, malignant hypercalcemia, renal cell tumour, polycythemia vera, adenocarcinoma, glioblastoma multiforma, leukemia, lymphoma, melanoma, epidermoid carcinoma, hepatocellular carcinoma and a solid tumour.

In some embodiments, the cancer is selected from pancreatic cancer, ovarian cancer, melanoma and lung cancer.

In some embodiments, the disease associated with abnormal haemoglobin synthesis is selected from sickle cell anaemia and β-thalassemia.

In some embodiments, the invention provides the combination, kit, process, powder or composition as defined in the claims appended hereto or as described herein for use in therapy or prophylaxis, for example for use in immunotherapy or for treating a disease as defined in claims appended hereto and described above or herein.

In some embodiments, the invention provides the use of the combination, kit, process, powder or composition as defined in the claims appended hereto or as described herein for the manufacture of a medicament for use in immunotherapy or in a method of treating a disease in claims appended hereto and described above or herein.

The combination, kit, process, powder or composition of the invention can be administered to a subject according to a dosage regimen of: (a) once, twice, three times, four times, five times, six times or seven times a week; or (b) every day for 5, 6, 7, 8, 9 or 10 days; or (c) every day for up to 10 days; or (d) every day for between 5 and 10 days; or (e) every day for 5 days, immediately followed by two dose-free days and then every day for the next 5 days. Administration can be subcutaneous.

### BRIEF DESCRIPTION OF THE DRAWINGS

**FIGURE 1** illustrates the mean plasma concentrations of the compound I-1 in male and female cynomolgus monkeys given weekly subcutaneous doses of compound I-1 in a pahrmacokinetic study.
**FIGURE 2** illustrates the mean plasma concentrations of decitabine in male and female cynomolgus monkeys given weekly subcutaneous doses of decitabine in a pharmacokinetic study.
**FIGURE 3** illustrates the decrease in LINE1 methylation levels observed in blood samples drawn from cynomolgus monkeys on various days (D) after pretest.
**FIGURE 4** illustrates the change in total related substances of the sodium salt of a compound of Formula I-1 in various DMSO and DMSO/water compositions.
**FIGURE 5** illustrates the anti-tumor effect of SGI-110 in combination with anti-mouse CTLA-4.
**FIGURE 6** illustrates the anti-tumor effect of two cycles of sequential administration of SGI-110 followed by anti-mouse CTLA-4 mAb 9H10.

### DETAILED DESCRIPTION OF THE INVENTION

The combinations of the present invention activate the expression of, or strongly up-regulate constitutive levels of expression of, components of the tumour recognition complex in neoplastic cells of diverse histotypes. They can therefore be used as immunomodulatory agents to increase immunogenicity and immune recognition of neoplastic cells. This, in turn, should allow for better therapeutic outcomes in terms of tumor control and regression, prolong disease-free progression, and improve overall survival.

Second generation DHAs derived from decitabine, including the DNA hypomethylating agent of compound I-1 (a dinucleotide of 5-aza-2'-deoxycytidine and deoxyguanosine), are described in WO2007/041071.

### Compounds of Formula I for use in the combinations of the invention

In some embodiments, the invention provides combinations comprising a compound of Formula I or a pharmaceutically-acceptable salt thereof:

(5-azacytosine group)-L-(guanine group) (I),

wherein L is a phosphorus-containing linker of Formula II. 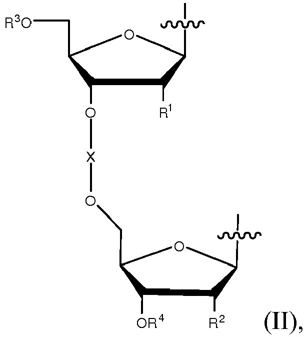 wherein, R¹ and R² are independently H, OH, an alkoxy group, an alkoxyalkoxy group, an acyloxy group, a carbonate group, a carbamate group, or a halogen; R³ is H, or R³ together with the oxygen atom to which R³ is bound forms an ether, an ester, a carbonate, or a carbamate; R⁴ is H, or R⁴ together with the oxygen atom to which R⁴ is bound forms an ether, an ester, a carbonate, or a carbamate; and X together with the oxygen atoms to which X is bound forms a phosphodiester, a phosphorothioate diester, a boranophosphate diester, or a methylphosphonate diester.

The 5-azacytosine group can be linked to either end of L, and the guanine group can be linked to the other end of L as long as the compound contains one 5-azacytosine group and one guanine group. Constitutional isomers can thus be prepared by exchanging the connectivity of the 5-azacytosine group and the guanine group.

R¹ and R² can be the same or different. In some embodiments, R¹ and R² are independently H, OH, OMe, OEt, OPh, OCH₂CH₂OMe, OCH₂CH₂OEt, OCH₂CH₂OBn,OBn, OAc, OBz, OCOOMe, OCOOEt, OCOOBn, OCONH₂, OCONMe₂, OCONEt₂, OCONBn₂, OCONHMe, OCONHEt, OCONHBn, F, Cl, Br, or I. In some embodiments, R¹ and R² are independently H, OH, OMe, OEt, OCH₂CH₂OMe, OBn, or F. In some embodiments, R¹ and R² are independently H or OH. In some embodiments, R¹ and R² are H. In some embodiments, R¹ and R² are OH.

R³ and R⁴ can be the same or different.

In some embodiments, R³ is H, or R³ together with the oxygen atom to which R³ is bound forms OH, OMe, OEt, OPh, OCH₂CH₂OMe, OCH₂CH₂OEt, OCH₂CH₂OBn,OBn, OAc, OBz, OCOOMe, OCOOEt, OCOOBn, OCONH₂, OCONMe₂, OCONEt₂, OCONBn₂, OCONHMe, OCONHEt, or OCONHBn. In some embodiments, R³ is H, or R³ together with the oxygen atom to which R³ is bound forms OH, OMe, OEt, OCH₂CH₂OMe, or OBn. In some embodiments, R³ is H.

In some embodiments, R⁴ is H, or R⁴ together with the oxygen atom to which R⁴ is bound forms OH, OMe, OEt, OPh, OCH₂CH₂OMe, OCH₂CH₂OEt, OCH₂CH₂OBn,OBn, OAc, OBz, OCOOMe, OCOOEt, OCOOBn, OCONH₂, OCONMe₂, OCONEt₂, OCONBn₂, OCONHMe, OCONHEt, or OCONHBn. In some embodiments, R⁴ is H, or R⁴ together with the oxygen atom to which R⁴ is bound forms OH, OMe, OEt, OCH₂CH₂OMe, or OBn. In some embodiments, R⁴ is H.

In some embodiments, X is P(O)OH, P(O)SH, P(→O)BH₃⁻, or P(O)Me. In some embodiments, X is P(O)OH. In some embodiments, X together with the oxygen atoms to which X is bound forms a phosphodiester.

Non-limiting examples of alkyl include straight, branched, and cyclic alkyl groups. Non-limiting examples of straight alkyl groups include methyl, ethyl, propyl, butyl, pentyl, hexyl, heptyl, octyl, nonyl, and decyl.

Branched alkyl groups include any straight alkyl group substituted with any number of alkyl groups. Non-limiting examples of branched alkyl groups include isopropyl, isobutyl, sec-butyl, and t-butyl.

Non-limiting examples of cyclic alkyl groups include cyclopropyl, cyclobutyl, cyclopentyl, cyclohexyl, cycloheptlyl, and cyclooctyl groups. Cyclic alkyl groups also include fused-, bridged-, and spiro-bicycles and higher fused-, bridged-, and spiro-systems. A cyclic alkyl group can be substituted with any number of straight or branched alkyl groups.

A halo-alkyl group can be any alkyl group substituted with any number of halogen atoms, for example, fluorine, chlorine, bromine, and iodine atoms.

An alkoxy group can be, for example, an oxygen atom substituted with any alkyl group. An ether or an ether group comprises an alkoxy group. Non-limiting examples of alkoxy groups include methoxy, ethoxy, propoxy, isopropoxy, and isobutoxy.

An alkoxyalkoxy group can be, for example, an alkoxy group substituted at any position with any alkoxy group. Non-limiting examples of alkoxyalkoxy groups include methoxyethoxy, ethyoxyethoxy, ethoxyethoxyethoxy, groups derived from any order of glyme, and groups derived from polyethylene glycol.

An aryl group can be heterocyclic or non-heterocyclic. An aryl group can be monocyclic or polycyclic. An aryl group can be substituted with any number of hydrocarbyl groups, alkyl groups, and halogen atoms. Non-limiting examples of aryl groups include phenyl, toluyl, naphthyl, pyrrolyl, pyridyl, imidazolyl, thiophenyl, and furyl.

An aryloxy group can be, for example, an oxygen atom substituted with any aryl group, such as phenoxy.

An aralkyl group can be, for example, any alkyl group substituted with any aryl group, such as benzyl.

An arylalkoxy group can be, for example, an oxygen atom substituted with any aralkyl group, such as benzyloxy.

A heterocycle can be any ring containing a ring atom that is not carbon. A heterocycle can be substituted with any number of alkyl groups and halogen atoms. Non-limiting examples of heterocycles include pyrrole, pyrrolidine, pyridine, piperidine, succinamide, maleimide, morpholine, imidazole, thiophene, furan, tetrahydrofuran, pyran, and tetrahydropyran.

An acyl group can be, for example, a carbonyl group substituted with hydrocarbyl, alkyl, hydrocarbyloxy, alkoxy, aryl, aryloxy, aralkyl, arylalkoxy, or a heterocycle. Non-limiting examples of acyl include acetyl, benzoyl, benzyloxycarbonyl, phenoxycarbonyl, methoxycarbonyl, and ethoxycarbonyl.

An acyloxy group can be an oxygen atom substituted with an acyl group. An ester or an ester group comprises an acyloxy group.

A carbonate group can be an oxygen atom substituted with hydrocarbyloxycarbonyl, alkoxycarbonyl, aryloxycarbonyl, or arylalkoxycarbonyl.

A carbamate group can be an oxygen atom substituted with a carbamoyl group, wherein the nitrogen atom of the carbamoyl group is unsubstituted, monosubstituted, or disubstituted with one or more of hydrocarbyl, alkyl, aryl, heterocyclyl, or aralkyl. When the nitrogen atom is disubstituted, the two substituents together with the nitrogen atom can form a heterocycle.

Any functional group of a compound described herein can be optionally capped with a capping group. For examples of capping groups, see GREENE'S PROTECTIVE GROUPS IN ORGANIC SYNTHESIS, 4th Ed. (Wiley 2006) (1980) and PROTECTING GROUPS, 3d Ed. (Thieme 2005) (1994).

Non-limiting examples of suitable capping groups for a hydroxyl group include alkyl, haloalkyl, aryl, aralkyl, carbonate, carbamate, and acyl groups.

Non-limiting examples of suitable capping groups for nitrogen-functionalities include alkyl, aryl, aralkyl, an acyl group, an alkoxycarbonyl group, an aryloxycarbonyl group, and an aminocarbonyl group. A capping group together with the nitrogen atom to which the capping group is bound can form, for example, an amide, a carbamate, a urethane, a heterocycle, or an amine. Two capping groups bound to the same nitrogen atom can form together with the nitrogen atom a heterocycle.

The invention provides pharmaceutically-acceptable salts of any compound described herein. Pharmaceutically-acceptable salts include, for example, acid-addition salts and base-addition salts. The acid that is added to a compound to form an acid-addition salt can be an organic acid or an inorganic acid. A base that is added to a compound to form a base-addition salt can be an organic base or an inorganic base. In some embodiments, a pharmaceutically-acceptable salt is a metal salt. In some embodiments, a pharmaceutically-acceptable salt is an ammonium salt.

Acid addition salts can arise from the addition of an acid to a compound described herein. In some embodiments, the acid is organic. In some embodiments, the acid is inorganic. Non-limiting examples of suitable acids include hydrochloric acid, hydrobromic acid, hydroiodic acid, nitric acid, nitrous acid, sulfuric acid, sulfurous acid, a phosphoric acid, nicotinic acid, isonicotinic acid, lactic acid, salicylic acid, 4-aminosalicylic acid, tartaric acid, ascorbic acid, gentisinic acid, gluconic acid, glucaronic acid, saccaric acid, formic acid, benzoic acid, glutamic acid, pantothenic acid, acetic acid, propionic acid, butyric acid, fumaric acid, succinic acid, citric acid, oxalic acid, maleic acid, hydroxymaleic acid, methylmaleic acid, glycolic acid, malic acid, cinnamic acid, mandelic acid, 2-phenoxybenzoic acid, 2-acetoxybenzoic acid, embonic acid, phenylacetic acid, N-cyclohexylsulfamic acid, methanesulfonic acid, ethanesulfonic acid, benzenesulfonic acid, p-toluenesulfonic acid, 2-hydroxyethanesulfonic acid, ethane-1,2-disulfonic acid, 4-methylbenzenesulfonic acid, naphthalene-2-sulfonic acid, naphthalene-1,5-disulfonic acid, 2-phosphoglyceric acid, 3-phosphoglyceric acid, glucose-6-phosphoric acid, and an amino acid.

Non-limiting examples of suitable acid addition salts include a hydrochloride salt, a hydrobromide salt, a hydroiodide salt, a nitrate salt, a nitrite salt, a sulfate salt, a sulfite salt, a phosphate salt, a hydrogen phosphate salt, a dihydrogen phosphate salt, a carbonate salt, a bicarbonate salt, a nicotinate salt, an isonicotinate salt, a lactate salt, a salicylate salt, a 4-aminosalicylate salt, a tartrate salt, an ascorbate salt, a gentisinate salt, a gluconate salt, a glucaronate salt, a saccarate salt, a formate salt, a benzoate salt, a glutamate salt, a pantothenate salt, an acetate salt, a propionate salt, a butyrate salt, a fumarate salt, a succinate salt, a citrate salt, an oxalate salt, a maleate salt, a hydroxymaleate salt, a methylmaleate salt, a glycolate salt, a malate salt, a cinnamate salt, a mandelate salt, a 2-phenoxybenzoate salt, a 2-acetoxybenzoate salt, an embonate salt, a phenylacetate salt, an N-cyclohexylsulfamate salt, a methanesulfonate salt, an ethanesulfonate salt, a benzenesulfonate salt, a p-toluenesulfonate salt, a 2-hydroxyethanesulfonate salt, an ethane-1,2-disulfonate salt, a 4-methylbenzenesulfonate salt, a naphthalene-2-sulfonate salt, a naphthalene-1,5-disulfonate salt, a 2-phosphoglycerate salt, a 3-phosphoglycerate salt, a glucose-6-phosphate salt, and an amino acid salt.

Metal salts can arise from the addition of an inorganic base to a compound described herein. The inorganic base consists of a metal cation paired with a basic counterion, such as, for example, hydroxide, carbonate, bicarbonate, or phosphate. The metal can be an alkali metal, alkaline earth metal, transition metal, or main group metal. Non-limiting examples of suitable metals include lithium, sodium, potassium, caesium, cerium, magnesium, manganese, iron, calcium, strontium, cobalt, titanium, aluminium, copper, cadmium, and zinc.

Non-limiting examples of suitable metal salts include a lithium salt, a sodium salt, a potassium salt, a caesium salt, a cerium salt, a magnesium salt, a manganese salt, an iron salt, a calcium salt, a strontium salt, a cobalt salt, a titanium salt, a aluminium salt, a copper salt, a cadmium salt, and a zinc salt.

Ammonium salts can arise from the addition of ammonia or an organic amine to a compound described herein. Non-limiting examples of suitable organic amines include triethyl amine, diisopropyl amine, ethanol amine, diethanol amine, triethanol amine, morpholine, N-methylmorpholine, piperidine, N-methylpiperidine, N-ethylpiperidine, dibenzyl amine, piperazine, pyridine, pyrrazole, pipyrrazole, imidazole, pyrazine, pipyrazine, ethylenediamine, N,N'-dibenzylethylene diamine, procaine, chloroprocaine, choline, dicyclohexyl amine, and N-methylglucamine.

Non-limiting examples of suitable ammonium salts include is a triethyl amine salt, a diisopropyl amine salt, an ethanol amine salt, a diethanol amine salt, a triethanol amine salt, a morpholine salt, an N-methylmorpholine salt, a piperidine salt, an N-methylpiperidine salt, an N-ethylpiperidine salt, a dibenzyl amine salt, a piperazine salt, a pyridine salt, a pyrrazole salt, a pipyrrazole salt, an imidazole salt, a pyrazine salt, a pipyrazine salt, an ethylene diamine salt, an N,N'-dibenzylethylene diamine salt, a procaine salt, a chloroprocaine salt, a choline salt, a dicyclohexyl amine salt, and a N-methylglucamine salt.

Non-limiting examples of compounds of Formula I include: 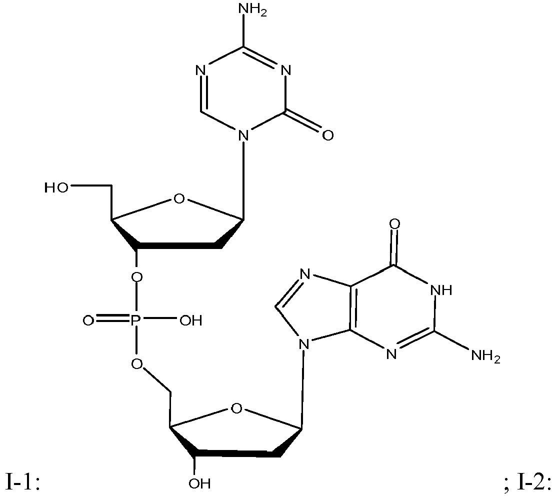 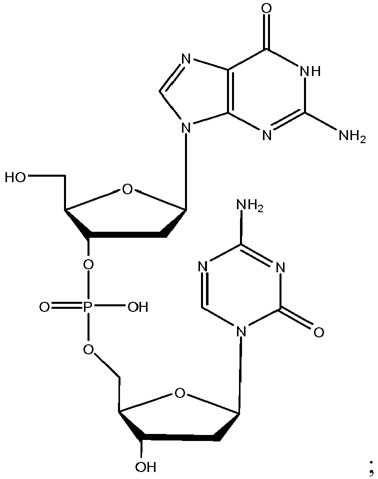 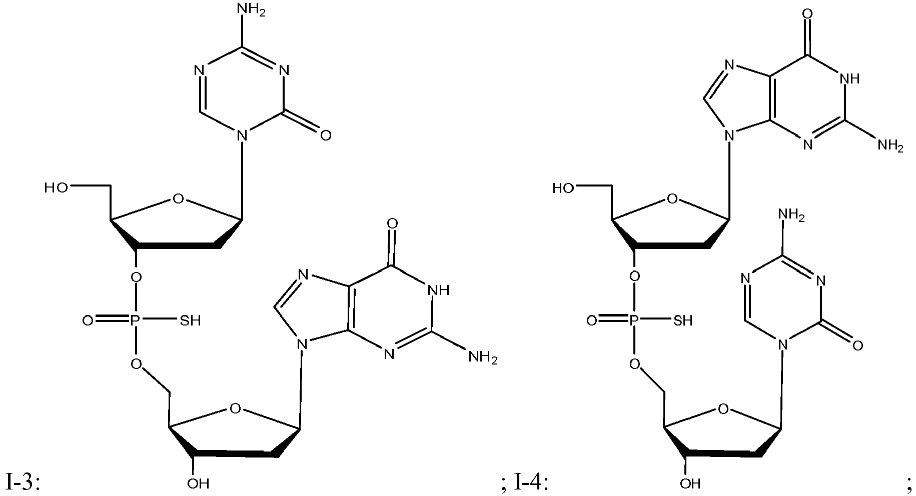 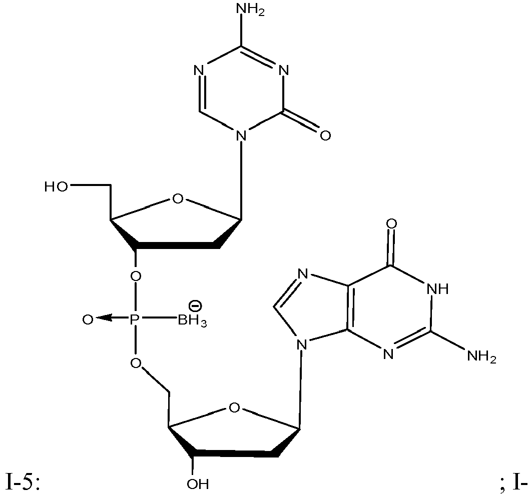 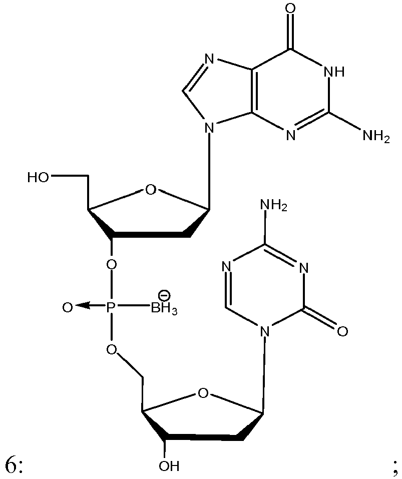 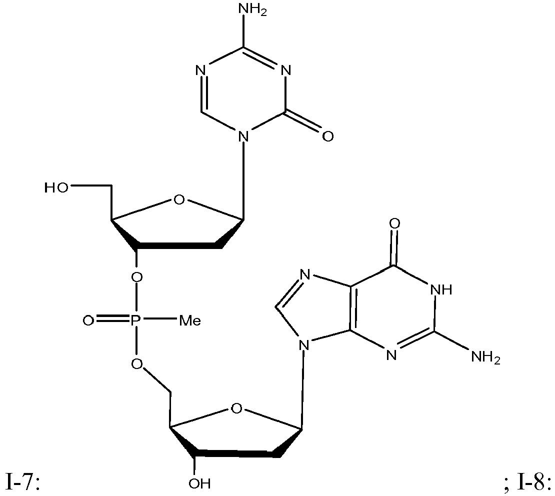 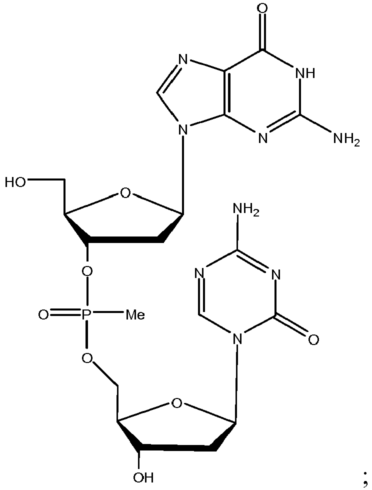 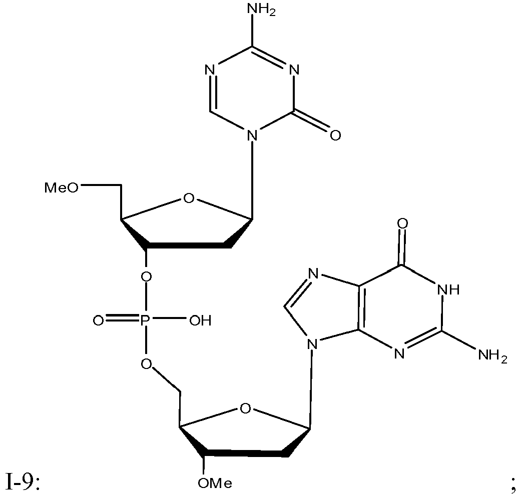 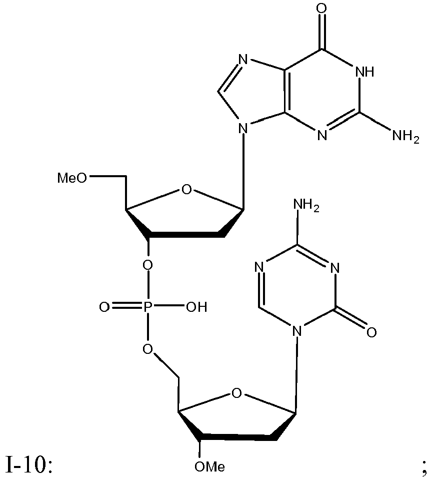 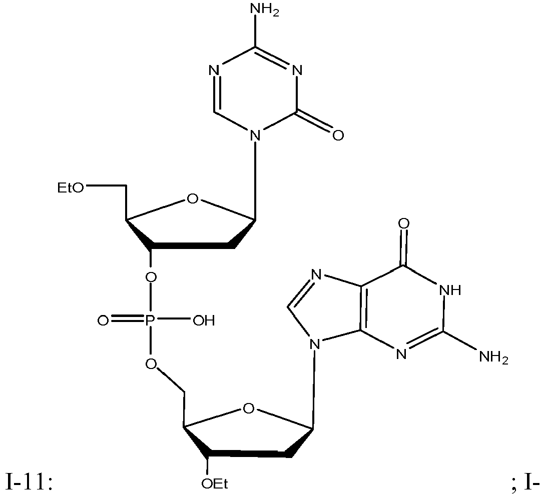 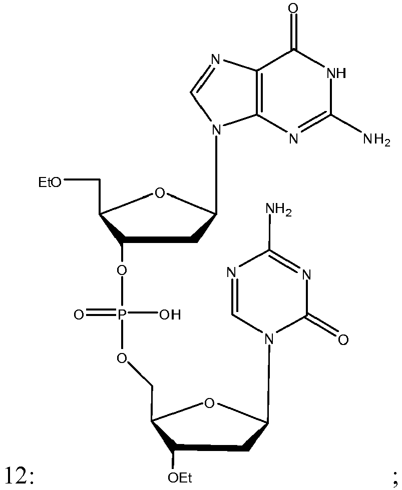 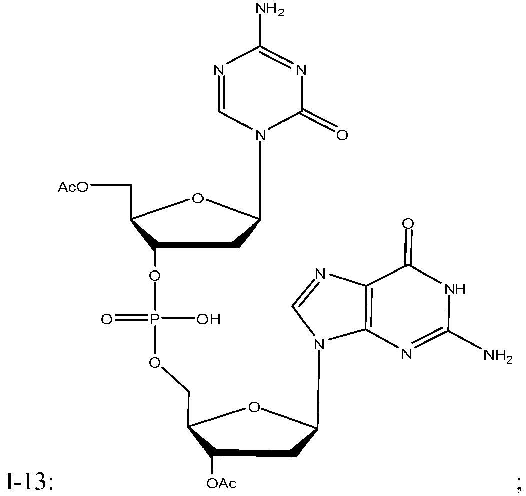 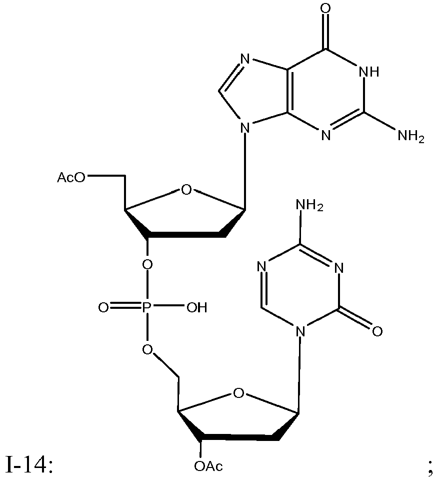 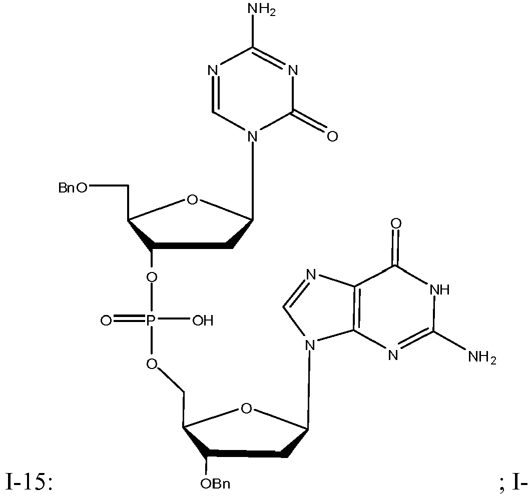 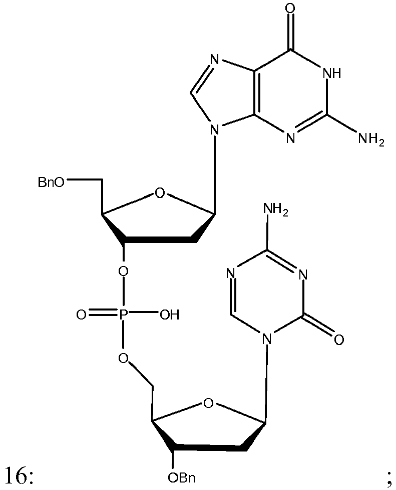 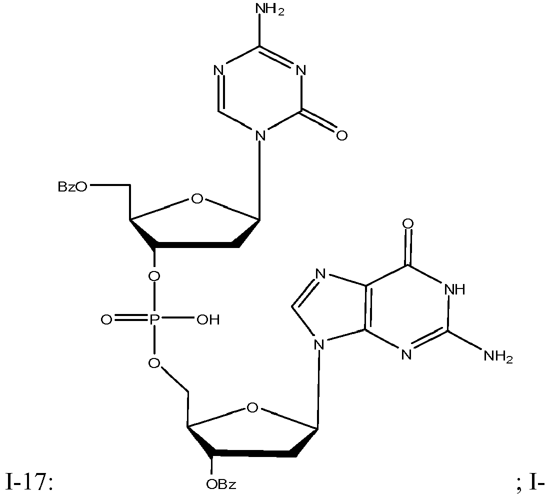 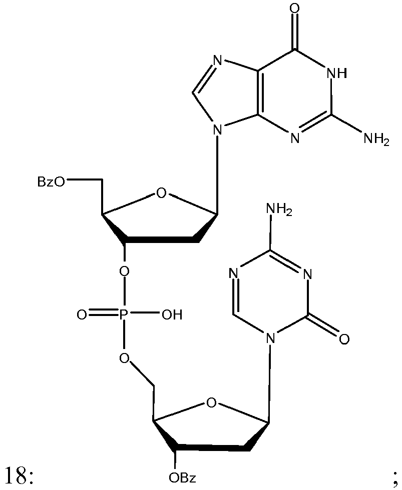 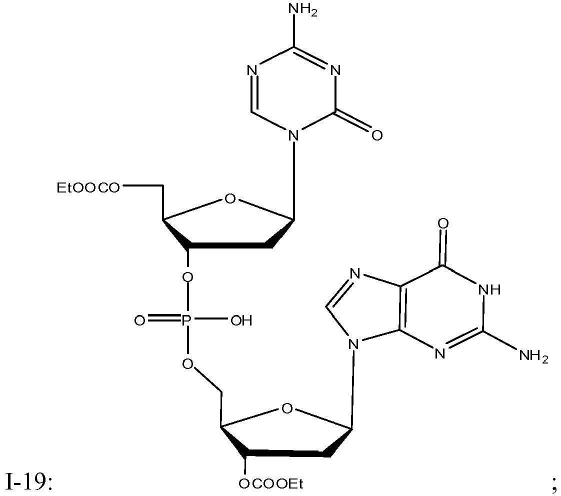 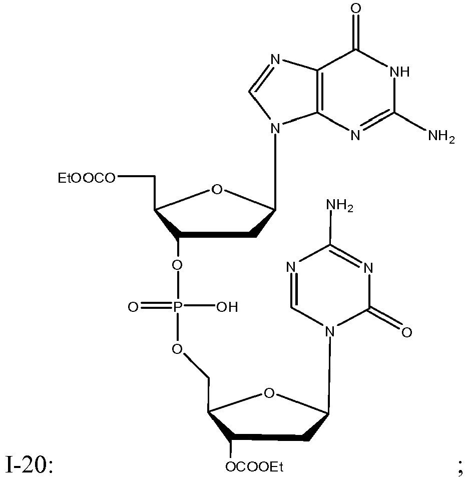 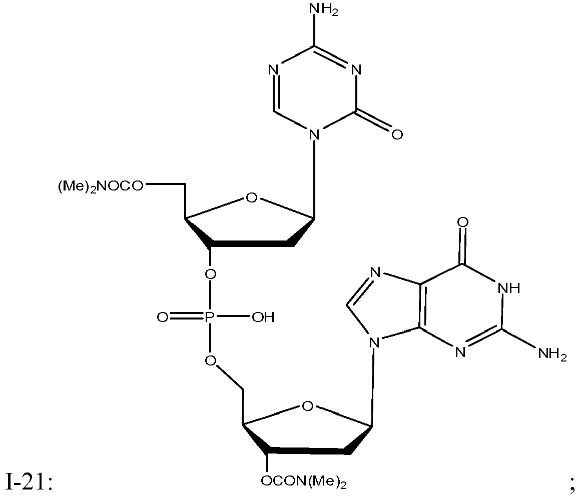 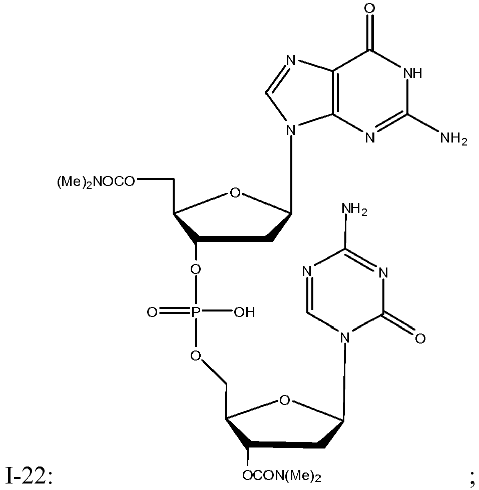 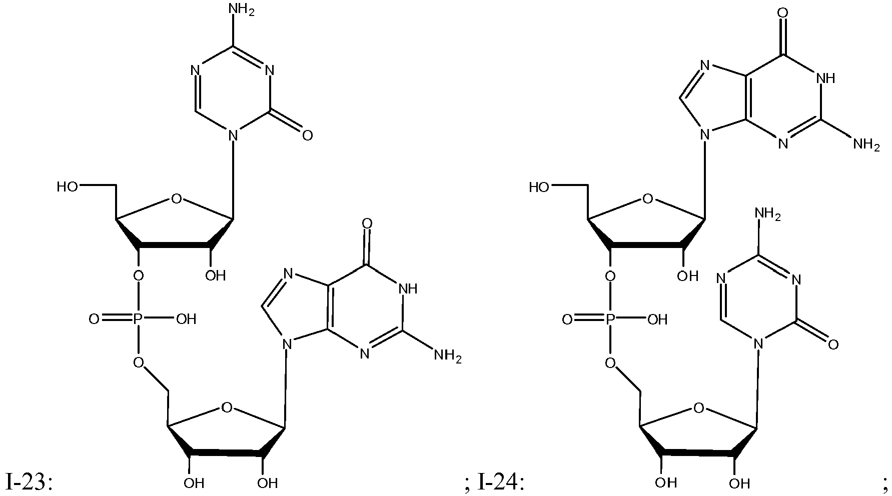 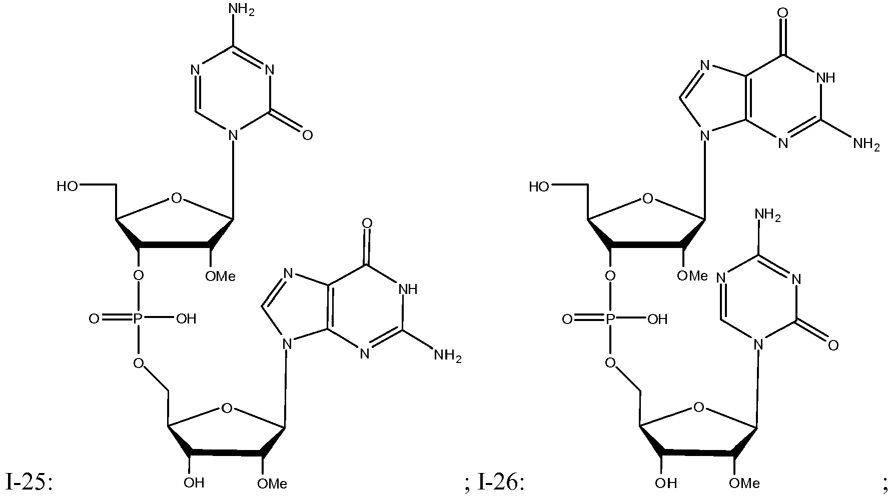 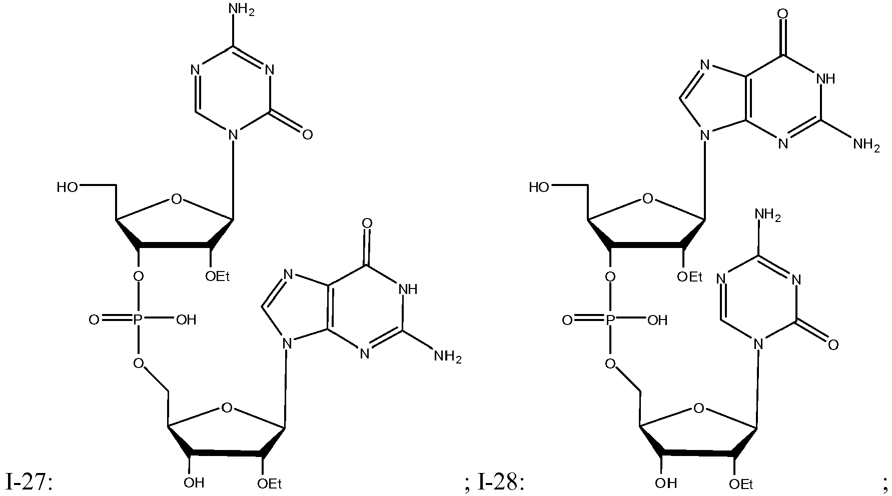 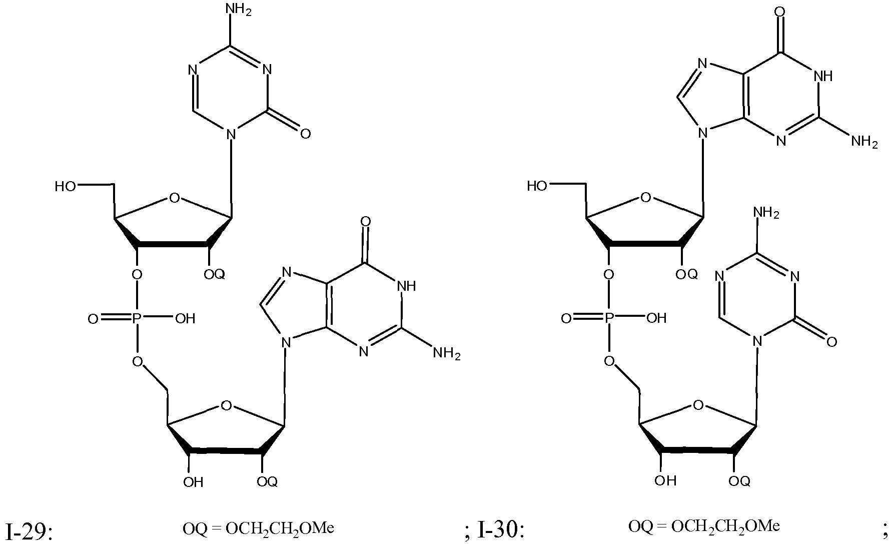 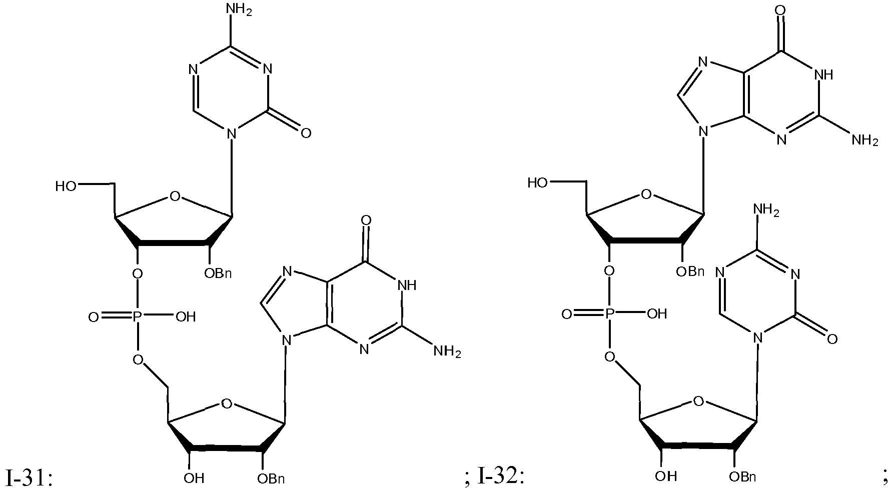 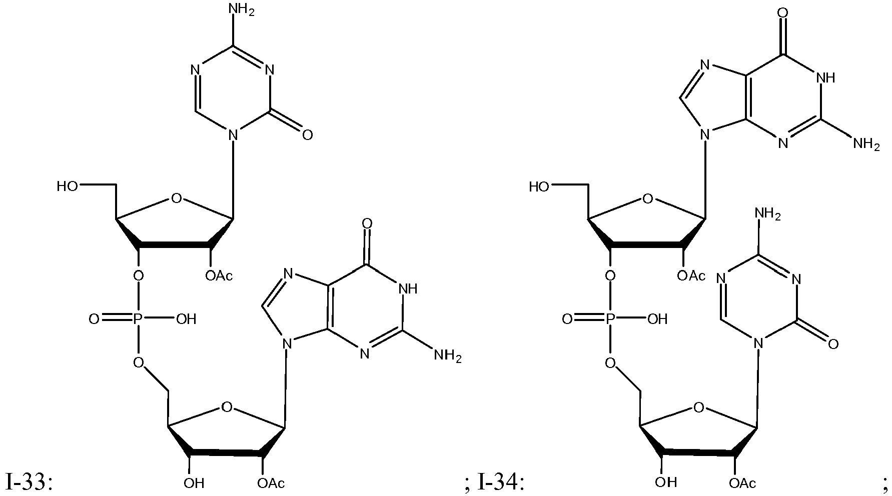 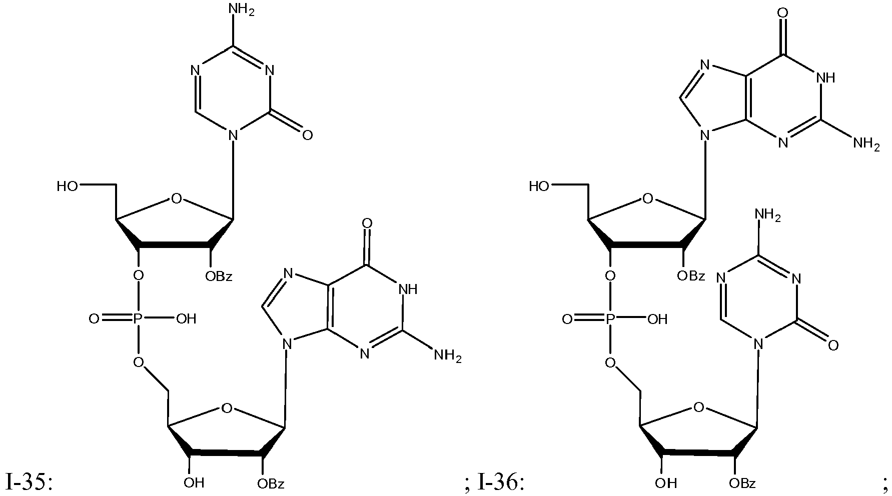 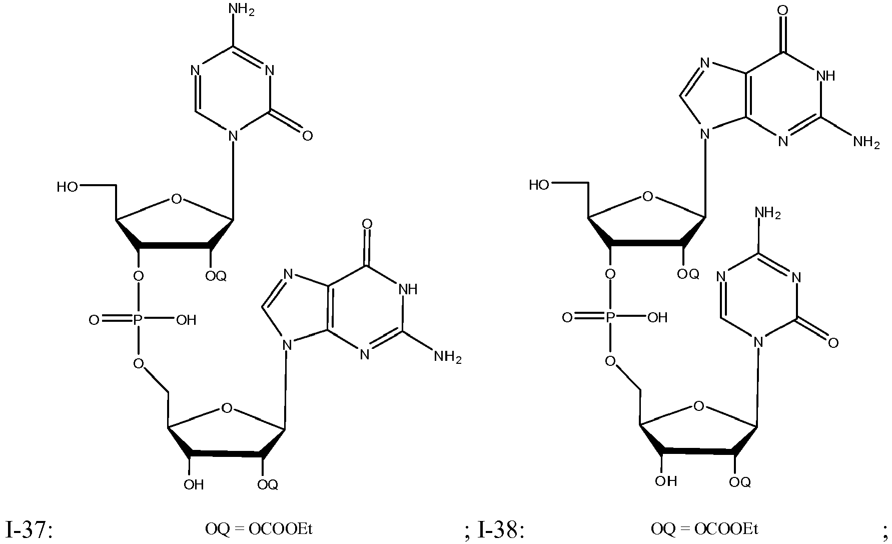 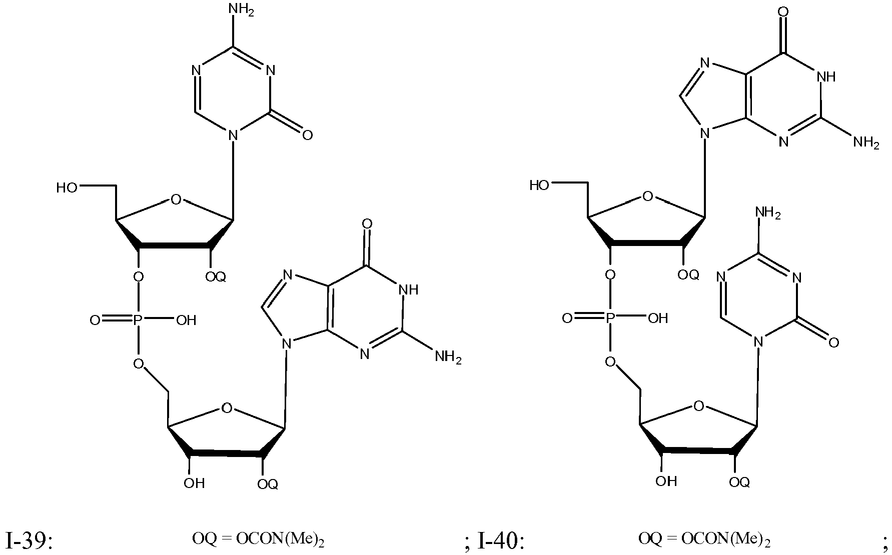 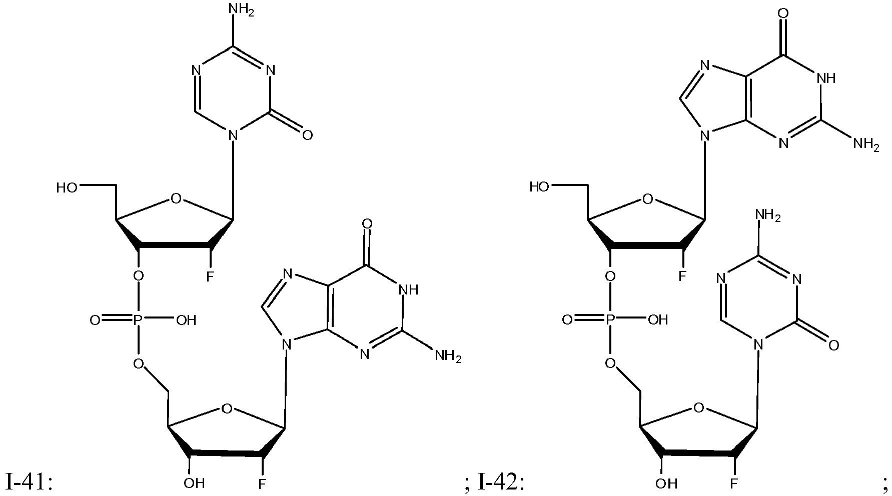 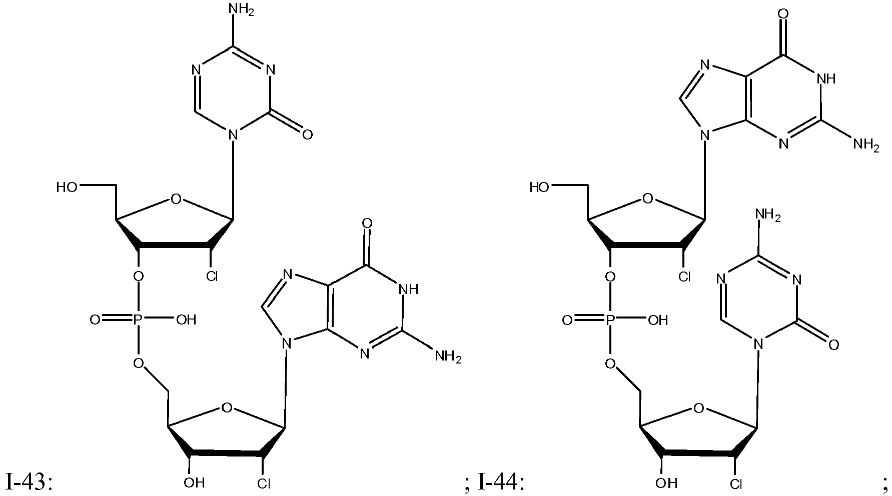 and pharmaceutically-acceptable salts of any of the foregoing. In some embodiments, a salt is a sodium salt of any of the foregoing.

The compounds described herein can be synthesized by methods known in the art, for example, solution phase or solid phase synthesis. For descriptions of the synthesis of compounds of the invention, and for a description of the mechanism of action of compounds of the invention, see WO2007/041071.

### Formulations for use in the combinations of the invention.

The compounds for use in the combinations of the invention can be provided in any suitable form and can be formulated in accordance with known techniques (see, for example, Remington's Pharmaceutical Sciences, Mack Publishing Company, Easton, PA, USA). Examples of suitable formulations are described in WO2007/041071 at pages 13-23.

An efficacious therapy can provide advantageous effects such as additivity, synergism, reduced side effects, reduced toxicity, increased time to disease progression, increased time of survival, sensitisation or desensitisation of one agent to another, or improved response rate. Advantageously, an efficacious effect can allow for lower doses of each or either component to be administered to a patient, thereby decreasing the toxicity of chemotherapy, whilst producing and/or maintaining the same therapeutic effect.

A response rate can describe the percentage of patients achieving a response status. Thus, for example, a 50% response rate means that half of the patients treated achieve response status. A response status can relate to a type of malignancy, for example, whether solid or haematological. In the former case it is usually defined by the RECIST criteria (Response Evaluation Criteria In Solid Tumors), while in the latter other response criteria are used (mainly those of the IWG (International Working Group)).

A synergistic effect can be a therapeutic effect produced by the combination which is larger than the sum of the therapeutic effects of the components of the combination when presented individually.

An additive effect can be a therapeutic effect produced by the combination which is larger than the therapeutic effect of any of the components of the combination when presented individually.

Non-limiting examples of pharmaceutical compositions include any composition suitable for administration to a patient, being, for example, in a form, concentration and/or level of purity suitable for administration to a human or animal subject. In some embodiments, pharmaceutical compositions are sterile and/or non-pyrogenic. A non-pyrogenic pharmaceutical composition does not elicit undesirable inflammatory responses when administered to a patient.

Non-limiting examples of a pharmaceutical kit include an array of one or more unit doses of a pharmaceutical composition together with a dosing device (e.g. measuring device) and/or a delivery device (e.g. inhaler or syringe), optionally all contained within common outer packaging. In pharmaceutical kits comprising a combination of two or more compounds/agents, the individual compounds/agents can be unitary or non-unitary formulations. In some embodiments, the unit dose(s) can be contained within a blister pack. In some embodiments, the pharmaceutical kit further comprises instructions for use.

A pharmaceutical pack can be an array of one or more unit doses of a pharmaceutical composition, optionally contained within common outer packaging. In pharmaceutical packs comprising a combination of two or more compounds/agents, the individual compounds/agents can be unitary or non-unitary formulations. The unit dose(s) can be contained within a blister pack. In some embodiments, the pharmaceutical pack further comprises instructions for use.

A patient pack can be a package, prescribed to a patient, which contains pharmaceutical compositions for the whole course of treatment. Patient packs can contain one or more blister pack(s). Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physician's instructions.

Non-limiting examples of non-physically associated combined compounds/agents include:
- material (e.g. a non-unitary formulation) comprising at least one of the two or more compounds/agents together with instructions for the extemporaneous association of the at least one compound/agent to form a physical association of the two or more compounds/agents;
- material (e.g. a non-unitary formulation) comprising at least one of the two or more compounds/agents together with instructions for combination therapy with the two or more compounds/agents;
- material comprising at least one of the two or more compounds/agents together with instructions for administration to a patient population in which the other(s) of the two or more compounds/agents have been (or are being) administered;
- material comprising at least one of the two or more compounds/agents in an amount or in a form which is specifically adapted for use in combination with the other(s) of the two or more compounds/agents.

Non-limiting examples of combination therapies include therapies which comprise the use of a combination of two or more compounds/agents (as defined above). The compounds can be administered as part of the same overall treatment regimen. As such, the posology of each of the two or more compounds/agents can differ: each can be administered at the same time or at different times. In some embodiments, the compounds/agents of the combination can be administered sequentially (e.g. before or after) or simultaneously, either in the same pharmaceutical formulation (i.e. together), or in different pharmaceutical formulations (i.e. separately). Simultaneously in the same formulation is as a unitary formulation whereas simultaneously in different pharmaceutical formulations is non-unitary. In some embodiments, the compound of Formula I or salt thereof is administered first (as a priming therapy), followed by administration of the ancillary therapeutic component(s). The posologies of each of the two or more compounds/agents in a combination therapy can also differ with respect to the route of administration.

In some embodiments, the combinations of the invention produce a therapeutically efficacious effect relative to the therapeutic effect of the individual compounds/agents when administered separately.

An ancillary therapeutic component can be a compound/agent which yields an efficacious combination when combined with a compound of the formula (I). The ancillary component can contribute to the efficacy of the combination (for example, by producing a synergistic or additive effect or improving the response rate).

The antitumour efficacy of the combinations can be evaluated by reference to effects on DNA methylation and/or modulation of tumour immunological profile. Global or gene-specific DNA methylation can be monitored by analysis of sodium bisulfite-treated DNA using pyrosequencing, quantitative methylation-specific PCR or RT-PCR and real-time quantitative RT-PCR analyses. Tumour immunological profile can be characterized by immunohistochemistry (IHC) for the presence and relative frequency of activated T cells. The immunomodulatory activity of the combinations can also be evaluated by RT-PCR and real time quantitative RT-PCR analyses of the induction or modulation of Cancer Testis Antigens (CTA) such as NY-ESO-1 or MAGE family of antigens. The efficacy of the combination treatment can be also determined by the immune response to the anti-tumour activity of the combinations. For example, modulation of an anti-tumour T cell response can be evaluated by Mixed Lymphocyte Tumour Cell (MLTC) assays. Further details of such analytical techniques are provided in e.g. Coral et al. (2012) Immunomodulatory activity of SGI-110, a 5-aza-2'-deoxycytidine-containing demethylating dinucleotide Cancer Immunol. Immunother. DOI 10.1007/s00262-012-1365-7.

Non-limiting examples of antibodies include: i) whole antibodies (including polyclonal antibodies and monoclonal antibodies (mAbs)); ii) antibody fragments, including F(ab), F(ab'), F(ab')2, Fv, Fc3 and single chain antibodies (and combinations thereof), which can be produced by recombinant DNA techniques or by enzymatic or chemical cleavage of intact antibodies; iii) bispecifc or bifunctional antibodies, which are synthetic hybrid antibodies having two different heavy/light chain pairs and two different binding sites; iv) chimaeric antibodies (antibodies having a human constant antibody immunoglobulin domain coupled to one or more non-human variable antibody immunoglobulin domain, or fragments thereof); v) minibodies (see WO 94/09817), single chain Fv-Fc fusions and human antibodies produced by transgenic animals; and vi) multimeric antibodies and higher-order complexes of proteins (e.g. heterodimeric antibodies). Bispecific antibodies can be produced by a variety of methods including fusion of hybridomas or linking of Fab' fragments. In some embodiments, chimaeric antibodies are humanized antibodies.

Non-limiting examples of immunotherapy include an intervention (e.g. the administration of the combination of the invention to a subject) which cures, ameliorates or lessens the symptoms of a disease or removes (or lessens the impact of) its cause(s), and which is mediated, at least in part, by components of the host immune system. Immunotherapy can be achieved by immunomodulation, can be the stimulation and/or suppression one or more components or activities of the immune system.

The formulations described herein provide the compounds described herein in a form with high solubility, low injection volumes, and good chemical stability and shelf-life. These properties provide formulations that retain a high percentage of the initial efficacy and deliver a therapeutically-effective amount of the compound even after storage at or below room temperature for extended times.

In some embodiments, the invention provides combinations comprising a formulation comprising: a) a compound of Formula I or a pharmaceutically-acceptable salt thereof:

(5-azacytosine group)-L-(guanine group) (I),

as defined herein; and b) a solvent comprising: 45% to 85% propylene glycol; 5% to 45% glycerin; and 0% to 30% ethanol; and c) optionally, a pharmaceutically-acceptable excipient.

Suitable formulations can be solutions or suspensions of a compound in a solvent or a mixture of solvents. Non-limiting examples of suitable solvents include propylene glycol, glycerin, ethanol, and any combination of the foregoing. The formulations can be prepared as non-aqueous formulations. The formulations can be anhydrous or substantially anhydrous.

A mixture of solvents can contain a percentage of propylene glycol on either a mass or a volume basis. In some embodiments, the percentage of propylene glycol can be at least 10%, at least 20%, at least 30%, at least 40%, or at least 50%. In some embodiments, the percentage of propylene glycol can be at most 90%, at most 80%, at most 70%, or at most 60%. In some embodiments, the percentage of propylene glycol can be 30% to 90%, 45% to 85%, 55% to 75%, or 60% to 70%. In some embodiments, the percentage of propylene glycol can be 30%, 35%, 40%, 45%, 50%, 55%, 60%, 65%, 70%, 75%, 80%, 85%, or 90%.

A mixture of solvents can contain a percentage of glycerin on either a mass or a volume basis. In some embodiments, the percentage of glycerin can be at least 5%, at least 10%, at least 15%, at least 25%, or at least 30%. In some embodiments, the percentage of glycerin can be at most 70%, at most 60%, at most 50%, at most 40%, or at most 30%. In some embodiments, the percentage of glycerin can be 0% to 50%, 5% to 45%, 15% to 35%, or 20% to 30%. In some embodiments, the percentage of glycerin can be 0%, 5%, 10%, 15%, 20%, 25%, 30%, 35%, 40%, 45%, or 50%.

A mixture of solvents can contain a percentage of ethanol on either a mass or a volume basis. In some embodiments, the percentage of ethanol can be at least 1%, at least 3%, at least 5%, at least 10%, or at least 15%. In some embodiments, the percentage of ethanol can be at most 30%, at most 25%, at most 20%, at most 15%, or at most 10%. In some embodiments, the percentage of ethanol can be 0% to 30%, 0% to 25%, 0% to 20%, or 5% to 15%. In some embodiments, the percentage of ethanol can be 0%, 1%, 2%, 3%, 4%, 5%, 6%, 7%, 8%, 9%, 10%, 11%, 12%, 13%, 14%, or 15%.

In some embodiments, a solvent or a mixture of solvents comprises 45% to 85% propylene glycol, 5% to 45% glycerin, and 0% to 30% ethanol. In some embodiments, a solvent or a mixture of solvents consists essentially of 45% to 85% propylene glycol, 5% to 45% glycerin, and 0% to 30% ethanol. In some embodiments, a solvent or a mixture of solvents is 45% to 85% propylene glycol, 5% to 45% glycerin, and 0% to 30% ethanol.

In some embodiments, a solvent or a mixture of solvents comprises 55% to 75% propylene glycol, 15% to 35% glycerin, and 0% to 20% ethanol. In some embodiments, a solvent or a mixture of solvents consists essentially of 55% to 75% propylene glycol, 15% to 35% glycerin, and 0% to 20% ethanol. In some embodiments, a solvent or a mixture of solvents is 55% to 75% propylene glycol, 15% to 35% glycerin, and 0% to 20% ethanol.

In some embodiments, a solvent or a mixture of solvents comprises 60% to 70% propylene glycol; 20% to 30% glycerin; and 5% to 15% ethanol. In some embodiments, a solvent or a mixture of solvents consists essentially of 60% to 70% propylene glycol; 20% to 30% glycerin; and 5% to 15% ethanol. In some embodiments, a solvent or a mixture of solvents is 60% to 70% propylene glycol; 20% to 30% glycerin; and 5% to 15% ethanol.

In some embodiments, a solvent or a mixture of solvents comprises 65% propylene glycol; 25% glycerin; and 10% ethanol. In some embodiments, a solvent or a mixture of solvents consists essentially of 65% propylene glycol; 25% glycerin; and 10% ethanol. In some embodiments, a solvent or a mixture of solvents is 65% propylene glycol; 25% glycerin; and 10% ethanol.

Formulations for use in the combinations of the invention can be prepared, stored, transported, and handled in anhydrous or substantially-anhydrous form. A solvent can be dried prior to preparing a formulation, and a compound can be dried, for example, by lyophilization. A drying agent, or dessicant, can be used during preparation, storage, transportation, or handling to regulate water content. Non-limiting examples of drying agents include silica gel, calcium sulfate, calcium chloride, calcium phosphate, sodium chloride, sodium bicarbonate, sodium sulfate, sodium phosphate, montmorillonite, molecular sieves (beads or powdered), alumina, titania, zirconia, and sodium pyrophosphate. A drying agent can contact a formulation directly, be inserted into the formulation in the form of a packet with a permeable membrane, or be stored with the formulation in a sealed environment, such as a dessicator, such that the drying agent and the formulation are simultaneously exposed to the same controlled atmosphere. A drying agent can be removed from a formulation, for example, by filtration or cannulation. Additionally, a formulation can be stored in a sealed container within a controlled atmosphere consisting essentially of, or enriched in, nitrogen or argon.

Anhydrous or substantially-anhydrous conditions benefit the shelf-life of a formulation disclosed herein at both ambient and reduced temperatures. This benefit reduces the costs associated with the storage, transportation, and spoilage of a formulation, increases the convenience of storage and handling, and avoids the need to administer cold formulations, thereby improving subject tolerance and compliance to a regimen of a formulation of the invention.

The formulations can further include a pharmaceutically-acceptable excipient. Non-limiting examples of excipients include mannitol, sorbitol, lactose, dextrose, and cyclodextrins. Excipients can be added to modulate the density, rheology, uniformity, and viscosity of the formulation.

The formulations can include acidic or basic excipients to modulate the acidity or basicity of the formulation. Non limiting examples of acids suitable to increase the acidity of a formulation include hydrochloric acid, hydrobromic acid, hydroiodic acid, sulfuric acid, phosphoric acid, nitric acid, ascorbic acid, citric acid, tartaric acid, lactic acid, oxalic acid, formic acid, benzenesulphonic acid, benzoic acid, maleic acid, glutamic acid, succinic acid, aspartic acid, diatrizoic acid, and acetic acid. Non limiting examples of bases suitable to increase the basicity of a formulation include lithium hydroxide, sodium hydroxide, potassium hydroxide, sodium carbonate, sodium bicarbonate, sodium phosphate, potassium phosphate, sodium acetate, sodium benzoate, tetrabutylammonium acetate, tetrabutylammonium benzoate, and trialkyl amines. Polyfunctional excipients, such as ethylene diamine tetraacetic acid (EDTA), or a salt thereof, can also be used to modulate acidity or basicity.

The compound of Formula I as hereinbefore defined can be present in a formulation in any amount. In some embodiments, the compound is present in a concentration of 1 mg/mL to 130 mg/mL, 10 mg/mL to 130 mg/mL, 40 mg/mL to 120 mg/mL, or 80 mg/mL to 110 mg/mL
In some embodiments, the compound is present in a concentration of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, or 200 mg/mL In some embodiments, the compound is present in a concentration of 100 mg/mL.

The formulation can be prepared by contacting a compound described herein with a solvent or a mixture of solvents. Alternatively, the compound can be contacted with a single solvent, and other solvents can be added subsequently, as a mixture, or sequentially. When the final formulation is a solution, complete solvation can be achieved at whatever step of the process is practical for manufacturing. Optional excipients can be added to the formulation at whatever step is practical for manufacturing.

Preparation of the formulation can be optionally promoted by agitation, heating, or extension of the dissolution period. Non-limiting examples of agitation include shaking, sonication, mixing, stirring, vortex, and combinations thereof.

In some embodiments, the formulation is optionally sterilized. Non-limiting examples of sterilization techniques include filtration, chemical disinfection, irradiation, and heating.

### Dimethyl sulfoxide (DMSO)

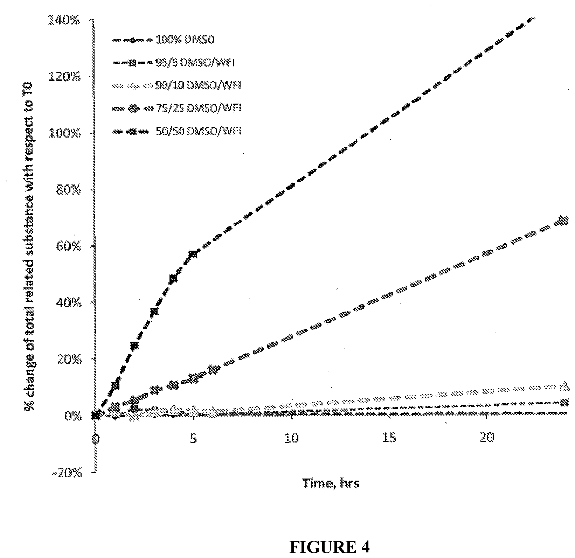

The use of DMSO as a solvent in the preparation of the formulations for use in the combinations of the invention permit reduction in bulk solution and fill volumes (both bulk and fill volumes can be reduced to 1/5^{th} of those used with aqueous systems) and relieves time and temperature restrictions on scale-up. Moreover, the use of substantially anhydrous DMSO greatly increases stability: increasing water concentration is correlated with a decrease in stability (as shown in Figure 4, which shows the % change in total related substances of the sodium salt of a compound of Formula I-1 when stored in DMSO or DMSO/water (water for injection, "WFI") at 25°C/60% RH for 24 hours).

Any source of DMSO can be used according to the invention. In some embodiments, the DMSO source is suitable for healthcare and drug delivery applications, for example conforming to USP or Ph. Eur monographs, and be manufactured under cGMP and API guidelines. Grades such as anhydrous or Pharma Solvent can be used according to the invention.

The DMSO for use according to the invention can have impurities in very low levels, for example <0.2% water by KF, <0.01% non-volatile residue and <0.1% of related compounds.

In some embodiments, DMSO can incliude isosteres thereof, including in particular DMSO isosteres in which one or more atom(s) is(are) replaced by a cognate isotope, for example hydrogen by deuterium.

### Dosing and Administration

Suitable doses of formulations of the invention can be administered to a subject by methods known in the art, and exemplary dosing and administration parameters are described in WO2007/041071.

Thus, non-limiting examples of methods of administration include subcutaneous injection, intravenous injection, and infusion. In some embodiments, a subject is in need or want of the formulation. In some embodiments, the administration is subcutaneous administration.

A therapeutically effective amount of a compound of the invention can be expressed as mg of the compound per kg of subject body mass. In some embodiments, a therapeutically effective amount is 1-1,000 mg/kg, 1-500 mg/kg, 1-250 mg/kg, 1-100 mg/kg, 1-50 mg/kg, 1-25 mg/kg, or 1-10 mg/kg. In some embodiments, a therapeutically-effective amount is 5 mg/kg, 10 mg/kg, 25 mg/kg, 50 mg/kg, 75 mg/kg, 100 mg/kg, 150 mg/kg, 200 mg/kg, 250 mg/kg, 300 mg/kg, 400 mg/kg, 500 mg/kg, 600 mg/kg, 700 mg/kg, 800 mg/kg, 900 mg/kg, or 1,000 mg/kg.

A therapeutically effective amount of a compound of the invention can also be expressed as mg of the compound per square metre of subject body area. In some embodiments, the combinations of the invention can be administered subcutaneously in a range of doses, for example 1 to 1500 mg (0.6 to 938 mg/m2), or 2 to 800 mg (1.25 to 500mg/m2), or 5 to 500 mg (3.1 to 312 mg/m2), or 2 to 200 mg (1.25 to 125 mg/m2) or 10 to 1000 mg (6.25 to 625 mg/m2), particular examples of doses including 10 mg (6.25 mg/m2), 20 mg (12.5 mg/m2), 50 mg (31.3 mg/m2), 80 mg (50 mg/m2), 100 mg (62.5 mg/m2), 200 mg (125 mg/m2), 300 mg (187.5 mg/m2), 400 mg (250 mg/m2), 500 mg (312.5 mg/m2), 600 mg (375 mg/m2), 700 mg (437.5 mg/m2), 800 mg (500 mg/m2), 900 mg (562.5mg/m2) and 1000 mg (625 mg/m2).

The combination can be administered once or more than once each day. The combination is typically administered continuously (i.e. taken every day without a break for the duration of the treatment regimen).

In some embodiments, a therapeutically effective amount can be administered 1-35 times per week, 1-14 times per week, or 1-7 times per week. In some embodiments, a therapeutically-effective amount can be administered 1-10 times per day, 1-5 times per day, 1 time, 2 times, or 3 times per day.

In some embodiments, the materials of the invention can be administered according to a dosage regimen of: (a) once, twice, three times, four times, five times, six times or seven times a week; or (b) every day for 5, 6, 7, 8, 9 or 10 days; or (c) every day for up to 10 days; or (d) every day for between 5 and 10 days; or (e) every day for 5 days, immediately followed by two dose-free days and then every day for the next 5 days. In some embodiments, administration is subcutaneous.

### Therapeutic Uses

The combinations of the present invention can be used to treat a wide variety of diseases. In one embodiment, the combination of the invention is for use in treating a disease selected from:
(a) a myelodysplastic syndrome (MDS);
(b) a cancer;
(c) a haematological disorder; and
(d) a disease associated with abnormal haemoglobin synthesis;

Indications that can be treated include those involving undesirable or uncontrolled cell proliferation. Such indications include benign tumours, various types of cancers such as primary tumours and tumour metastasis, restenosis (e.g. coronary, carotid, and cerebral lesions), haematological disorders, abnormal stimulation of endothelial cells (atherosclerosis), insults to body tissue due to surgery, abnormal wound healing, abnormal angiogenesis, diseases that produce fibrosis of tissue, repetitive motion disorders, disorders of tissues that are not highly vascularized, and proliferative responses associated with organ transplants.

Generally, cells in a benign tumour retain their differentiated features and do not divide in a completely uncontrolled manner. A benign tumour is usually localized and nonmetastatic. Specific types benign tumours that can be treated using the present invention include hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas.

In a malignant tumour cells become undifferentiated, do not respond to the body's growth control signals, and multiply in an uncontrolled manner. The malignant tumour is invasive and capable of spreading to distant sites (metastasizing). Malignant tumours are generally divided into two categories: primary and secondary. Primary tumours arise directly from the tissue in which they are found. A secondary tumour, or metastasis, is a tumour which is originated elsewhere in the body but has now spread to a distant organ. The common routes for metastasis are direct growth into adjacent structures, spread through the vascular or lymphatic systems, and tracking along tissue planes and body spaces (peritoneal fluid, cerebrospinal fluid, etc.).

Specific types of cancers or malignant tumours, either primary or secondary, that can be treated using this invention include breast cancer, skin cancer, bone cancer, prostate cancer, liver cancer, lung cancer, brain cancer, cancer of the larynx, gall bladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumour, small-cell lung tumour, gallstones, islet cell tumour, primary brain tumour, acute and chronic lymphocytic and granulocytic tumours, hairy-cell tumour, adenoma, hyperplasia, medullary carcinoma, pheochromocytoma, mucosal neuronms, intestinal ganglioneuromas, hyperplastic corneal nerve tumour, marfanoid habitus tumour, Wilm's tumour, seminoma, ovarian tumour, leiomyomater, cervical dysplasia and in situ carcinoma, neuroblastoma, retinoblastoma, soft tissue sarcoma, malignant carcinoid, topical skin lesion, mycosis fimgoide, rhabdomyosarcoma, Kaposi's sarcoma, osteogenic and other sarcoma, malignant hypercalcemia, renal cell tumour, polycythemia vera, adenocarcinoma, glioblastoma multiforma, leukemias, lymphomas, malignant melanomas, epidermoid carcinomas, and other carcinomas and sarcomas.

Haematologic disorders include abnormal growth of blood cells which can lead to dysplastic changes in blood cells and haematologic malignancies such as various leukemias. Examples of haematologic disorders include but are not limited to acute myeloid leukemia, acute promyelocytic leukemia, acute lymphoblastic leukemia, chronic myelogenous leukemia, the myelodysplastic syndromes, and sickle cell anaemia.

Treatment of abnormal cell proliferation due to insults to body tissue during surgery can be possible for a variety of surgical procedures, including joint surgery, bowel surgery, and cheloid scarring. Diseases that produce fibrotic tissue include emphysema. Repetitive motion disorders that can be treated include carpal tunnel syndrome. An example of cell proliferative disorders that can be treated using the invention is a bone tumour.

Also described herein, proliferative responses associated with organ transplantation can be treated including those proliferative responses contributing to potential organ rejections or associated complications. Specifically, these proliferative responses can occur during transplantation of the heart, lung, liver, kidney, and other body organs or organ systems.

Also described herein, abnormal angiogenesis can be treated including, for example, those abnormal angiogenesis accompanying rheumatoid arthritis, ischaemic-reperfusion related brain oedema and injury, cortical ischemia, ovarian hyperplasia and hypervascularity, (polycystic ovary syndrome), endometriosis, psoriasis, diabetic retinopathy, and other ocular angiogenic diseases such as retinopathy of prematurity (retrolental fibroplastic), muscular degeneration, corneal graft rejection, neuroscular glaucoma and Oster Webber syndrome.

Diseases associated with abnormal angiogenesis require or induce vascular growth. For example, corneal angiogenesis involves three phases: a pre-vascular latent period, active neovascularization, and vascular maturation and regression.

Described herein, the formulations and compositions can be used for treating diseases associated with undesired or abnormal angiogenesis. The the pharmaceutical formulations are for use alone, or in combination with anti-neoplastic agent whose activity as an anti-neoplastic agent *in vivo* is adversely affected by high levels of DNA methylation. The particular dosage of these agents required to inhibit angiogenesis and/or angiogenic diseases can depend on the severity of the condition, the route of administration, and related factors that can be decided by the attending physician. Generally, accepted and effective daily doses are the amount sufficient to effectively inhibit angiogenesis and/or angiogenic diseases.

Described herein, the pharmaceutical formulations can be used to treat a variety of diseases associated with undesirable angiogenesis such as retinal/choroidal neovascularization and corneal neovascularization. Examples of retinal/choroidal neovascularization include, but are not limited to, Bests diseases, myopia, optic pits, Stargarts diseases, Paget's disease, vein occlusion, artery occlusion, sickle cell anaemia, sarcoid, syphilis, pseudoxanthoma elasticum carotid obstructive diseases, chronic uveitis/vitritis, mycobacterial infections, Lyme's disease, systemic lupus erythematosis, retinopathy of prematurity, Eales disease, diabetic retinopathy, macular degeneration, Bechets diseases, infections causing a retinitis or chroiditis, presumed ocular histoplasmosis, pars planitis, chronic retinal detachment, hyperviscosity syndromes, toxoplasmosis, trauma and post- laser complications, diseases associated with rubesis (neovascularization of the angle) and diseases caused by the abnormal proliferation of fibrovascular or fibrous tissue including all forms of proliferative vitreoretinopathy.

Non-limiting examples of corneal neuvascularization include, but are not limited to, epidemic keratoconjunctivitis, Vitamin A deficiency, contact lens overwear, atopic keratitis, superior limbic keratitis, pterygium keratitis sicca, sjogrens, acne rosacea, phylectenulosis, diabetic retinopathy, retinopathy of prematurity, corneal graft rejection, Mooren ulcer, Terrien's marginal degeneration, marginal keratolysis, polyarteritis, Wegener sarcoidosis, Scleritis, periphigoid radial keratotomy, neo vascular glaucoma and retrolental fibroplasia, syphilis, Mycobacteria infections, lipid degeneration, chemical burns, bacterial ulcers, fungal ulcers, Herpes simplex infections, Herpes zoster infections, protozoan infections and Kaposi sarcoma.

Described herein, the pharmaceutical formulations can be used for treating chronic inflammatory diseases associated with abnormal angiogenesis. The pharmaceutical formulations are for use alone, or in combination with an anti-neoplastic agent whose activity as an anti-neoplastic agent in vivo is adversely affected by high levels of DNA methylation. The chronic inflammation depends on continuous formation of capillary sprouts to maintain an influx of inflammatory cells. The influx and presence of the inflammatory cells produce granulomas and thus, maintains the chronic inflammatory state. Inhibition of angiogenesis using the pharmaceutical formulations of the present invention can prevent the formation of the granulosmas, thereby alleviating the disease. Examples of chronic inflammatory disease include, but are not limited to, inflammatory bowel diseases such as Crohn's disease and ulcerative colitis, psoriasis, sarcoidois, and rheumatoid arthritis.

Inflammatory bowel diseases such as Crohn's disease and ulcerative colitis are characterized by chronic inflammation and angiogenesis at various sites in the gastrointestinal tract. For example, Crohn's disease occurs as a chronic transmural inflammatory disease that most commonly affects the distal ileum and colon but can also occur in any part of the gastrointestinal tract from the mouth to the anus and perianal area. Patients with Crohn's disease generally have chronic diarrhoea associated with abdominal pain, fever, anorexia, weight loss and abdominal swelling. Ulcerative colitis is also a chronic, nonspecific, inflammatory and ulcerative disease arising in the colonic mucosa and is characterized by the presence of bloody diarrhoea. These inflammatory bowel diseases are generally caused by chronic granulomatous inflammation throughout the gastrointestinal tract, involving new capillary sprouts surrounded by a cylinder of inflammatory cells. Inhibition of angiogenesis by the pharmaceutical formulations described herein should inhibit the formation of the sprouts and prevent the formation of granulomas. The inflammatory bowel diseases also exhibit extra intestinal manifectations, such as skin lesions. Such lesions are characterized by inflammation and angiogenesis and can occur at many sites other the gastrointestinal tract. Inhibition of angiogenesis by the pharmaceutical formulations described herein should reduce the influx of inflammatory cells and prevent the lesion formation.

Sarcoidois, another chronic inflammatory disease, is characterized as a multi-system granulomatous disorder. The granulomas of this disease can form anywhere in the body and, thus, the symptoms depend on the site of the granulomas and whether the disease is active. The granulomas are created by the angiogenic capillary sprouts providing a constant supply of inflammatory cells. By using the pharmaceutical formulations described herein to inhibit angiogenesis, such granulomas formation can be inhibited. Psoriasis, also a chronic and recurrent inflammatory disease, is characterized by papules and plaques of various sizes. Treatment using the pharmaceutical formulations described herein should prevent the formation of new blood vessels necessary to maintain the characteristic lesions and provide the patient relief from the symptoms.

Rheumatoid arthritis (RA) is also a chronic inflammatory disease characterized by non-specific inflammation of the peripheral joints. It is believed that the blood vessels in the synovial lining of the joints undergo angiogenesis. In addition to forming new vascular networks, the endothelial cells release factors and reactive oxygen species that lead to pannus growth and cartilage destruction. The factors involved in angiogenesis can actively contribute to, and help maintain, the chronically inflamed state of rheumatoid arthritis. Treatment using the pharmaceutical formulations described herein alone or in conjunction with other anti-RA agents can prevent the formation of new blood vessels necessary to maintain the chronic inflammation and provide the RA patient relief from the symptoms.

In some embodiments, the pharmaceutical formulations of the present invention can be used for treating diseases associated with abnormal haemoglobin synthesis. The method comprises administering the pharmaceutical formulations of the present invention to a patient suffering from disease associated with abnormal haemoglobin synthesis. Decitabine containing formulations stimulate foetal haemoglobin synthesis because the mechanism of incorporation into DNA is associated with DNA hypomethylation. Examples of diseases associated with abnormal haemoglobin synthesis include, but are not limited to, sickle cell anaemia and beta-thalassemia.

In some embodiments, the pharmaceutical formulations described herein can be used to control intracellular gene expression. The method comprises administering the pharmaceutical formulations to a patient suffering from disease associated with abnormal levels of gene expression. DNA methylation is associated with the control of gene expression. Specifically, methylation in or near promoters inhibit transcription while demethylation restores expression. Examples of the possible applications of the described mechanisms include, but are not limited to, therapeutically modulated growth inhibition, induction of apoptosis, and cell differentiation.

Gene activation facilitated by the pharmaceutical formulations described herein can induce differentiation of cells for therapeutic purposes. Cellular differentiation is induced through the mechanism of hypomethylation. Examples of morphological and functional differentiation include, but are not limited to differentiation towards formation of muscle cells, myotubes, cells of erythroid and lymphoid lineages.

Myelodysplastic syndromes (MDS) are heterogeneous clonal haematopoietic stem cell disorders associated with the presence of dysplastic changes in one or more of the haematopoietic lineages, including dysplastic changes in the myeloid, erythroid, and megakaryocytic series. These changes result in cytopenias in one or more of the three lineages. Subjects afflicted with MDS typically develop complications related to anaemia, neutropenia (infections), or thrombocytopenia (bleeding). Generally, from about 10% to about 70% of subjects with MDS develop acute leukemia. Representative myelodysplastic syndromes include acute myeloid leukemia, acute promyelocytic leukemia, acute lymphoblastic leukemia, and chronic myelogenous leukemia.

Acute myeloid leukemia (AML) is the most common type of acute leukemia in adults. Several inherited genetic disorders and immunodeficiency states are associated with an increased risk of AML. These include disorders with defects in DNA stability leading to random chromosomal breakage, such as Bloom's syndrome, Fanconi's anaemia, Li-Fraumeni kindreds, ataxia-telangiectasia, and X-linked agammaglobulinemia.

Acute promyelocytic leukemia (APML) represents a distinct subgroup of AML. This subtype is characterized by promyelocytic blasts containing the 15; 17 chromosomal translocation. This translocation leads to the generation of a fusion transcript comprising a retinoic acid receptor sequence and a promyelocytic leukemia sequence.

Acute lymphoblastic leukemia (ALL) is a heterogeneous disease with distinct clinical features displayed by various subtypes. Reoccurring cytogenetic abnormalities have been demonstrated in ALL. The most common associated cytogenetic abnormality is the 9; 22 translocation leading to development of the Philadelphia chromosome.

Chronic myelogenous leukemia (CML) is a clonal myeloproliferative disorder of a pluripotent stem cell, generally caused by ionizing radiation. CML is characterized by a specific chromosomal abnormality involving the translocation of chromosomes 9 and 22, creating the Philadelphia chromosome.

Compounds described herein and formulations thereof can be used to provide therapy for a MDS. In some embodiments, a compound or formulation thereof can provide therapy for more than one MDS in a single administration.

In some embodiments, the combination of the invention is for use in treating a myelodysplastic syndrome (MDS). In some embodiments, the combination of the invention is for use in treating one or more myelodysplastic syndromes, leukemia, or solid tumours. In some embodiments, the combination of the invention is for use in treating acute myeloid leukemia (AML). In some embodiments, the combination of the invention is for use in treating acute promyelocytic leukemia (APML) in a subject. In some embodiments, the combination of the invention is for use in treating acute lymphoblastic leukemia (ALL). In some embodiments, the combination of the invention is for use in treating chronic myelogenous leukemia (CML).

In some embodiments, the myelodysplastic syndrome is acute myeloid leukemia (AML), acute promyelocytic leukemia (APL), acute lymphoblastic leukemia (ALL), or chronic myelogenous leukemia (CML).

In some embodiments, the administration is subcutaneous.

Treatment of any condition described above, such as tumor growth, a myelodysplastic syndrome, or an angiogenesis-related condition, can be accomplished with a level of efficacy. A level of efficacy can be 5%, 10%, 15%, 20%, 25%, 30%, 35%, 40%, 45%, 50%, 55%, 60%, 62.9%, 65%, 70%, 75%, 80%, 84.4%, 85%, 90%, 95%, 97%, 98%, 99%, or 100%. A level of efficacy can be at least 5%, at least 10%, at least 15%, at least 20%, at least 25%, at least 30%, at least 35%, at least 40%, at least 45%, at least 50%, at least 55%, at least 60%, at least 62.9%, at least 65%, at least 70%, at least 75%, at least 80%, at least 84.4%, at least 85%, at least 90%, at least 95%, at least 97%, at least 98%, or at least 99%.

### Combination Therapy

The compounds, compositions and formulations of the present invention are used in combination with one or more ancillary therapeutic components wherein said ancillary therapeutic component is a T-cell activating agent, including immunomodulating antibodies. In one preferred embodiment, the ancillary therapeutic component comprises: (a) a T-cell activating agent and a cancer vaccine; or (b) a T-cell activating agent and an IDO inhibitor; optionally wherein the ancillary therapeutic component further comprises an adjuvant.

In some embodiments, an ancilliary agent can be an ionic form, salt, solvate, isomer, tautomer, N-oxide, ester, prodrug, isotope or protected form of an ancilliary agent (for example, the salts or tautomers or isomers or N-oxides or solvates thereof).

### 1. T-cell activating agents, including immunomodulating antibodies

Suitable ancillary therapeutic components for use in the combinations of the invention include T-cell activating agents.

Such agents include, for example, those which promote T-cell activation and render T effector cells resistant to T regulatory cells (Tregs), including agents which block CTLA-4.

Anti-CTLA-4 mAb therapy represents an anti-tumour strategy implicated in augmentation of the cell-mediated immune system by blocking inhibitory pathways of T-cell activation (O'Day SJ, et al. Cancer 2007; 110:2614-2627). Blockade of CTLA-4 signalling has been shown to induce tumour rejection in animal models, when used alone or combined with other immunotherapeutic strategies (Leach DR, et al. Science 1996;271:1734-1736; Weber J, Semin Oncol 2010;37:430-439). Anti-CTLA-4 mAb are proving effective in inducing long lasting clinical responses and improved survival in metastatic cutaneous melanoma patients (Hodi FS, et al. N Engl J Med 2010;363:711-23; Di Giacomo A, et al. Cancer Immunol Immunother 2011;60:467-77).

In embodiments in which the ancillary therapeutic component comprises an agent which blocks CTLA-4 signalling (e.g. an anti-CTLA-4 mAb as described below), the compound of Formula I or salt thereof is preferably administered first (as a priming therapy), followed by administration of the agent which blocks CTLA-4 (e.g. anti-CTLA mAb).

Non-limiting examples of a suitable anti-CTLA-4 mAbs include Tremelimumab (CP675,206) (Pfizer), an IgG2 isotype monoclonal antibody and Ipilimumab (MDX-010) (BMS/Medarex), an IgG1 isotype monoclonal antibody.

Tremelimumab is described in e.g. WO00/037504 and; and in WO2006/048749.

Ipilimumab is described in e.g. WO01/014424 ; and in WO2012/033953.

Another class of T-cell activating agents suitable for use as ancillary therapeutic components are agents which eliminate or suppress peripheral tolerance and/or reduce the numbers of Tregs at the tumour site. Examples of such agents include agents which block programmed death receptor-1 (PD-1), programmed death receptor-1 ligand (PD-L1) and programmed death receptor-2 ligand (PD-L2), including antibodies against PD-1, PD-L1 and PD-L2.

Programmed death 1 (PD-1) protein, a T-cell co-inhibitory receptor, and its ligands, PD-L1 and PD-L2, play a crucial role in the ability of tumour cells to evade the host's immune system. Blockade of interactions between PD-1 and PD-L1/2 mediates antitumour activity in preclinical models (Iwai Y et al., Proc Natl Acad Sci USA (2002) 99: 12293-12297). Studies identified clinical activity of both anti-PD-1 and anti-PD-Ll mAb in patients with advanced cancers, including non-small-cell lung cancer, melanoma, and renal-cell cancer (Brahmer JR, et al. N Engl J Med. 2012 Jun 2; Topalian SL, et al. N Engl J Med. 2012 Jun 2).

Non-limiting examples of suitable anti-PD-1 and anti-PD-Ll mAbs include BMS-936558 (Nivolumab, ONO 4538), a fully-human monoclonal IgG4 antibody against PD-1 and BMS-936559 (a fully-human, PD-L1-specific, IgG4 (S228P) monoclonal antibody that inhibits the binding of PD-L1 to PD-1 and CD80) described in WO2007/005874. These mAbs can be adminsitered according to the recommendations of the manufacturers, and suitable dosages for Nivolumab are described in WO2006/121168.

Other anti-PD-1 agents include MK-3475 (Lambrolizumab), a humanized anti-PD-1 IgG4 monoclonal antibody. Suitable dosages for Lambrolizumab include 10 mg/kg once every 2 weeks.

Other anti-PDL-1 agents include MED14736 (a human IgG1 monoclonal antibody against PDL-1), and MPDL3280A (is a human IgG monoclonal antibody whose Fc-domain has been specifically engineered to prevent antibody-dependent cell-mediated cytotoxicity). These mAbs can be adminsitered according to the recommendations of the manufacturers.

Other classes of T-cell activating agents suitable for use as ancillary therapeutic components include agonists for CD137, CD40 and OX40. Examples of such agents include monoclonal antibodies which act as agonists of CD137, CD40 or OX40.

CD137 is also known as the 4-1BB receptor (4-1BBR), a glycoprotein which is a member of the tumor necrosis factor receptor superfamily 1-4 and binds to a high-affinity ligand (4-1BBL) expressed on several antigen-presenting cells such as macrophages and activated B cells. A suitable agonist for CD137 is PF-05082566, a fully human IgG2 mAb that binds to the extracellular domain of human CD137 with high affinity and specificity (see Fisher et al. (2012) Cancer Immunology, Immunotherapy, Volume 61, Issue 10, pp 1721-1733).

Other classes of T-cell activating agents suitable for use as ancillary therapeutic components include agonists for ICOS, GITR, MHC, CD80, CD86, Galectin 9 and LAG-3. Examples of such agents include monoclonal antibodies which act as agonists of ICOS, GITR, MHC, CD80, CD86, Galectin 9 and LAG-3.

Combinations of two or more T-cell activating agents (for example, combinations of CTLA-4-blocking antibodies and/or antibodies against PD-1 and PD-L1 and/or PD-L2) can also be used as ancillary therapeutic components for use according to the invention.

### 2. Cancer vaccines

Cancer vaccines which stimulate the adaptive immune response find application as ancillary therapeutic components for use in the combinations of the invention.

Cancer vaccines present tumour associated antigen(s) (TAA) to the immune system of a host to prompt that host to mount a therapeutic adaptive cellular and/or humoral immune response, for example *via* T-cell activation and/or dendritic cell (DC) activation. Cancer vaccines can be based on whole tumour cells, tumour cell extracts or fractions. Also suitable for use according to the invention are subunit cancer vaccines, conjugate cancer vaccines and DNA vaccines.

Any suitable antigen or combination of TAAs can be used in the vaccines of the invention, including, for example, nucleic acid(s) (DNA or RNA) which encode one or more TAA(s); protein(s) or peptide(s); glycoprotein(s); polysaccharide(s) and other carbohydrate(s)); fusion protein(s); lipid(s); glycolipid(s); peptide mimic(s) of polysaccharides; carbohydrate(s) and a protein(s) in admixture; carbohydrate-protein conjugate(s); cells or extracts thereof or tumour cells or extracts thereof.

Subunit vaccines are based on synthetic or isolated antigens created using (bio)chemical and/or recombinant techniques (e.g. recombinant peptides, protein and/or carbohydrate synthesis or purification). Conjugate vaccines involve the linkage (usually by chemical crosslinking) of relatively non-immunogenic (usually carbohydrate-based) antigens to more strongly immunogenic carrier proteins. DNA/RNA vaccines deliver the antigen(s) in the form of encoding nucleic acid and so rely on endogenous expression of the coding sequence after administration. Such vaccines normally require an appropriate vector (e.g. plasmid, viral or lipid vesicle) to deliver the coding nucleic acid to the appropriate compartment of the host.

Suitable cancer vaccines for use in combination with the compositions, formulations and compounds of the invention can be classified according to the nature of the TAA, and include cancer testis antigen (CTA) vaccines.

Cancer/testis antigens (CTAs) are highly immunogenic with no or highly restricted expression in normal tissues (testis and placenta). Examples include vaccines based on: NY-ESO-1, LAGE-1, MAGE-A1, -A2, -A3, -A4, -A6, -A10, -A12, CT7, CT10, GAGE1-6, GAGE 1-2, BAGE, SSX1-5, SSX 2, HAGE, PRAME, RAGE-1, XAGE-1, MUC2, MUC5B, B7.1/2, CD28, B7-H1, HLA, CD40L and HMW-MAA. In some embodiments, the CTA vaccines includes those based on MAGE-A1, MAGE-A3 (for example recMAGE-A3), NY-ESO-1 and PRAME. The foregoing CTA's can be used alone or in combination, for example a mixture of two or more of MAGE-A1, MAGE-A3 (for example recMAGE-A3), NY-ESO-1 and/or PRAME can be used.

MAGE-A1 is described in e.g. WO2002/094859. It can be used unadjuvanted or adjuvanted.

MAGE-A3 (and in particular recMAGE-A3), or Astuprotimut-R) is described in e.g. WO1999/040188. It can be used unadjuvanted or adjuvanted, for example in the form of Astuprotimut-R. It can be used at a dose of about 1 to about 1000 µg of protein. In some embodiments, ther dose is about 30 - about 300 µg.

NY-ESO-1 is described in e.g. WO2005/032475 It can be used unadjuvanted or adjuvanted, and may be used with other chemotherapeutic agents (including, for example, doxorubicin).

PRAME (a.k.a. preferential antigen of melanoma, MAPE, DAGE and OIP4) is described in e.g. WO2006/071983. It can be used unadjuvanted or adjuvanted.

The various CTA antigens described above can be used in the context of various cell-based vaccines, for example dendritic cell-based vaccines. For example, in one dendritic cell-based treatment paradigm, the cells are loaded (pulsed, primed or spiked) with a particular antigen or antigens (e.g. MAGE-A1, MAGE-A3 (for example recMAGE-A3), NY-ESO-1 or PRAME) and then administered to promote an immune response. This approach can be used in conjunction with various adjuvants, including for example TLR agonists (e.g. imiquimod).

In an illustrative example of a cell-based treatment paradigm, the patient receives 3 monthly cycles of the compound of Formula I (or salt thereof) for 5 days, with each cycle followed by 2 weekly vaccines comprising autologous dendritic cells pulsed with overlapping peptides derived from full-length MAGE-A1, MAGE-A3, and NY-ESO-1 (JPT Peptide Technologies, Berlin, Germany). Imiquimod is administered at the vaccine site before and after vaccination, to promote immune cell infiltration at the vaccination site.

Another suitable class of cancer vaccines are those based on differentiation antigens, including MART-1, tyrosinase and Gp100.

In some embodiments, cancer vaccines are used in combination with one or more adjuvants as further ancillary therapeutic components for use in the combinations of the invention, as described below.

### 3. IDO inhibitors

Suitable ancillary therapeutic components for use with the combinations of the invention include inhibitors of indoleamine 2,3-dioxygenase (IDO).

IDO is an important immune regulator, having a key role in tumour immunosurveillance. Immune escape is a fundamental trait of cancer in which the Th1-type cytokine interferon- γ (IFN-γ) seems to play a key role. Among other tumoricidal biochemical pathways, IFN-γ induces IDO in a variety of cells including macrophages, dendritic cells (DCs) and tumor cells. IDO works by preventing T cell activation and blocking immune responses to cancer cells. While the pathways responsible for tryptophan depletion are unknown, hypermethylation could be one of the causative mechanisms that result in failure to mount an immune response.

IDO inhibitors may therefore increase the efficacy of anticancer immunotherapy, and may be used in the combinations of the invention to prevent IDO-mediated immunologic tolerance/immune escape (see e.g. Sucher et al. (2010) IDO-Mediated Tryptophan Degradation in the pathogenesis of Malignant Tumor Disease. International Journal of Tryptophan Research: 3, 113-120).

Any suitable IDO inhibitor may be used according to the invention. Also suitable are inhibitors of IDO isoenzymes, including for example tryptophan (2,3)-dioxygenase (TDO) and/or IDO2. Thus, the IDO inhibitor for use with the combinations of the invention may inhibit, directly or indirectly, IDO and/or TDO and/or IDO2.

Suitable IDO inhibitors include those based on natural products, such as the cabbage extract brassinin, the marine hydroid extract annulin B and the marine sponge extract exiguamine A, including synthetic derivatives thereof.

Other suitable IDO inhibitors include molecular analogues of its substrate, tryptophan. Such inhibitors include the tryptophan mimetic 1-methyl tryptophan (1-MT). 1-MT occurs as two stereoisomers: the L isomer significantly inhibits IDO1, while the D isomer is more specific for IDO2. The D isomer (D-1-MT, indoximod) is currently being evaluated in a phase II, double-blind, randomized, placebo-controlled trial.

Other suitable IDO inhibitors include INCB24360, a hydroxyamidine small-molecule inhibitor. Unlike 1-MT-based inhibitors, hydroxyamidine inhibitors also inhibit tryptophan (2,3)-dioxygenase (TDO), an enzyme with identical activity to IDO.

Yet another suitable IDO inhibitor is NLG919.

Agents which do not inhibit the IDO enzyme directly, but rather block the downstream effects of IDO activation, are also suitable for use with the combinations of the invention. Such IDO pathway inhibitors are intended to be encompassed by the term "IDO inhibitors" as used herein.

### 4. Adjuvants

Suitable ancillary therapeutic components for use with the combinations of the invention include adjuvants.

An adjuvant is any compound or composition that increases the strength and/or duration of an immune response to a foreign antigen relative to that elicited by the antigen alone. Key functional characteristics of an adjuvant therefore include its ability to enhance an appropriate immune response to the target antigen, long-term safety in widespread application, and flexibility in use with different antigen/disease applications. An adjuvant can be, for example, an agent that does not constitute a specific antigen, but boosts the strength and/or longevity of the immune response (including for example the innate immune response) to a co-administered antigen.

Where an adjuvant is used as an ancillary therapeutic component, the compound and adjuvant can be co-administered, i.e. simultaneously or sequentially. When the adjuvants are administered simultaneously they can be administered in the same or separate formulations, and in the latter case at the same or separate sites, but can be administered at the same time. The adjuvants can be administered sequentially, when the administration of the at least two adjuvants is temporally separated. The separation in time between the administration of the two adjuvants can be a matter of minutes or it can be longer. The separation in time can be less than 14 days, less than 7 days, or less than 1 day. The separation in time can also be, for example, with one adjuvant at prime and one at boost, or one at prime and the combination at boost, or the combination of one at prime and one at boost.

Non-limiting examples of suitable adjuvants/adjuvant classes include Pathogen-Recognition Receptors (PRRs) ligands. The include ligands/agonists for RIG-1 receptors, NOD-protein ligands, Toll-like receptor (TLR) ligands and C-type lectin ligands. Suitable PRR ligands bind to one or more of TLR1, TLR2, TLR3, TLR4, TLR5, TLR6, TLR7, TLR8, TLR9, TLR10 and TLR11, i.e. TLR ligands. In some embodiments, a ligand is a TLR9 or TLR4 ligand. In some embodiments, a ligand is an adjuvants that comprises TLR ligands for two or more different TLRs, including for example adjuvants which comprise double or triple TLR ligands, for example TLR2 and/or TLR6 and/or TLR3 and/or TLR9. In some embodiments, a triple TLR ligand comprises a ligand of TLR2, TLR3, and TLR9. In some embodiments, the adjuvant comprises a combination of: (i) a TLR 7/8 agonist with a TLR 9 agonist; (ii) a TLR 7/8 agonist with a TLR 4 agonist; or (iii) a TLR 9 agonist with a TLR 3 agonist.

TLR-based adjuvants are reviewed in Steinhagen et al. (2011) TLR-Based Immune Adjuvants Vaccine 29(17): 3341-3355.

The combinations of the invention can also be used adjunctively with other chemotherapeutic and/or non-chemotherapeutic treatments such as radiotherapy, surgery and stem cell transplantation. For example, the combinations of the invention can be used following surgery and/or radiotherapy of a primary tumour to prevent or delay relapse/metastasis. In the case of adjunctive use with stem cell transplants, the combinations can be used as a "bridge to transplant", where the combinations of the invention are used to achieve a response rate sufficient (for example, curative) for stem cell transplantation. The combinations can also be used at a lower, maintenance dose after stem cell transplantation to reduce/prevent recurrence.

### EXAMPLES

### EXAMPLE 1: Molecular, phenotypic, and functional in vivo effects of SGI-110 combined with immunostimulatory mAb in a murine cancer model

A syngeneic model of murine cancer is utilized to evaluate, at preclinical level, the molecular, phenotypic, and functional in vivo effects of the sodium salt of a compound of Formula I-1 (SGI-110), administered alone or combined with anti-murine immunostimulatory mAb.

The study analyses: (a) the therapeutic effectiveness of combined administration of SGI-110 and immunostimulatory mAb in murine cancer; and (b) the involvement of host immune response in the potential anti-tumour effects of the most effective therapeutic administration of SGI-110 and immunostimulatory mAb.

### In vitro immunomodulatory activity SGI-110 in murine cancer

Preliminary *in vitro* experiments will be carried out to study the immunomodulatory effects of SGI-110 on the murine mammary carcinoma cells, TS/A, selected for their immunophenotype, growth rate and tumour take in mice. Cells are *in vitro* treated according to a standard schedule and RT-PCR and Real-Time quantitative RT-PCR analyses investigate the efficacy of treatment to induce and/or up-regulate murine CTA (including P1A, Mage-a family) in cancer cells.

### Therapeutic efficacy of SGI-110 in combination with immunostimulatory mAb in mouse cancer

Immunostimulatory mAb treatment is given either concomitantly or subsequently to SGI-110 schedules according to the dosage regimen described below. BALB/c mice (6 per group) are grafted into the flank region with TS/A cells and treated with SGI-110, administered alone or in combination with the anti-murine CTLA-4 or anti-murine PD-1 mAbs. The effectiveness and tolerability of treatments are evaluated by tumour volume and body weight measurements, respectively.
- Day -6: Murine TS/A cells are inoculated subcutaneously into the flank region of all mice.
- Day 0: After a latency period of 7 days, mice bearing clearly palpable and visible tumour grafts (diameter ≥ 0.2 cm) are separated in different groups of treatment (6 animals per group).

Treatment schedule: subcutaneous (SQ) administration over 5 days ("SQ5")

| | |
|---|---|
| Group 1: | Vehicle qdx5 SQ at days 1-5 |
| Group 2: | SGI-110, 3mg/kg qdx5 SQ at days 1-5 |
| Group 3: | anti-murine CTLA-4 at days 2, 5, 8 |
| Group 4: | anti-murine CTLA-4 at days 8, 11, 14 |
| Group 5: | SGI-110, 3mg/kg qdx5 SQ (at days 1-5) + anti-murine CTLA-4 (at days 2, 5, 8) (concomitant schedule) |
| Group 6: | SGI-110, 3mg/kg qdx5 SQ (at days 1-5) + anti-murine CTLA-4 (at days 8, 11, 14) (subsequent schedule) |
| Group 7: | anti-murine PD-1 at days 2, 5, 8 |
| Group 8: | anti-murine PD-1 at days 8, 11, 14 |
| Group 9: | SGI-110, 3mg/kg qdx5 SQ (at days 1-5) + anti-murine PD-1 (at days 2, 5, 8) (concomitant schedule) |
| Group 10: | SGI-110, 3mg/kg qdx5 SQ (at days 1-5) + anti-murine PD-1 (at days 8, 11, 14) (subsequent schedule) |

Treatment schedule: i.p. weekly

| | |
|---|---|
| Group 11: | Vehicle (3 i.p. injections every 3 hours) at days 1 and 8 |
| Group 12: | SGI-110, 36.6 mg/kg day (3 i.p. injections every 3 hours) at days 1 and 8 |
| Group 13: | anti-murine CTLA-4 at days 3, 6, 9 |
| Group 14: | SGI-110, 36.6 mg/kg day (3 i.p. injections every 3 hours) at days 1 and 8 + anti-murine CTLA-4 (at days 3, 6, 9) |

### Therapeutic efficacy of the SQ5 schedule

Based on the results generated by *in vivo* administration of SGI-110 according to the SQ5 schedule (see above), a single dose of "SQ weekly" schedule of SGI-110 administration is tested in combination with the immunostimulatory mAb. MAb treatment is given either concomitantly or subsequently to SGI-110 schedules. To this end, BALB/c mice (6 per group) are grafted into the flank region with TS/A cells and treated with SGI-110, administered alone or in combination with the anti-murine CTLA-4 or anti-murine PD-1 mAbs. The effectiveness and tolerability of treatments is evaluated by tumour volume and body weight measurements, respectively.

Treatment schedule: SQ weekly.

| | |
|---|---|
| Group 1: | Vehicle weekly SQ at days 1, 8, 15 |
| Group 2: | SGI-110, 24.4 mg/kg weekly SQ at days 1, 8, 15 |
| Group 3: | anti-murine CTLA-4 at days 3, 6, 9 |
| Group 4: | anti-murine CTLA-4 at days 17, 20, 23 |
| Group 5: | SGI-110, 24.4 mg/kg weekly SQ (at days 1, 8, 15) + anti-murine CTLA-4 (at days 3, 6, 9) (concomitant schedule) |
| Group 6: | SGI-110, 24.4 mg/kg weekly SQ (at days 1, 8, 15) + anti-murine CTLA-4 (at days 17, 20, 23) (subsequent schedule) |
| Group 7: | anti-murine PD-1 at days 3, 6, 9 |
| Group 8: | anti-murine PD-1 at days 17, 20, 23 |
| Group 9: | SGI-110, 24.4 mg/kg weekly SQ (at days 1, 8, 15) + anti-murine PD-1 (at days 3, 6, 9) (concomitant schedule) |
| Group 10: | SGI-110, 24.4 mg/kg weekly SQ (at days 1, 8, 15) + anti-murine PD-1 (at days 17, 20, 23) (subsequent schedule) |

Treatment schedule: i.p. weekly.

| | |
|---|---|
| Group 11: | Vehicle (3 i.p. injections every 3 hours) at days 1 and 8 |
| Group 12: | SGI-110, 36.6 mg/kg day (3 i.p. injections every 3 hours) at days 1 and 8 |
| Group 13: | SGI-110, 36.6 mg/kg day (3 i.p. injections every 3 hours) at days 1 and 8 + anti-murine CTLA-4 (at days 3, 6, 9) |

### Molecular, phenotypic and functional correlates of SGI-110 combined with immunostimulatory mAb

The modifications induced *in vivo* by the most effective therapeutic regimen utilizing SGI-110, combined with the immunostimulatory mAb is investigated on both tumours and host's immune compartments. To this end, based on the results generated in Step 2, BALB/c mice (6 per group) are grafted into the flank region with TS/A cells and treated with the most effective therapeutic regimen of SGI-110, administered in combination with one immunostimulatory mAb.

DNA hypomethylation, as well as modulation of immune profile of murine tumour tissues, excised from control and treated mice, is evaluated by molecular assays (including quantitative methylation-specific PCR, RT-PCR and real-time quantitative RT-PCR analyses). Immune infiltrates of neoplastic tissues is characterized for presence and relative frequency of activated T cells by immunohistochemistry (IHC). Furthermore, normal tissues from control and treated mice are surgically removed and adequately conserved for following experimental analyses (see below).

### In vivo immunomodulatory effects of combined administration of SGI-110 and immunostimulatory mAb, in benign tissues

The association of the *in vivo* immunomodulatory activity with systemic autoimmunity phenomena (including the induction and/or modulation of immune-related genes in normal tissues) is investigated.

The presence and levels of expression of murine CTA (i.e. P1A, Mage-a family) are evaluated by RT-PCR and real time quantitative RT-PCR analyses in at least 4 benign samples (including the heart, lung, liver, intestine, kidney, muscle and skin). Furthermore, immune infiltrates of normal tissues are characterized for presence and relative frequency of activated T cells by IHC.

### Contribution of immune response to the anti-tumour activity of the investigated combination therapies

The involvement of the host immune response in the potential anti-tumour effects of the selected combined regimen is investigated. Both immunocompetent (i.e. BALB/c) and immunodeficient (i.e. T cell-deficient athymic nude mice and T-cell-, B-cell- and natural killer cell-deficient SCID/Beige) mouse strains (6 mice per group) are grafted into the flank region with TS/A cells and treated with the chosen therapeutic regimen. The effectiveness of treatment is evaluated by tumour volume assessment, which comparative analysis allows to determine the contribution of T, B and NK cell immunity to the presumed anti-tumour activity of combined chemo-immunotherapies.

Modulation of anti-tumour T cell response can be evaluated through MLTC, cell proliferation, IFN-g release and/or cytotoxicity assays.

### EXAMPLE 2: Inhibition of DNA Methylation by Compounds of the Invention

The demethylating activity of the compounds was tested in a cell-based green fluorescent protein (GFP) assay. In the assay, a decrease in methylation resulting from exposure to a methylation inhibitor leads to GFP expression, and is readily scored.

The CMV-EE210 cell line containing the epigenetically silenced GFP transgene was used to assay for reactivation of GFP expression by flow cytometry. CMV-EE210 was made by transfecting NIH 3T3 cells with the pTR-UF/UF1/UF2 plasmid, which contained pBS(+) (Stratagene, Inc.) with a cytomegalovirus (CMV) promoter driving a humanized GFP gene adapted for expression in mammalian cells. After transfection, high-level GFP expressing cells were initially selected by FACS analysis and sorting using a MoFlo cytometer (Cytomation, Inc.).

Decitabine, a potent inhibitor of mammalian DNMT1, was used as a positive control. To screen for reactivation of CMV-EE210, decitabine (1 µM) or a test compound (30-50 µM) was added to complete medium (phenol red free DMEM (Gibco, Life Technologies) supplemented with 10% foetal bovine serum (Hyclone)). Cells were then seeded to 30% confluence (∼5000 cell/well) in a 96-well plate containing the test compounds, and grown for three days in at 37 °C. in 5% CO₂.

The plates were examined under a fluorescent microscope using a 450-490 excitation filter (13 filter cube, Leica, Deerfield Ill.). Wells were scored g1 positive, g2 positive, or g3 if GFP was expressed in 10%, 30%, >75% of viable cells, respectively.

Table 1 provides the results of the test for decitabine and the test compounds as DNA methylation inhibitors. GFP₅₀ is the concentration of an inhibitor at which the Green Fluorescent Protein (GFP) expression level is reduced from g3 to g1/2. Table 1 demonstrates that the tested compounds were inhibited DNA methylation effectively at low concentrations, resulting in reactivation of GFP gene transcription.

**TABLE 1.**

| | Compound | GFP Expression Level | GFP₅₀ (nM) |
|---|---|---|---|
| | Decitabine | g3 | 500 |
| I-1: | 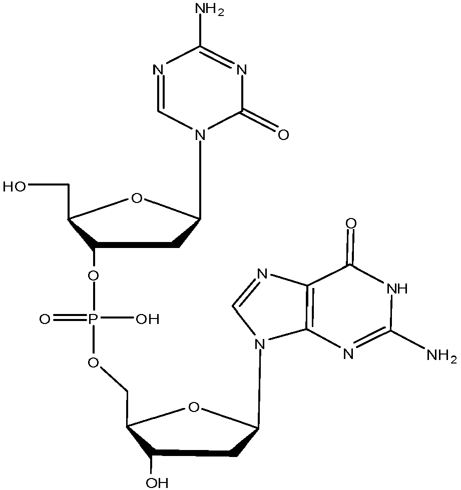 | g3 | 400 |
| 1-2: | 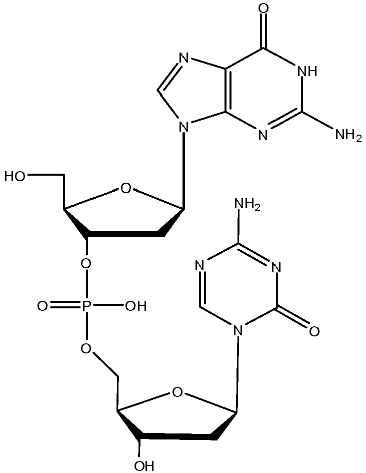 | g3 | 700 |

### EXAMPLE 3: Stability of a Representative Compound in Solvent Formulations.

The stability of a compound of the invention in various formulations under various storage conditions was investigated. Stability was determined by HPLC at the designated time intervals. The results are summarized in Table 2 for formulations comprising a sodium salt of compound I-1 (i.e. SGI-110):

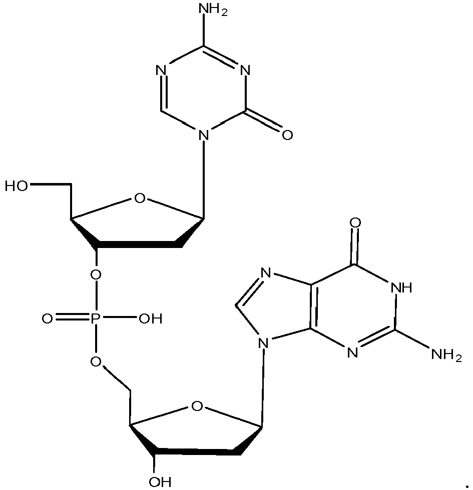

**TABLE 2.**

| Formulation | Storage Conditions | Time Point | Percent compound detected | % decomposition per hour |
|---|---|---|---|---|
| water, pH 7.0 | 2-8 °C | 0 | 95.8% | |
| | | 5 hours | 95.1% | 0.14 |
| water, pH 7.0 | Room temperature | 0 | 95.8% | |
| | | 5 hours | 90.4% | 1.1 |
| DMSO / water (1:1, w/w) | 25 °C/60% relative humidity | 0 | 93.7% | |
| | | 5 hours | 90.1% | 0.72 |
| DMSO / water (3:1, w/w) | 25 °C/60% relative humidity | 0 | 96.6% | |
| | | 24 hours | 94.2% | 0.10 |
| Propylene glycol / Glycerin (70:30, v/v) | Room temperature | 0 | 96.8% | 0.021 |
| | | 24 hours | 96.3% | |
| Propylene Glycol / Glycerin / Ethanol (65:25:10, w/w/w) | 2-8 °C | 0 | 95.8% | |
| | | 3 months | 95.1% | 0.00032 |
| | 25 °C / 60% relative humidity | 0 | 95.8% | |
| | | 3 months | 67.6% | 0.013 |

Solution of SGI-110 in water at pH 7, the pH at which compounds of this class are most stable, led to rapid decomposition in a few hours, even at lower temperatures making it unsuitable for manufacturing process. Use of DMSO / water (1:1) gave slightly better results at higher temperatures. A slight improvement was noted in using 3:1 DMSO / water formulation. The said compound is stable in anhydrous DMSO, therefore a solvent of choice for manufacturing process.

In regard to selection of pharmaceutically acceptable solvents for final formulation ready for administration, the anhydrous propylene glycol / glycerin system provided better stability. The final formulation was prepared by substituting small amounts of propylene glycol and glycerin with ethanol, to provide propylene glycol / glycerin / ethanol (65:25:10). This formulation was the only one of several tested that provided a great improvement in the solubility and stability of the compound at both higher and lower temperatures.

Based on the experiments conducted in water, a 10-fold improvement in stability could have been expected upon changing from room temperature to colder (2-8 °C) storage conditions. However, in the propylene glycol / glycerin / ethanol (65:25:10) system, changing from warmer to colder storage conditions provided a 40-fold improvement in stability. The combined effects of cooling plus the addition of ethanol to the propylene glycol / glycerin system provided a 66-fold improvement in stability. Such great improvements in the stability of SGI-110 during storage could not have been expected.

The propylene glycol / glycerin / ethanol (65:25:10) system provided SGI-110 as a solution, which was smooth, free-flowing, and suitable for passage through a 23-gague needle without complications or clogging. The maximum solubility of the compound in this medium was determined to be about 130-150 mg/mL, which compares favourably to the aqueous solubility of 20 mg/mL. The good chemical stability taken together with the excellent solubility identified the glycol / glycerin / ethanol (65:25:10) system as a formulation for use in animal experiments.

### EXAMPLE 4: Animal Studies with the Formulation of EXAMPLE 3

The glycol / glycerin / ethanol (65:25:10) formulation of EXAMPLE 3, containing 100 mg/mL free base equivalent of the sodium salt of compound I-1 was administered to live animals. An analogous decitabine formulation was used for comparison (50 mg lyophilized decitabine powder vial reconstituted to 10 mg/mL with water for injection and administered as infusions by diluting in infusion bags).

Administration of a single dose of the formulations to monkeys (10 mg/kg) produced higher physiological concentrations of compound I-1 (Cₘₐₓ 1,130 ng/mL; AUC of 1,469 ng•hr/mL) than of decitabine (Cₘₐₓ 160 ng/mL; AUC of 340 ng•hr/mL).

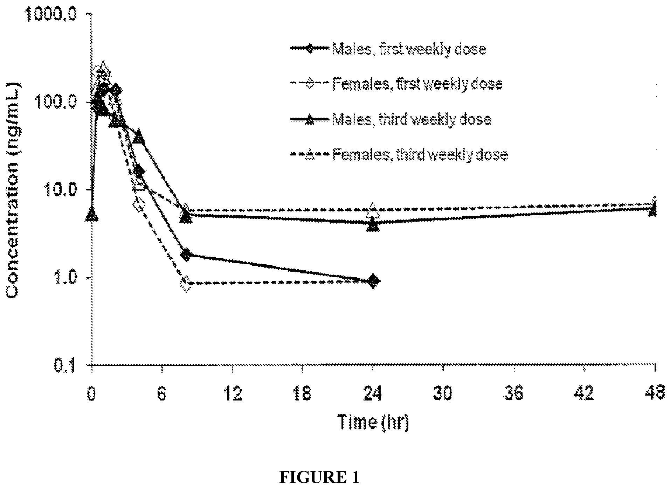

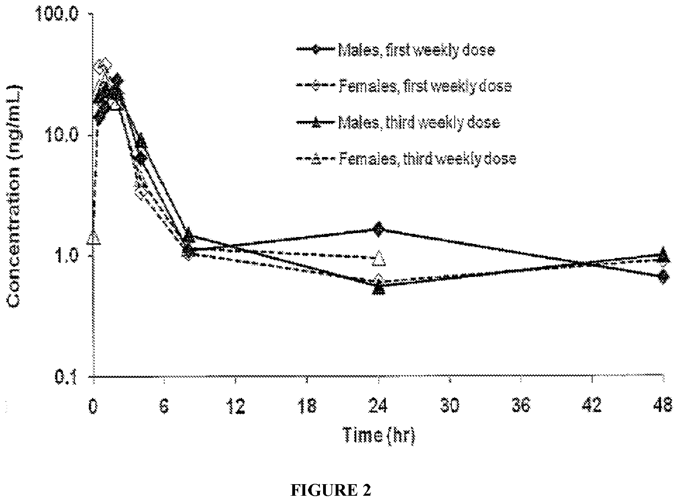

In a repeat dose study, monkeys were dosed 3x weekly subcutaneously (3 mg/kg). At day 15, the systemic exposure to compound I-1 (Cₘₐₓ 181 ng/mL; AUC of 592 ng•hr/mL) was greater than that of decitabine (Cₘₐₓ 28 ng/mL; AUC of 99 ng•hr/mL). The pharmacokinetic parameters of the compounds did not vary significantly over the 22-day observation period, and minimal accumulation was detected. (FIGURES 1 and 2.) Pharmacodynamic properties (not shown) were monitored and were acceptable. Blood samples were drawn periodically to assay LINE-1 DNA methylation.

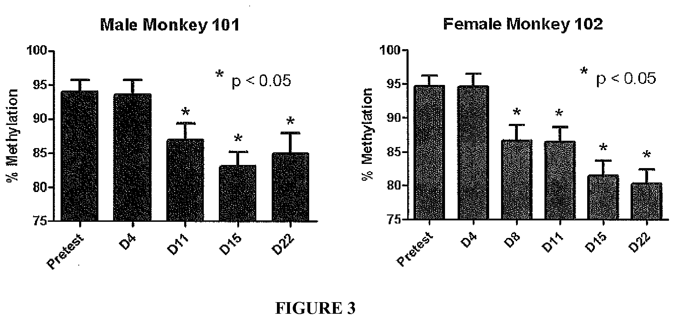

Decreases in LINE-1 DNA methylation, the indicator of biological activity, were observed, and the decrease continued until termination of the study on day 22. The observed LINE-1 methylation was significantly different (ρ < 0.05) from the methylation level observed prior to initial dosing. (FIGURE 3.)

The formulation was well-tolerated in the species tested. Three regimens were evaluated: a) once daily subcutaneous dose in rats and rabbits for 5 days; b) once weekly subcutaneous dose in rabbits and cynomolgus monkeys for 28 days as tolerated; and c) twice weekly subcutaneous dose in rats for 28 days as tolerated. Rabbits tolerated the 5-day regimen well, up to a dose of 1.5 mg/kg/day, which is equivalent to 18 mg/kg/day in humans, and the weekly regimen up to a dose of 1.5 mg/kg/week for 3 weeks.

Cynomolgus monkeys tolerated the weekly regimen well, up to a dose of 3.0 mg/kg/week for 3 weeks, which is equivalent to 36 mg/kg/week. Rats tolerated much higher doses: 30 mg/kg/day over 5 days; and 20 mg/kg twice weekly for 4 weeks.

The main toxicity in all experiments was myelosuppression. However, the subcutaneous formulation tested exhibited less myelosuppression and faster recovery.

### EXAMPLE 5: Preparation of a kit for use according to the invention

### First vessel: Compound of formula 1-1 for Injection, 100 mg

The sodium salt of the compound of the formula: 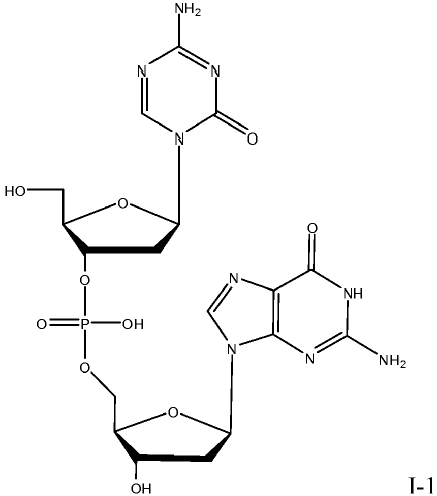 (also referred to herein as "SGI-110") was prepared as described in US 7700567 by coupling Is (where R₁ = carbamate protective group) with phosphoramidite building block **1d:**

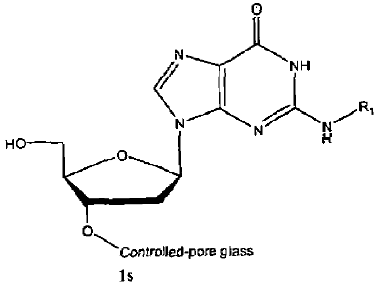

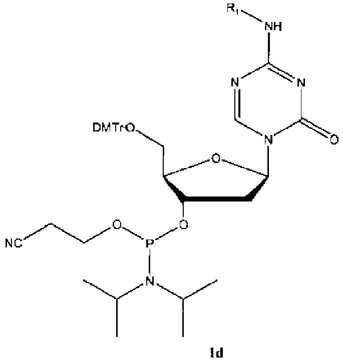

A protected 2'-deoxyguanosine-linked CPG solid support **Is** (where R₁ = tert-butyl phenoxyacetyl) is coupled with 2-2.5 equivalents of phenoxyacetyl decitabine phosphoramidite (**1d**, where R₁ = phenoxyacetyl) in the presence of 60% of 0.3 M benzylthiotetrazole activator (in acetonitrile) for 10 minutes. The CPG solid support containing protected DpG dinucleotide is treated with 20 mL of 50 mM K₂CO₃ in methanol for 1 hour and 20 minutes. The coupled product is oxidized, protective group removed, washed, filtered, and purified by the ÄKTA Explorer 100 HPLC with a Gemini C18 preparative column (Phenomenex), 250x21.2 mm, 10µm with guard column (Phenomenex), 50x21.2mm, 10µm, with 50 mM triethylammonium acetate (pH 7) in MilliQ water (Mobile Phase A) and 80% acetonitrile in MilliQ water (Mobile Phase B), with 2% to 20/25% Mobile Phase B in column volumes.

The ESI-MS (-ve) of DpG dinucleotide **2b:** 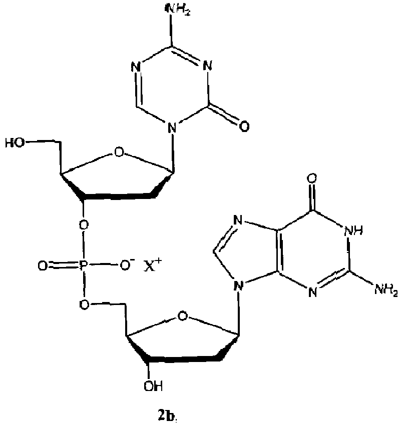 where X⁺ = triethylammonium (calculated exact mass for the neutral compound C₁₈H₂₄N₉O₁₀P is 557.14), exhibited m/z 556.1 [M-H]⁻ and 1113.1 for [2M-H]⁻ (see mass spectrum in Figure 31 of US 7700567).

The sodium salt of the compound of formula I-1 (i.e. DpG dinucleotide 2b, where X⁺ = sodium; SGI-110) is obtained by re-dissolving the triethylammonium salt in 4 ml water, 0.2 ml 2M NaClO₄ solution. When 36 mL acetone is added, the dinucleotide precipitates. The solution is kept at -20°C for several hours and centrifugated at 4000 rpm for 20 minutes. The supernatant is discarded and the solid is washed with 30 mL acetone followed by an additional centrifugation at 4000 rpm for 20 minutes. The precipitate is dissolved in water and freeze dried, which exhibited m/z 556.0 [M-H]⁻ (see mass spectrum in Figure 36 of US 7700567).

### Compounding and filling of bulk formulation

Based on the assay value of SGI-110 lot, needed quantities of SGI-110 and DMSO are calculated and weighed appropriately for the intended batch scale.
2. SGI-110 is dissolved in DMSO utilizing an overhead mixer in an appropriately sized stainless steel (SS) vessel.
3. Upon complete solubilization of the drug in DMSO, samples of the bulk solution are tested using a UV or HPLC in-process method to determine that the amount of SGI-110 is within 95-105% of the target concentration.
4. Bulk solution is filtered through a series of two pre-sterilized 0.2 micron sterilizing filters that are DMSO compatible, and collected into a 2L SS surge vessel.

Filtration rate is continuously adjusted by visual monitoring of quantity available for filling in the surge vessel.

One gram of the filtered bulk solution is filled into each of the 5 cc depyrogenated, clear glass vials and the operation is continued with until all of the filtered bulk solution is filled.

Each vial is automatically and partially stoppered on the fill line with a fluoropolymer coated, chlorobutyl rubber lyo stopper that is pre-sterilized.

Product vials are transferred to lyophilizer under aseptic transfer conditions for initiation of lyophilization cycle.

### Lyophilization and capping of vials

Vials are lyophilized using the cycle parameters as below.

| **Freezing** | **Primary/Secondary Drying** | | | | | **Final Set point (stoppering conditions)** |
|---|---|---|---|---|---|---|
| Temperature | -40°C | -5°C | 10°C | 30°C | 60°C | 25°C |
| Ramp time (min) | 133 | 117 | 50 | 67 | 100 | - |
| Time (min.) | 360 | 1440 | 1440 | 1440 | 1440 | hold |
| Vacuum (mTorr) | - (note: 100 mT for evacuation at -50°C) | 100 | 100 | 50 | 50 | 50 mT before back fill |

2. Upon completion of the lyo cycle, lyophilizer is back filled with nitrogen, and the vials are completely and automatically stoppered.
3. Vials are aseptically transferred to an isolator where each of the vials is automatically capped with a blue aluminum flip-off cap.
4. Vials are visually inspected before proceeding with sampling for release testing, and the labeling and packaging operation. Vials are kept at 2-8°C until ready.

### Labeling and Packaging

Each vial is labeled per approved content, and packaged individually into heat-sealed aluminum foil pouch with a desiccant under vacuum. The foil pouch is labeled outside with the same label as was used for the product vial. Labeled and packaged vials are stored at 2-8°C until further distribution.

### Residual DMSO

Four batches of the same scale of 3000 vials/batch were prepared using the same process as described above. DMSO was consistently removed to the following residual levels to yield a solid white powder, demonstrating that lyophilization of SGI-110 out of DMSO as described above yields a safe and chemically stable SGI-110 powder:

| # | DMSO in mg/vial |
|---|---|
| Batch 1 | 25 |
| Batch 2 | 28 |
| Batch 3 | 27 |
| Batch 4 | 29 |

### Second vessel: SGI-110 Diluent for Reconstitution, 3 mL

### Compounding and filling of bulk formulation

Calculated quantities (see table below) of propylene glycol, ethanol, and glycerin in the aforementioned order are added into an appropriately sized stainless steel vessel equipped with an overhead mixer.

| | % of each ingredient | Grade | Function |
|---|---|---|---|
| Propylene glycol | 65 | NF, PhEur | Solvent |
| Glycerin | 25 | NF, PhEur | solvent |
| Alcohol/Ethanol | 10 | USP, PhEur | Thinning agent |

2. Intermittent mixing during addition of components is followed by at least 30 minutes of mixing to yield a well-mixed solution.
3. Bulk solution is filtered through a series of two pre-sterilized 0.2 micron compatible sterilizing filters, and collected into a 2L SS surge vessel.
4. Filtration rate is adjusted by visual monitoring of quantity available for filling in the surge vessel.

At least 3.15 g, equivalent to 3.0 mL, of the filtered bulk solution is filled into each of the 5 cc depyrogenated, clear glass vials followed by automatic stoppering using fluoropolymer coated chlorobutyl rubber closures.

Stoppered vials are capped with sterilized white aluminum flip-off caps.

Vials are visually inspected prior to sampling for the release testing and labeling operation and are stored at 2-30°C until ready.

### Labeling and Packaging

Each diluent vial is labeled per approved content. Labeled vials are stored at 2-30°C until further distribution.

### EXAMPLE 6: Anti-tumour activity of SGI-110 in combination with anti-CTLA-4 antibody

The anti-tumor effect of SGI-110 in combination with anti-mouse CTLA-4 was evaluated in murine breast cancer model.

### Materials and Methods

TS/A is a murine mammary carcinoma originated from a moderately differentiated, weakly immunogenic mammary adenocarcinoma spontaneously arising in a 20-mo-old female BALB/c mouse.

The anti-mouse CTLA-4 was the CTLA-4 mAb 9H10, obtained from BioXCell (West Lebanon, NH, USA), dosed at 100µg/mouse in 200µl (ip).

Balb/c mice (6/group) were injected SQ in the flank region with murine mammary carcinoma cells TS/A (2×10⁵).

Mice bearing palpable tumor grafts (diameter ≥ 0.2 cm) were injected subcutaneously with 3mg/kg of reconstituted SGI-110 QDx5 (daily for five days) at days 1-5, alone or in combination with 100µg/hamster anti-murine CTLA-4 monoclonal antibodies (mAb), either concurrently (at days 2, 5 and 8) or subsequently (at days 8, 11 and 14).

Control mice were injected with diluent for reconstitution. The effectiveness and tolerability of treatments was evaluated by tumor volume and body weight measurements, respectively.

### Results and conclusions

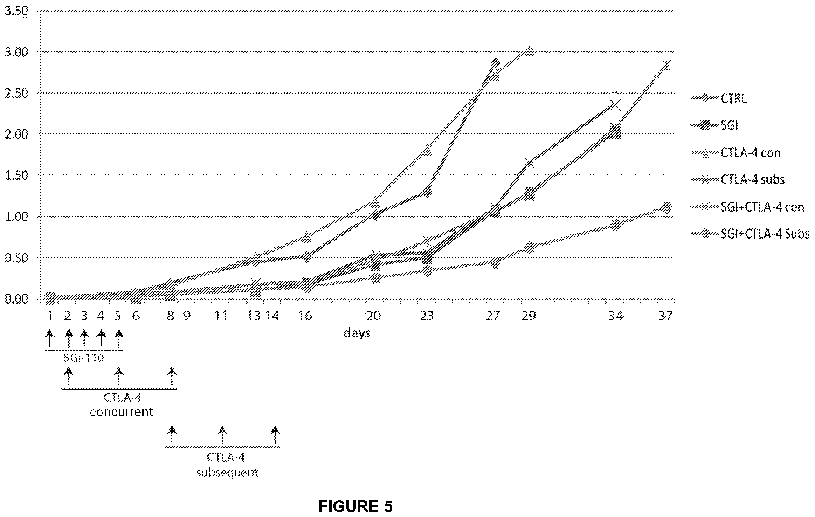

The results are shown in Figure 5. The best antitumour effect was achieved in mice treated with SGI-110 followed by anti-CTLA-4 mAb. In this case, a tumour mass significantly (p<0.05) smaller than that of control mice was observed, indicating, at day 26, a tumor growth inhibition of 84.4%. Moreover, a significant, but lower, reduction in tumor mass occurred in SGI-110-treated mice as compared to control mice, with a tumor growth inhibition of 62.9% at days 26. No difference in tumor growth inhibition was observed in mice treated with SGI-110 combined with anti-CTLA-4 mAb given concomitantly as compared to mice treated with SGI-110 alone. No loss in body weight was observed (data not shown) in all mice investigated, demonstrating a good tolerability of all therapeutic regimens tested.

Thus, epigenetic priming with SGI-110 followed by CTLA-4 blockade provides improved antitumour activity.

### EXAMPLE 7: Anti-tumour activity of two cycles of sequential SGI-110 and anti-CTLA-4

The anti-tumor effect of two cycles of sequential administration of SGI-110 followed by anti-mouse CTLA-4 mAb 9H10 was also evaluated in TS/A murine model.

### Materials and Methods

Balb/c mice (6/group) were injected SQ in the flank region with murine mammary carcinoma cells TS/A (2×10⁵).

Mice bearing palpable tumor grafts (diameter ≥ 0.2 cm) were treated with two cycles of 3mg/kg of reconstituted SGI-110 QDx5 (injected subcutaneously daily for five days) at days 1-5 and 21-25, alone or in combination with two subsequent cycles of 100µg/hamster anti-murine CTLA-4 monoclonal antibodies (mAb), at days 8, 11, 14, 28, 31 and 34.

Control mice were injected with diluent for reconstitution. The effectiveness and tolerability of treatments was evaluated by tumor volume and body weight measurements, respectively.

### Results and conclusions

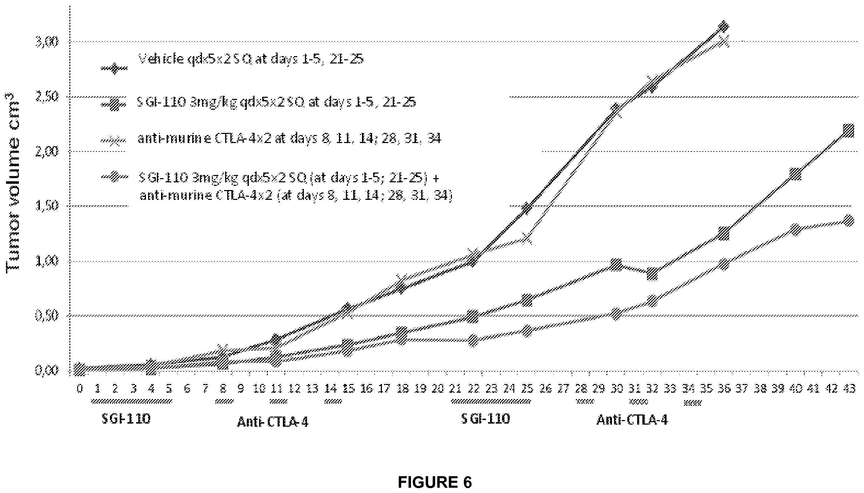

The results are shown in Figure 6: two cycles of sequential administration of SGI-110 and anti-CTLA4 antibody is efficacious and enhances the antitumour effect of the antibody. Body weight measurements (not shown) showed that the treatment was well-tolerated.

## Claims

1. A combination comprising the following separate components:
(i) a compound of Formula I or a pharmaceutically acceptable salt thereof:
(5-azacytosine group)-L-(guanine group) (I)
wherein L is a phosphorous-containing linker of Formula (II): 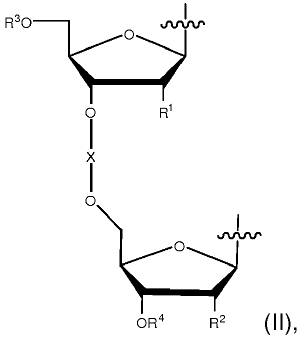 wherein, R¹ and R² are independently H, OH, an alkoxy group, an alkoxyalkoxy group, an acyloxy group, a carbonate group, a carbamate group, or a halogen; R³ is H, or R³ together with the oxygen atom to which R³ is bound forms an ether, an ester, a carbonate, or a carbamate; R⁴ is H, or R⁴ together with the oxygen atom to which R⁴ is bound forms an ether, an ester, a carbonate, or a carbamate; and X together with the oxygen atoms to which X is bound forms a phosphodiester, a phosphorothioate diester, a boranophosphate diester, or a methylphosphonate diester; and
(ii) one or more ancillary therapeutic component(s), wherein said ancillary therapeutic component is a T-cell activating agent;
wherein said compound of Formula I, or pharmaceutically-acceptable salt thereof, is for administration before said ancillary therapeutic component.

2. The combination of claim 1, wherein R¹ and R² are independently H, OH, OMe, OEt, OCH₂CH₂OMe, OBn, or F, and preferably R¹ and R² are H.

3. The combination of claim 1 or claim 2 wherein X together with the oxygen atoms to which X is bound forms a phosphodiester.

4. The combination of any one of the preceding claims, wherein the compound of Formula I is any one of I-(1-44): 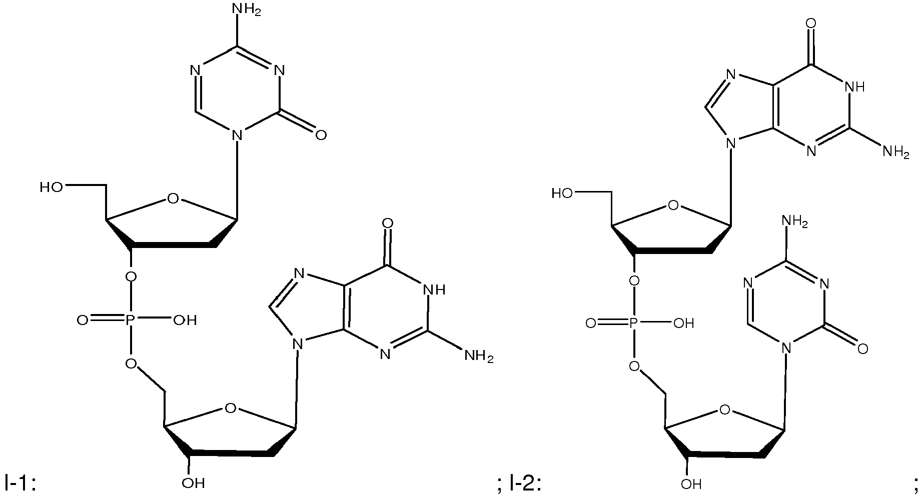 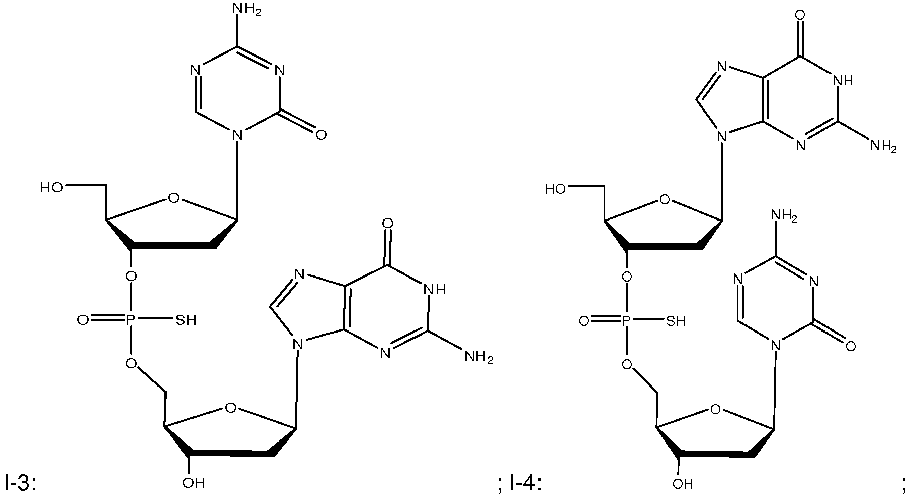 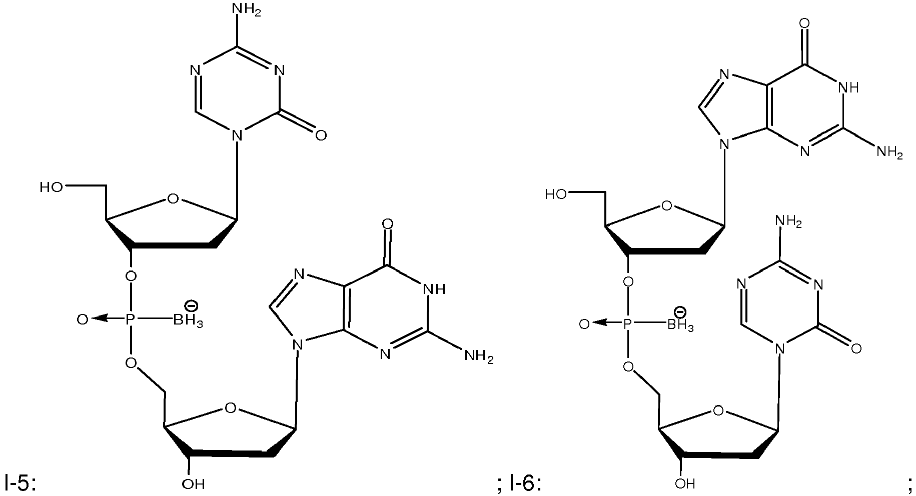 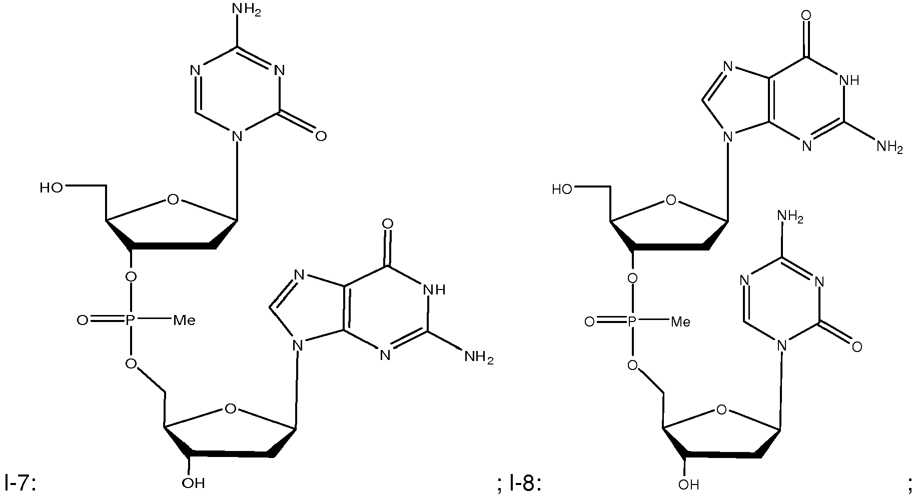 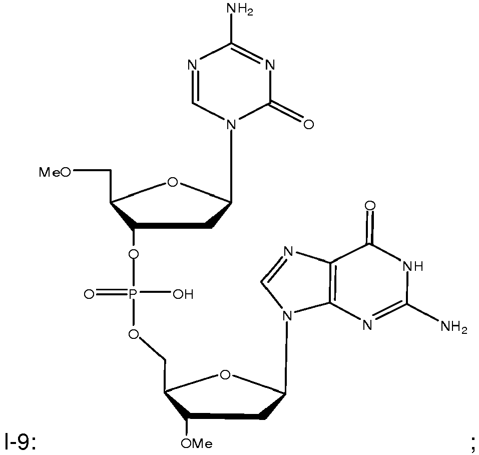 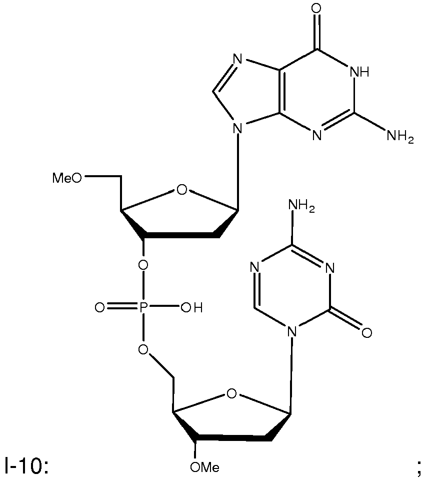 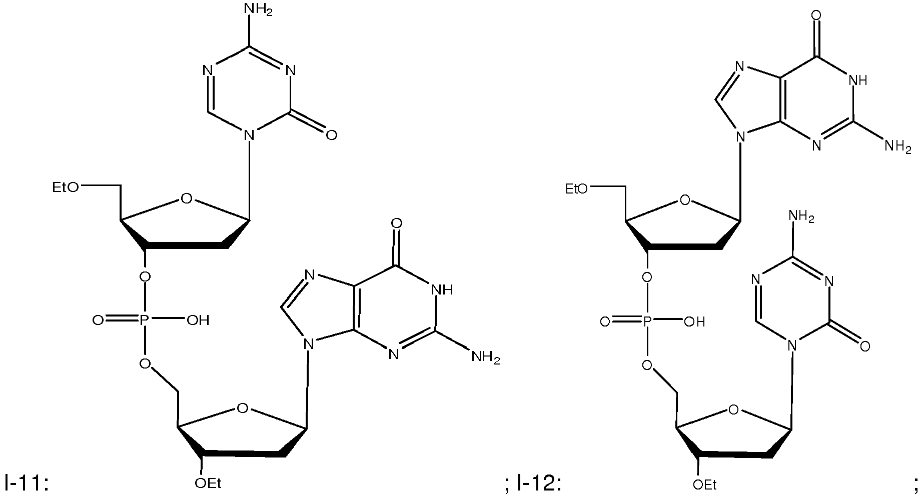 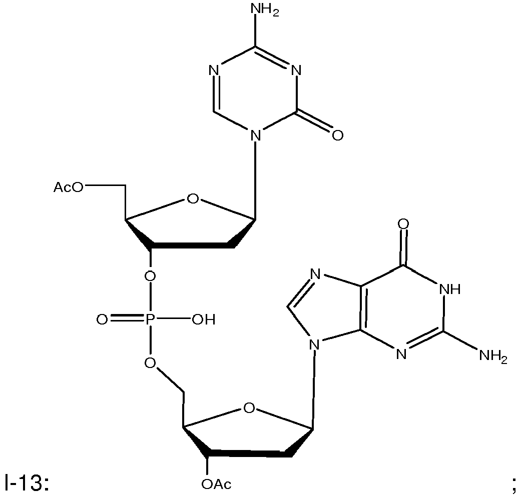 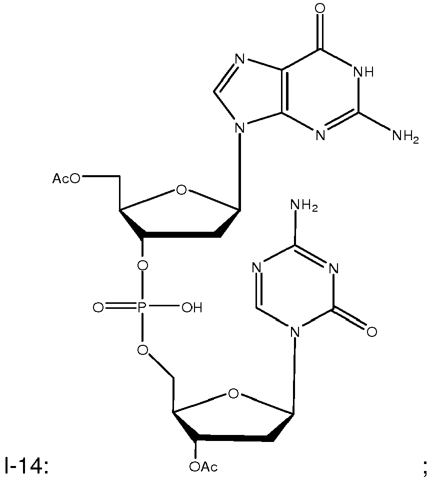 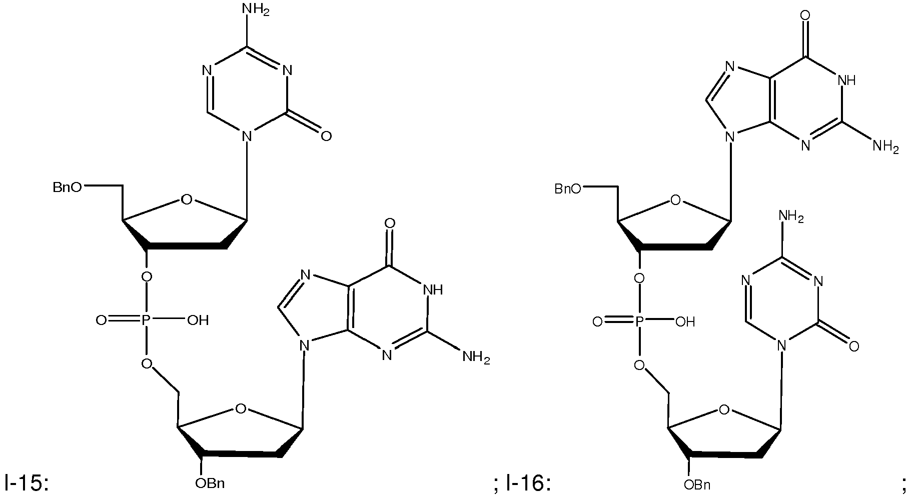 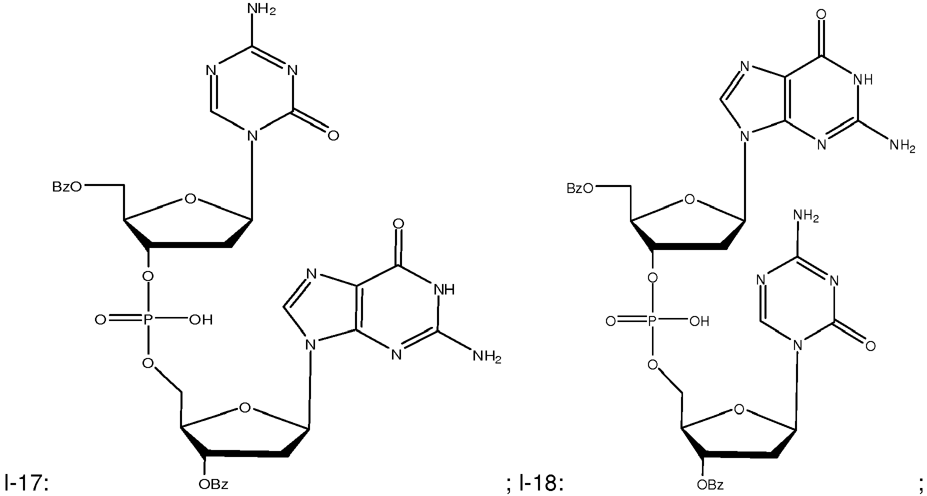 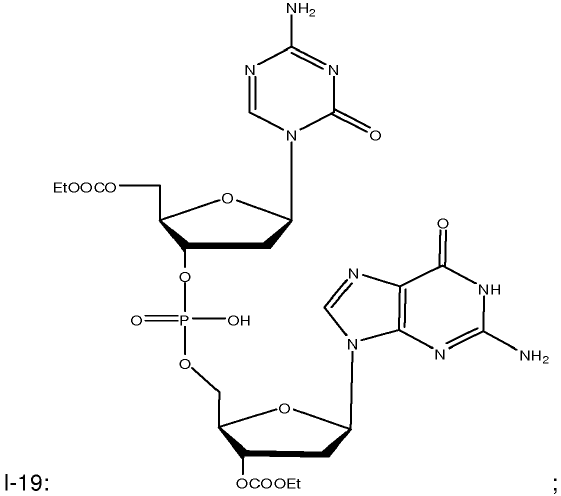 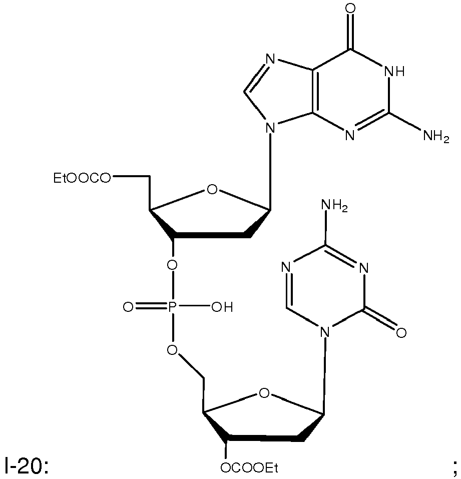 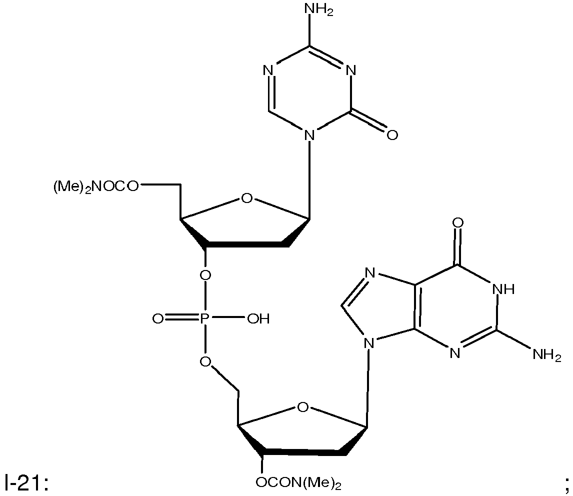 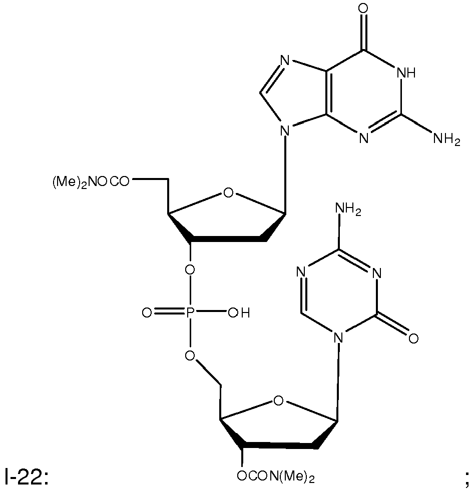 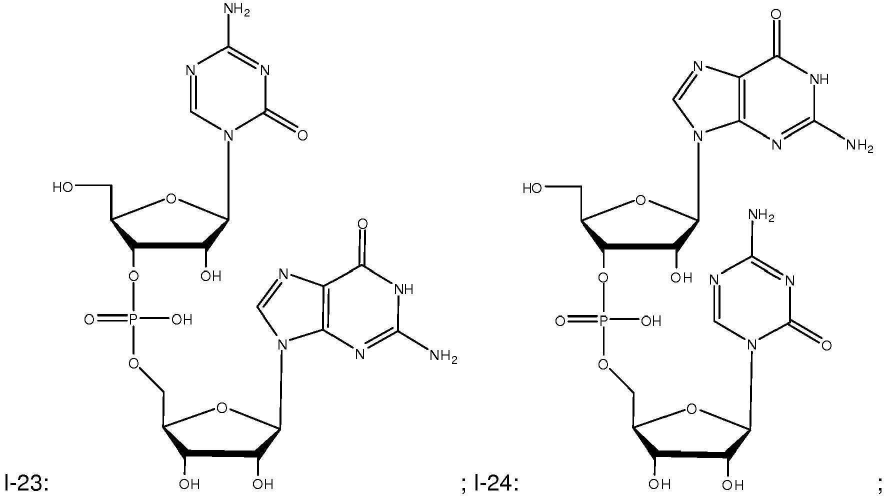 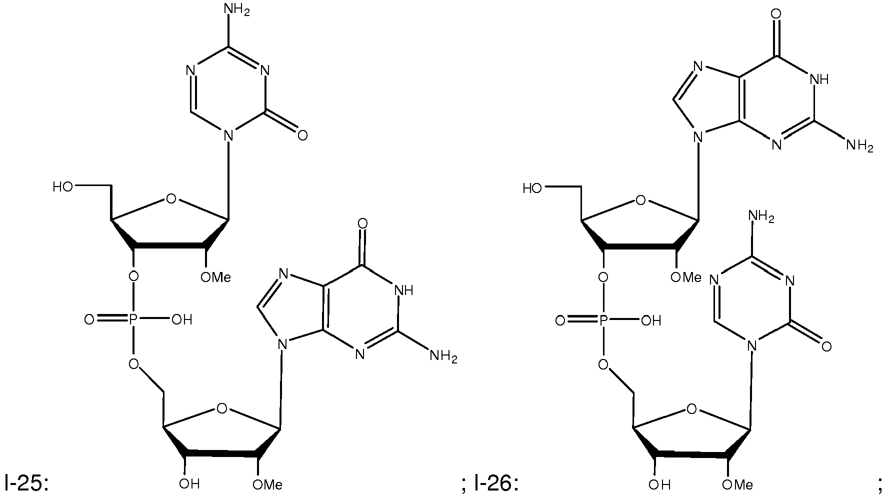 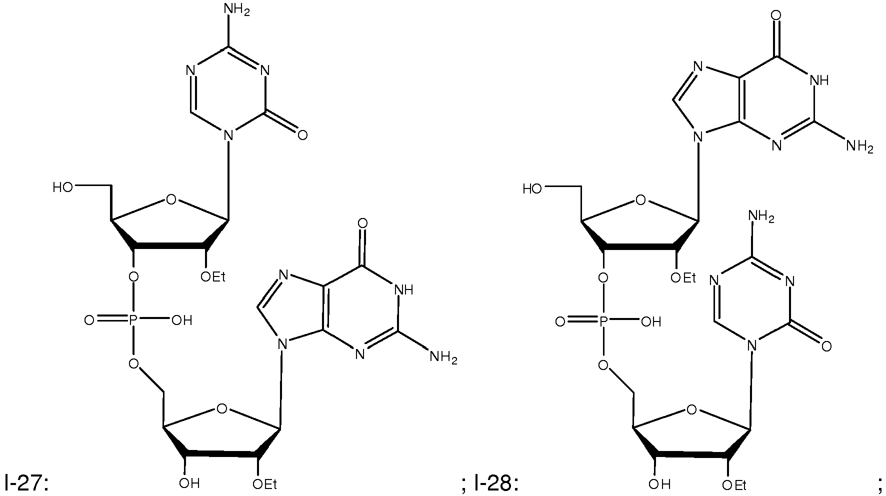 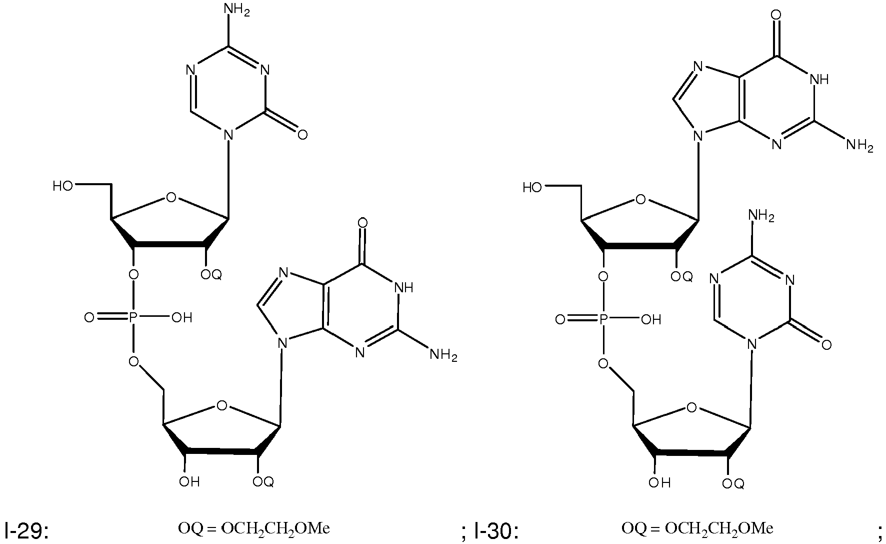 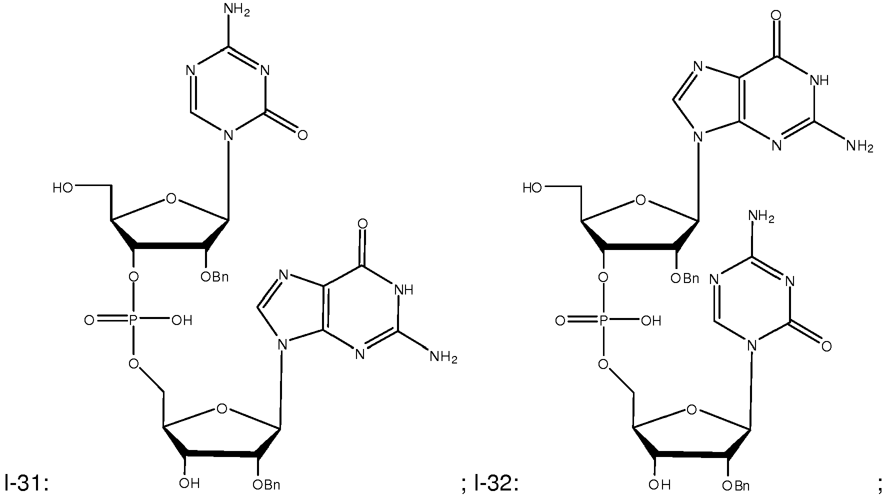 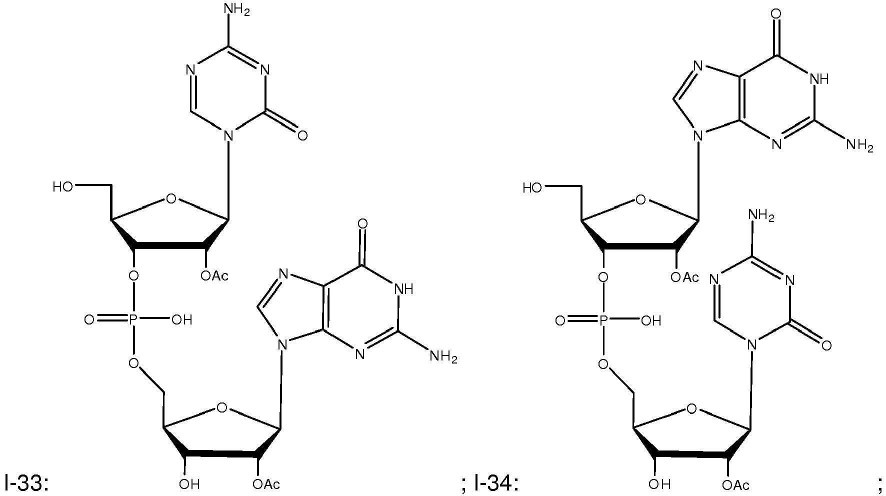 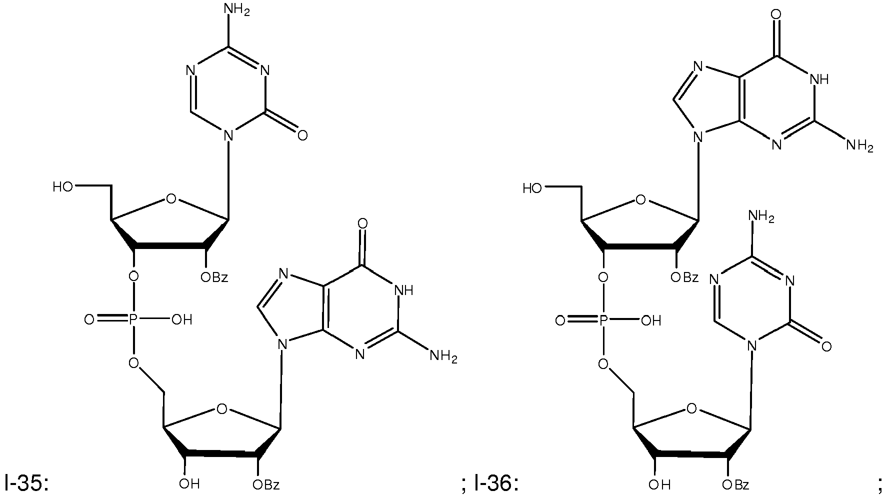 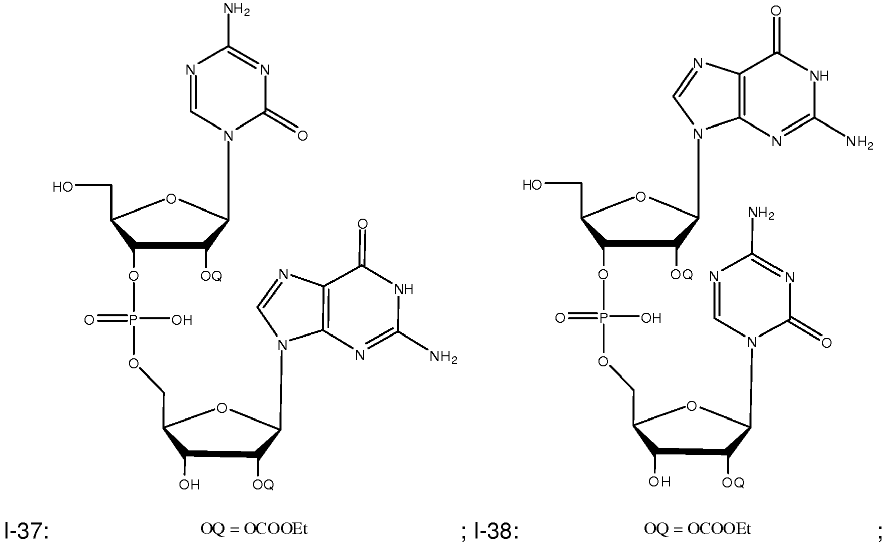 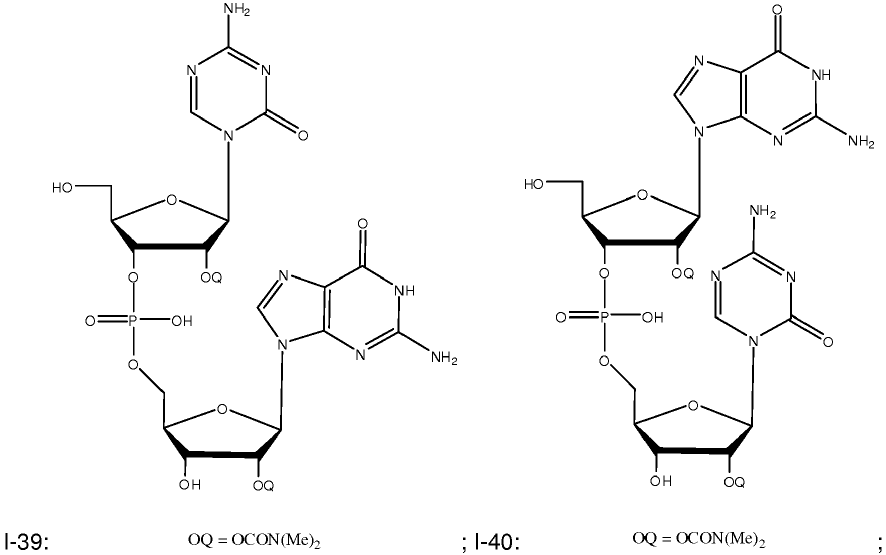 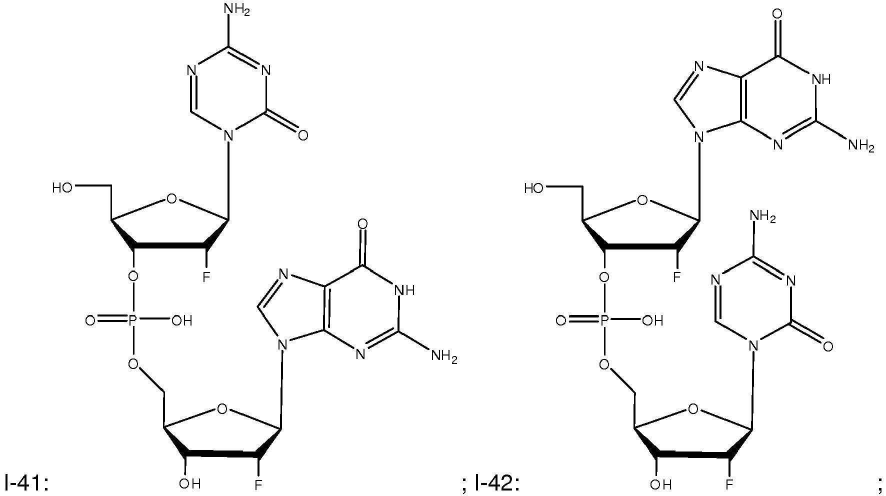 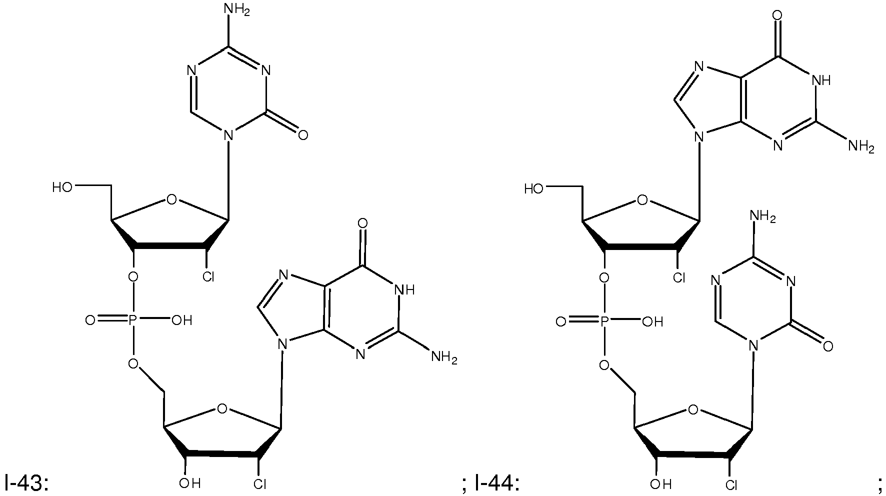 and pharmaceutically-acceptable salts of thereof.

5. The combination of any one of the preceding claims, wherein the compound of Formula I is:

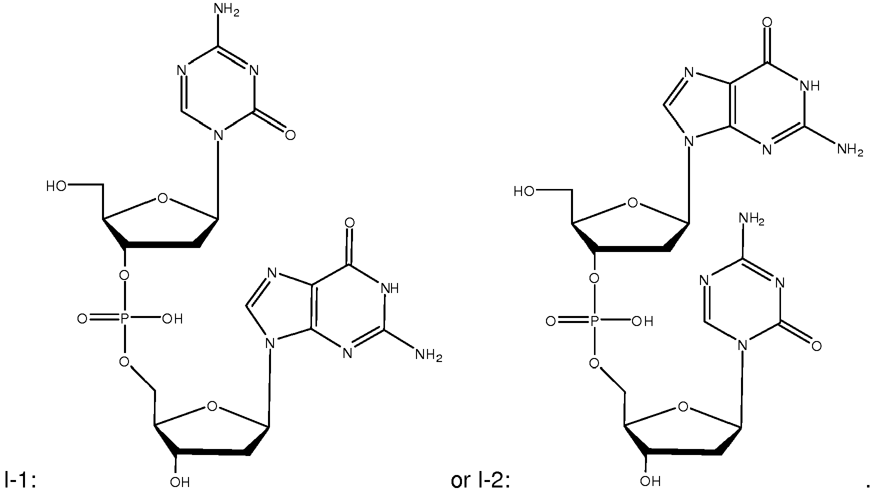

6. The combination of any one of the preceding claims wherein the compound of formula I is of the formula: 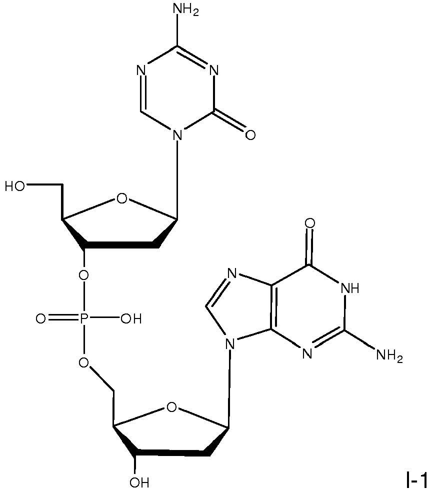 or a pharmaceutically-acceptable salt thereof.

7. The combination of claim 6 wherein said salt is a sodium salt.

8. The combination of any one of the preceding claims wherein the compound of Formula I or salt thereof is in the form of a formulation, being dissolved in a substantially anhydrous solvent comprising 45% to 85% propylene glycol; 5% to 45% glycerin; and 0% to 30% ethanol.

9. The combination of any preceding claim wherein the compound of Formula I or salt thereof is present in the formulation at a concentration of 80 mg/mL to 110 mg/mL.

10. The combination of claim 8 wherein the formulation is suitable for administration by subcutaneous injection.

11. A kit comprising:
(a) a first vessel containing the compound of Formula I or salt thereof as defined in any one of claims 1-7;
(b) a second vessel containing a substantially anhydrous solvent as defined in claim 8; and
(c) one or more ancillary therapeutic component(s) as defined in claim 1;
wherein said compound of Formula I is for administration before said one or more ancillary therapeutic component(s).

12. The kit of claim 11 wherein the compound of Formula I is in the form of a substantially anhydrous powder, and is more preferably lyophilized.

13. The kit of any one of claims 11-12 wherein the first vessel contains 80 mg to 110 mg of said compound of Formula I or salt thereof.

14. The kit of any one of claims 11-13 further comprising instructions for administration by subcutaneous injection.

15. The combination of any one of claims 1-10 or kit of any one of claims 11-14, wherein the ancillary therapeutic component comprises: (a) a T-cell activating agent and a cancer vaccine; or (b) a T-cell activating agent and an IDO inhibitor; optionally wherein the ancillary therapeutic component further comprises an adjuvant.

16. The combination of any one of claims 1-10 or kit of any one of claims 11-14 wherein the ancillary therapeutic component comprises a T-cell activating agent selected from agonists or antibodies for: ICOS, GITR, MHC, CD80, CD86, Galectin 9 and LAG-3.

17. The combination of any one of claims 1-10 or kit of any one of claims 11-14 wherein the T-cell activating agent is an antibody selected from: (a) a CD137 agonist; (b) a CD40 agonist; (c) an OX40 agonist; (d) a PD-1 mAb; (e) a PD-L1 mAb; (f) a PD-L2 mAb; (g) a CTLA-4 mAb; and (h) combinations of (a)-(g).

18. The combination of any one of claims 1-10 or kit of any one of claims 11-14 wherein the T-cell activating agent is an antibody, and the antibody comprises an antibody selected from: (a) Tremelimumab; (b) Ipilimumab; (c) Nivolumab; (d) Lambrolizumab; (e) BMS-936559; (f) MEDI4736; (g) MPDL3280A; and (h) PF-05082566.

19. A combination or kit according to any preceding claim for use in treating a disease selected from:
(a) a myelodysplastic syndrome (MDS);
(b) a cancer;
(c) a haematological disorder; and
(d) a disease associated with abnormal haemoglobin synthesis;
wherein the compound of Formula I or salt thereof as defined in any one of claims 1-7 is administered before the one or more ancillary therapeutic component(s).

20. A combination or kit for use according to claim 19 wherein the one or more ancillary therapeutic component(s) comprises a CTLA-4 mAb.

21. A combination or kit for use according to any one of claims 19-20 wherein the haematological disorder is leukemia, more preferably selected from acute myeloid leukemia (AML), acute promyelocyte leukemia, acute lymphoblastic leukemia, and chronic myelogenous leukemia.

22. A combination or kit for use according to any one of claims 19-20 wherein the cancer is selected from breast cancer, skin cancer, bone cancer, prostate cancer, liver cancer, lung cancer, non-small cell lung cancer, squamous non-small cell lung adenocarcinoma, brain cancer, cancer of the larynx, gall bladder, pancreas, rectum, parathyroid, thyroid, adrenal, neural tissue, head and neck, colon, stomach, bronchi, and kidney cancer, basal cell carcinoma, squamous cell carcinoma of both ulcerating and papillary type, metastatic skin carcinoma, osteo sarcoma, Ewing's sarcoma, reticulum cell sarcoma, myeloma, giant cell tumour, small-cell lung tumour, islet cell tumour, primary brain tumour, acute and chronic lymphocytic and granulocytic tumours, hairy-cell tumour, adenoma, hyperplasia, medullary carcinoma, pheochromocytoma, mucosal neuroma, intestinal ganglioneuromas, hyperplastic corneal nerve tumour, marfanoid habitus tumour, Wilm's tumour, seminoma, ovarian tumour, platinum resistant ovarian cancer, leiomyomater tumour, cervical dysplasia and in situ carcinoma, neuroblastoma, retinoblastoma, soft tissue sarcoma, malignant carcinoid, mycosis fungoide, rhabdomyosarcoma, Kaposi's sarcoma, osteogenic sarcoma, malignant hypercalcemia, renal cell tumour, polycythemia vera, adenocarcinoma, glioblastoma multiforma, leukemia, lymphoma, melanoma, epidermoid carcinoms, hepatocellular carcinoma and a solid tumour.

23. A combination or kit for use according to any one of claims 19-20 wherein the disease associated with abnormal haemoglobin synthesis is selected from sickle cell anaemia and β-thalassemia.

24. A combination or kit for use according to any one of claims 19-20 wherein the MDS is selected from low-, intermediate- and high-risk MDS and myloproliferative neoplasms.

25. The combination of any one of claims 1-11 or kit of any one of claims 12-15 for use in therapy or prophylaxis.

26. A combination or kit for use according to any one of claims 19-24 comprising administering a compound of formula (I) to a subject according to a dosage regimen of every day for 5 days, immediately followed by two dose-free days, followed by administration of the one or more ancillary therapeutic component(s).

27. A combination or kit for use according to claim 26, comprising administering a compound of formula (I) to a subject according to a dosage regimen of every day for 5 days, immediately followed by two dose-free days, followed by administration of an ancillary therapeutic component(s) comprising a CTLA-4 mAb.

28. A combination or kit according to any one of claims 1 to 18, wherein the one or more ancillary therapeutic component(s) further comprises an adjuvant.

29. A combination or kit according to claim 28 wherein said adjuvant is a Pathogen-Recognition Receptor (PRR) ligand.

30. A combination or kit according to claim 28 wherein said adjuvant comprises a TLR ligand, preferably one or more of TLR1, TLR2, TLR3, TLR4, TLR5, TLR6, TLR7, TLR8, TLR9, TLR10 and TLR11.

## Patentansprüche

1. Kombination, welche die folgenden gesonderten Bestandteile umfasst:
(i) eine Verbindung der Formel I oder ein pharmazeutisch unbedenkliches Salz derselben:
(5-Azacytosingruppe)-L-(Guaningruppe) (I)
wobei L ein phosphorhaltiges Bindeglied der Formel (II) ist: 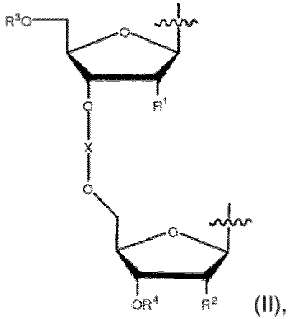 wobei R¹ und R² auf unabhängige Weise für H, OH, eine Alkoxygruppe, eine Alkoxyalkoxygruppe, eine Acyloxygruppe, eine Carbonatgruppe, eine Carbamatgruppe, oder ein Halogen stehen; R³ für H steht oder R³ zusammen mit dem Sauerstoffatom, an welches R³ gebunden ist, einen Ether, einen Ester, ein Carbonat oder ein Carbamat bildet; R⁴ für H steht oder R⁴ zusammen mit dem Sauerstoffatom, an welches R⁴ gebunden ist, einen Ether, einen Ester, ein Carbonat oder ein Carbamat bildet; und X zusammen mit dem Sauerstoffatom, an welches X gebunden ist, einen Phosphodiester, einen Phosphorothioat-Diester, einen Boranophosphat-Diester oder einen Methylphosphonat-Diester bildet; und
(ii) einen oder mehrere unterstützende therapeutische Bestandteil(e), wobei es sich bei dem unterstützenden therapeutischen Bestandteil um ein T-Zellen-aktivierendes Mittel handelt;
wobei die Verbindung der Formel I, oder ein pharmazeutisch unbedenkliches Salz davon, dazu bestimmt ist, vor dem unterstützenden therapeutischen Bestandteil verabreicht zu werden.

2. Kombination nach Anspruch 1, wobei R¹ und R² auf unabhängige Weise für H, OH, OMe, OEt, OCH₂CH₂OMe, OBn oder F stehen, wobei R¹ und R² vorzugsweise für H stehen.

3. Kombination nach Anspruch 1 oder Anspruch 2, wobei X zusammen dem Sauerstoffatom, an welches X gebunden ist, einen Phosphodiester bildet.

4. Kombination nach einem beliebigen der vorhergehenden Ansprüche, wobei es sich bei der Verbindung der Formel I um eine beliebige von I-(1-44) : 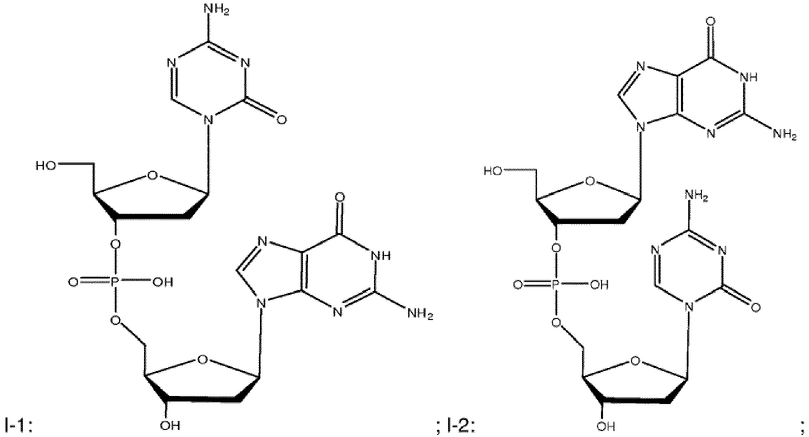 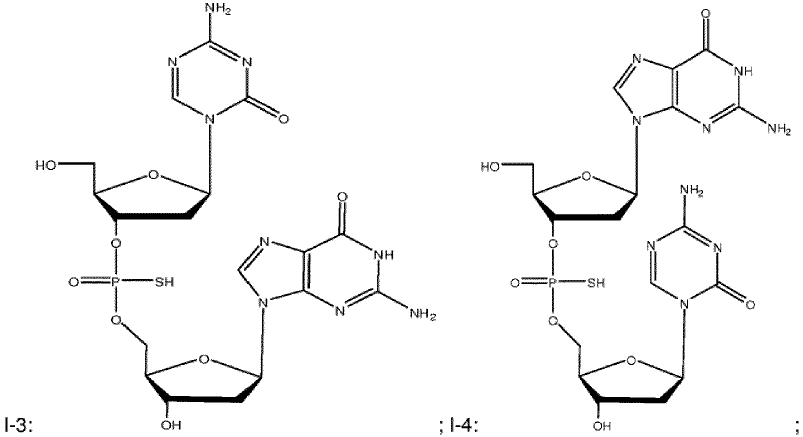 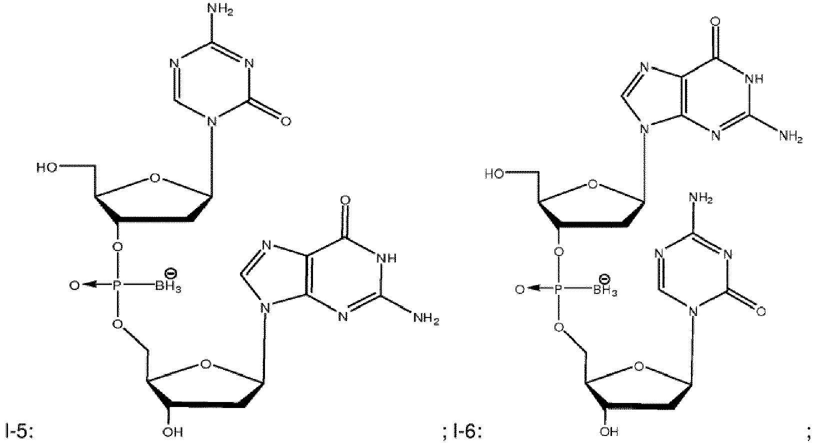 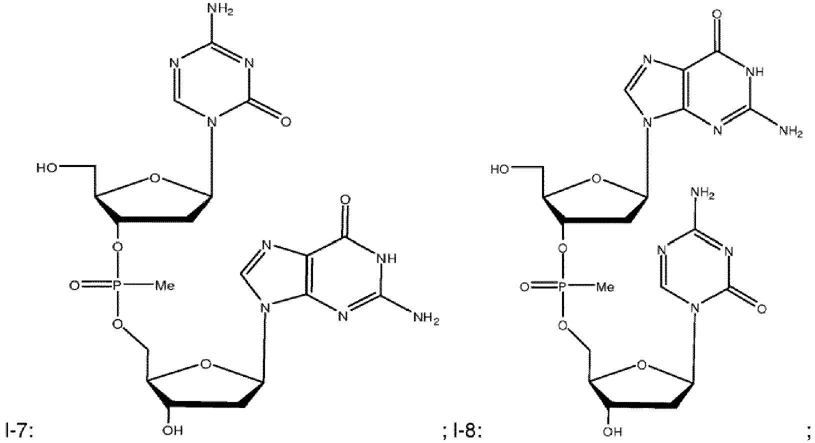 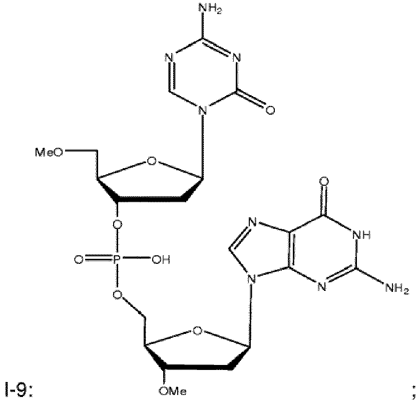 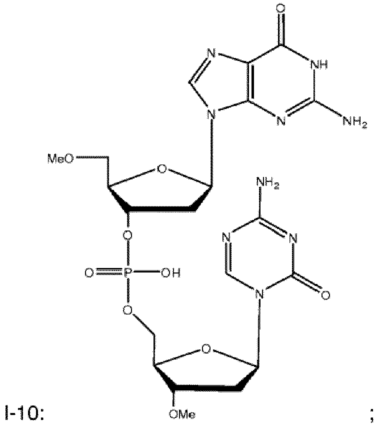 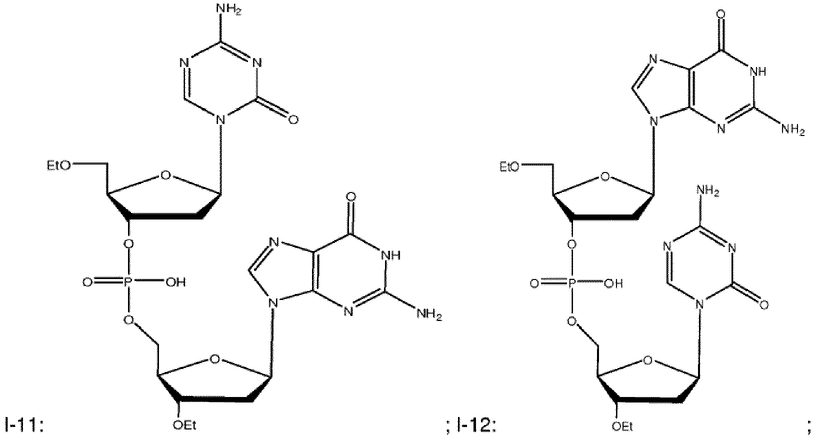 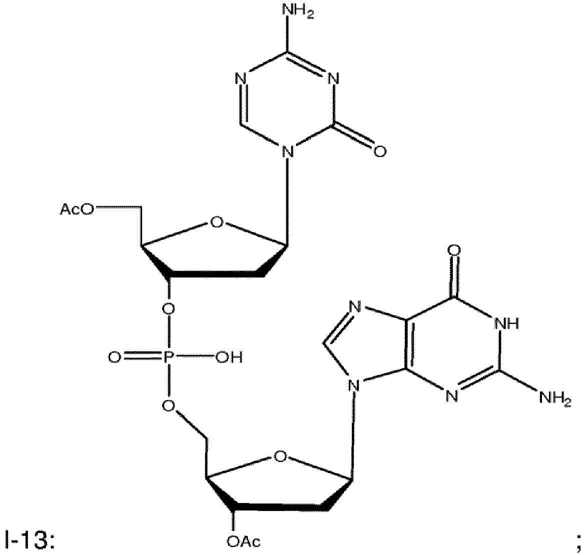 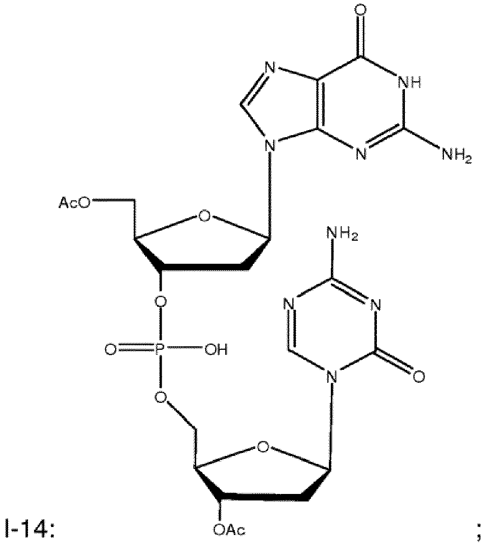 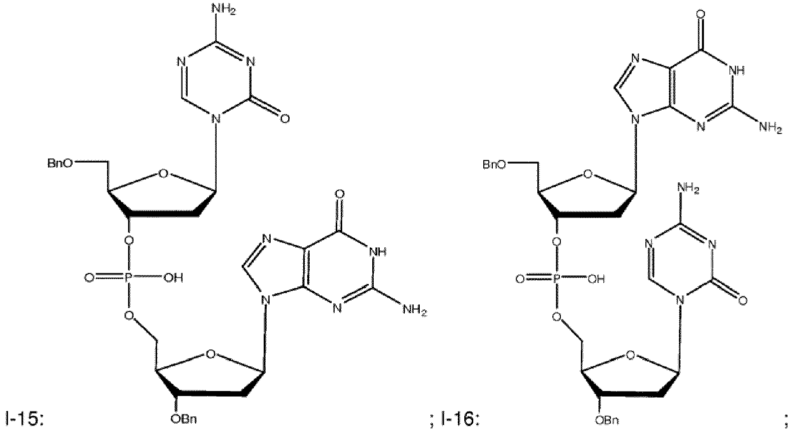 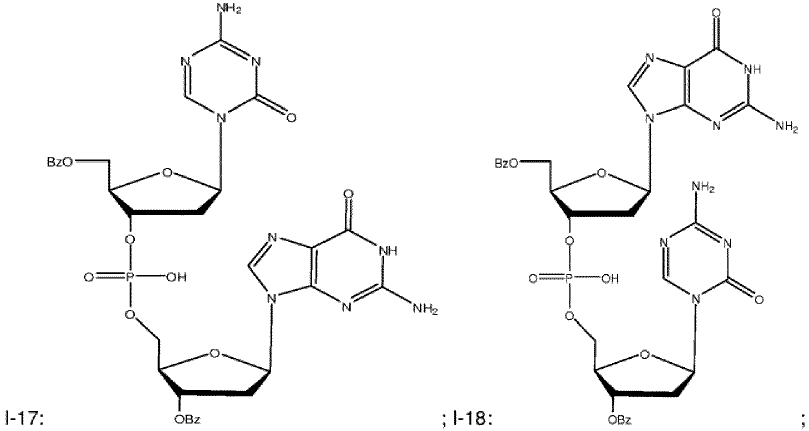 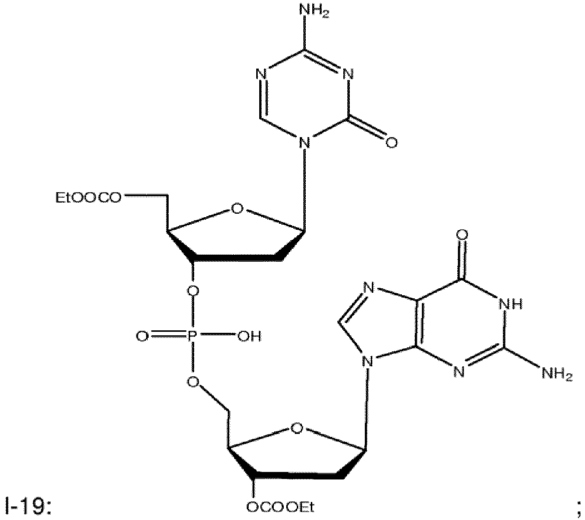 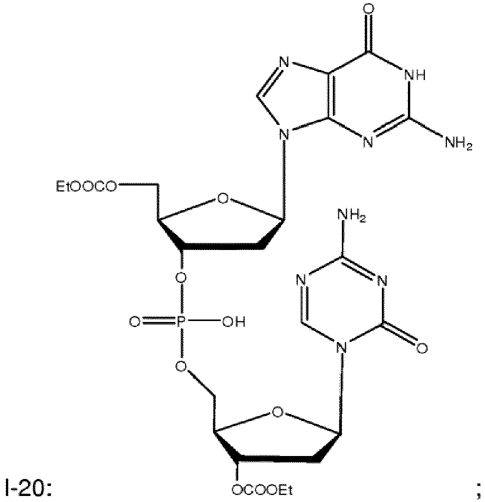 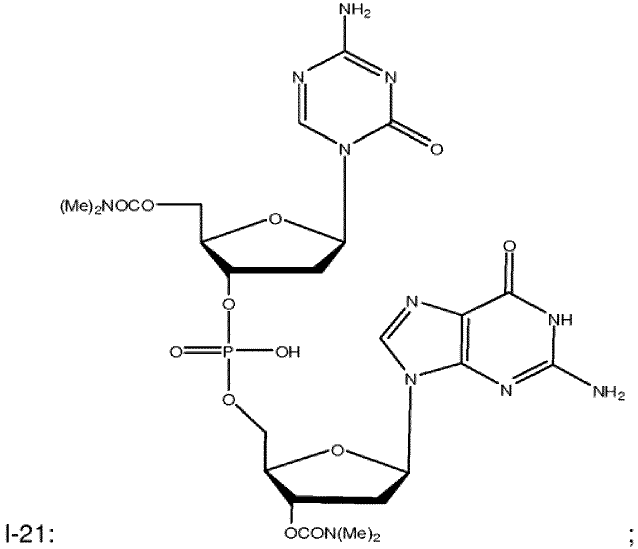 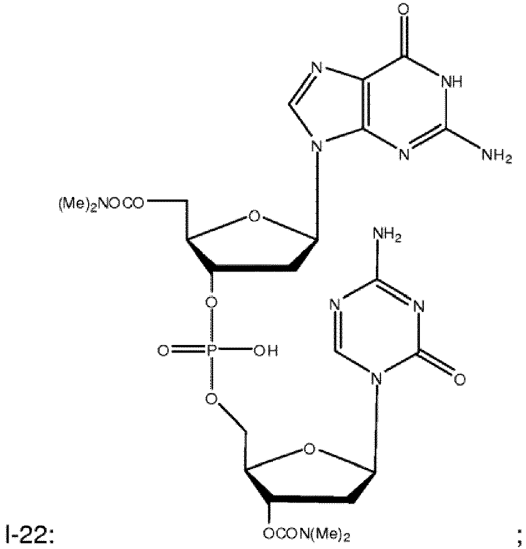 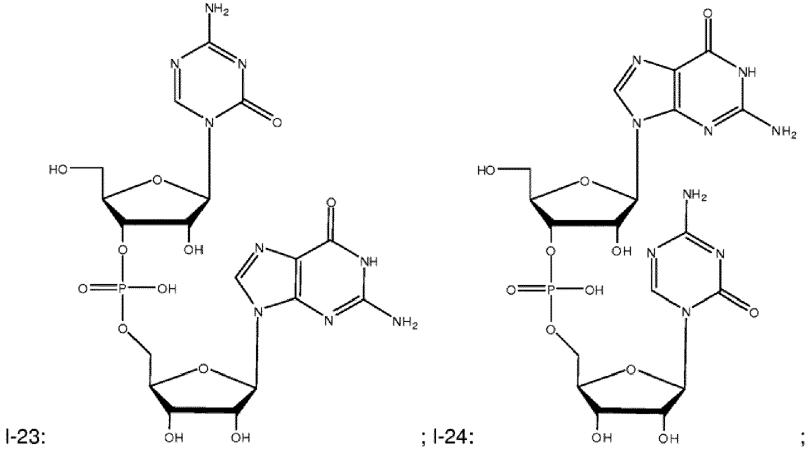 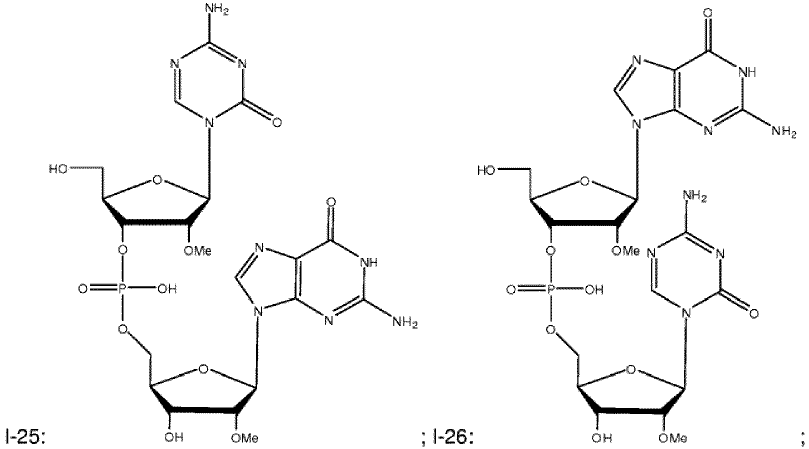 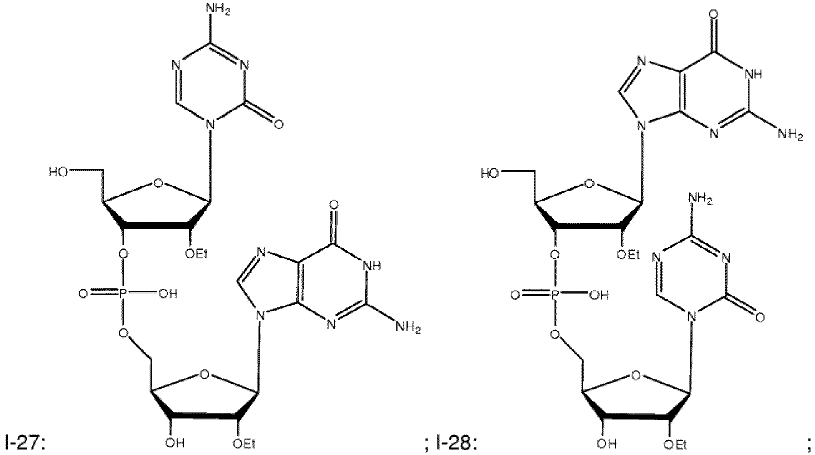 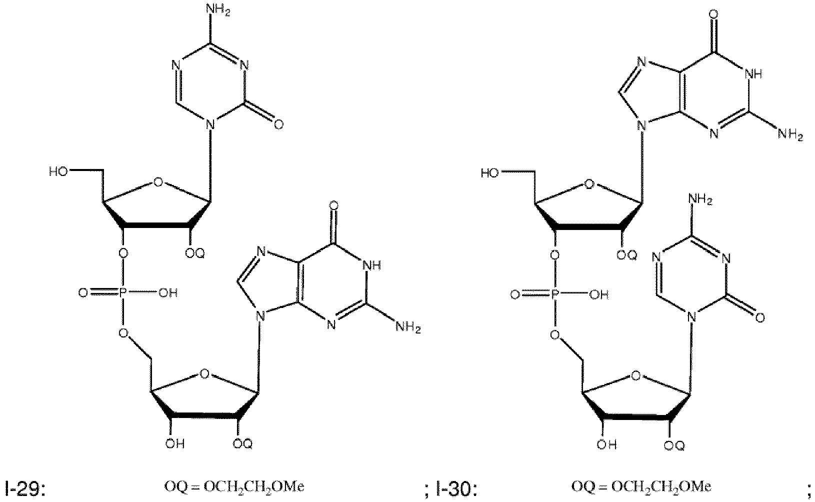 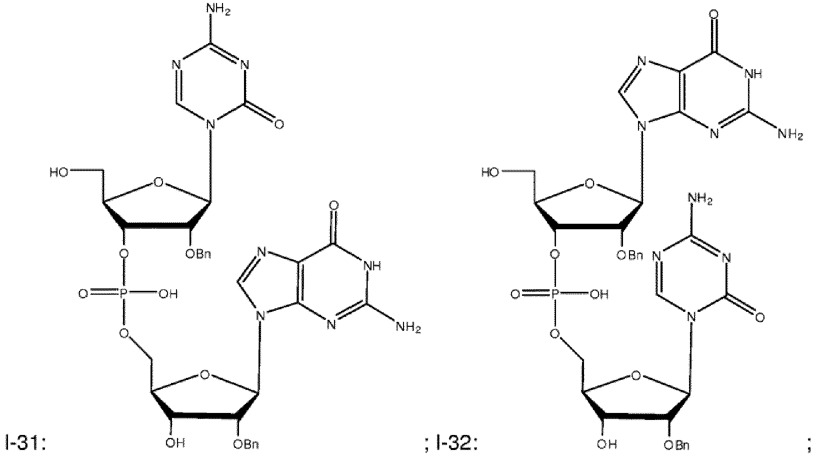 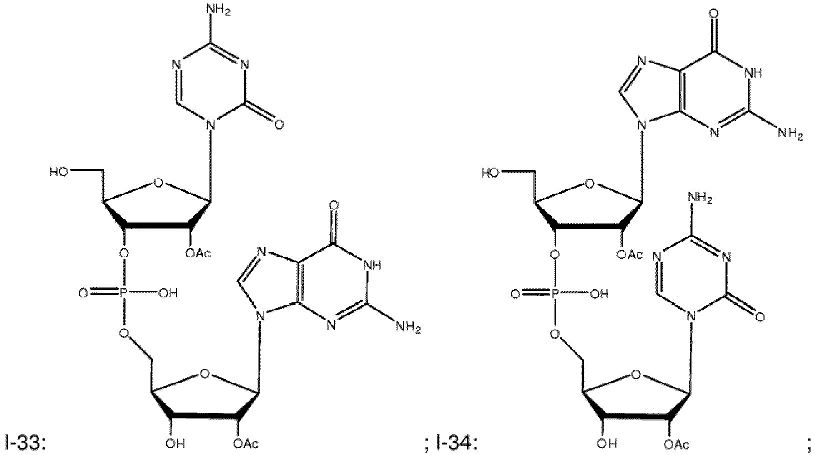 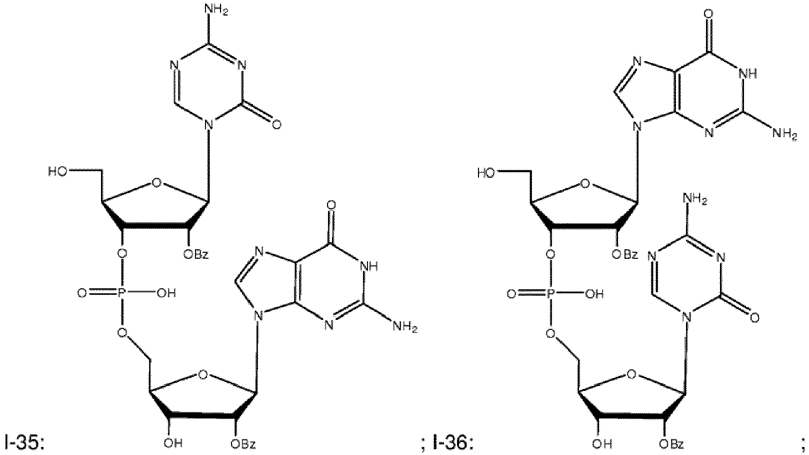 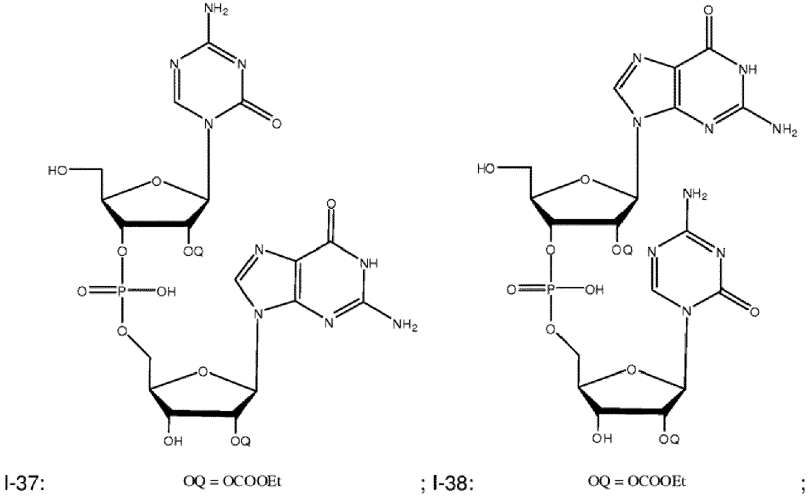 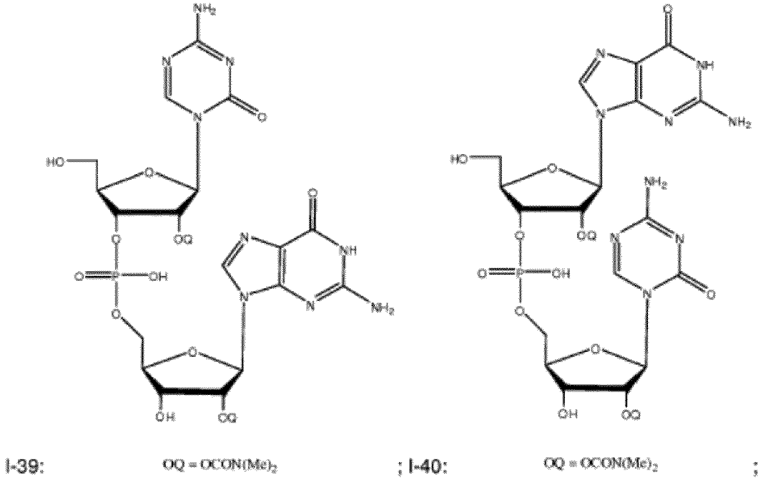 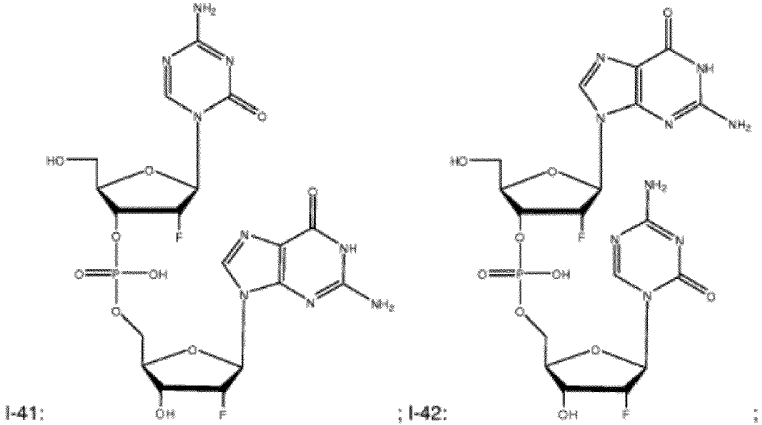 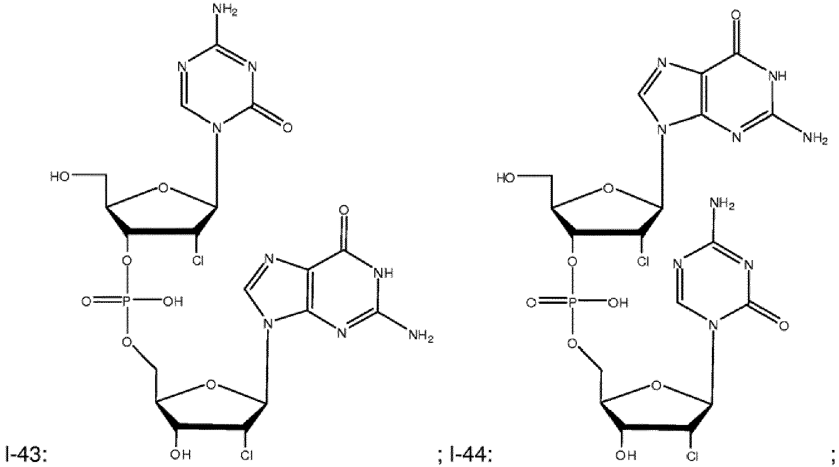 und pharmazeutisch unbedenkliche Salze davon handelt.

5. Kombination nach einem beliebigen der vorhergehenden Ansprüche, wobei es sich bei der Verbindung der Formel I um Folgendes handelt:

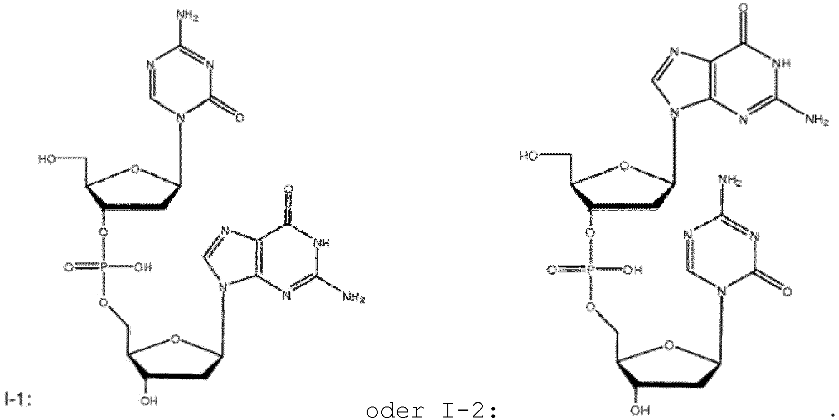

6. Kombination nach einem beliebigen der vorhergehenden Ansprüche, wobei es sich bei der Verbindung der Formel I um die folgende Formel: 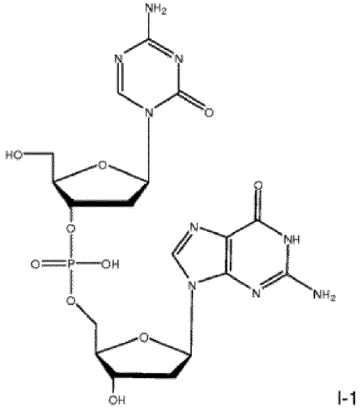 oder ein pharmazeutisch unbedenkliches Salz derselben handelt.

7. Kombination nach Anspruch 6, wobei es sich bei dem Salz um ein Natriumsalz handelt.

8. Kombination nach einem beliebigen der vorhergehenden Ansprüche, wobei die Verbindung der Formel I, oder das Salz derselben, in Form einer Formulierung vorliegt, wobei sie/es in einem im Wesentlichen wasserfreien Lösungsmittel aufgelöst wird, welches 45 % bis 85 % Propylenglykol; 5 % bis 45 % Glycerin; und 0 % bis 30 % Ethanol umfasst.

9. Kombination nach einem beliebigen vorhergehenden Anspruch, wobei die Verbindung der Formel I, oder das Salz derselben, in der Formulierung in einer Konzentration von 80 mg/mL bis 110 mg/mL vorliegt.

10. Kombination nach Anspruch 8, wobei die Formulierung sich zur Verabreichung mittels subkutaner Injektion handelt.

11. Kit, das Folgendes umfasst:
(a) einen ersten Behälter, welcher die Verbindung der Formel I, oder ein Salz derselben, gemäß der Begriffsbestimmung in einem beliebigen der Ansprüche 1 bis 7 enthält;
(b) einen zweiten Behälter, der ein im Wesentlichen wasserfreies Lösungsmittel gemäß der Begriffsbestimmung in Anspruch 8 enthält; und
(c) einen oder mehrere unterstützende(n) therapeutische(n) Bestandteil(e) gemäß der Begriffsbestimmung in Anspruch 1;
wobei die Verbindung der Formel I dazu bestimmt ist, vor dem einen oder den mehreren unterstützenden therapeutischen Bestandteil(en) verabreicht zu werden.

12. Kit nach Anspruch 11, wobei die Verbindung der Formel I in Form eines im Wesentlichen wasserfreien Pulvers vorliegt, wobei sie insbesondere gefriergetrocknet ist.

13. Kit nach einem beliebigen der Ansprüche 11 bis 12, wobei der erste Behälter 80 mg bis 110 mg der Verbindung der Formel I, oder des Salzes derselben, enthält.

14. Kit nach einem beliebigen der Ansprüche 11 bis 13, wobei es Anweisungen zur Verabreichung mittels subkutaner Injektion umfasst.

15. Kombination nach einem beliebigen der Ansprüche 1 bis 10 oder Kit nach einem beliebigen der Ansprüche 11 bis 14, wobei der unterstützende therapeutische Bestandteil Folgendes umfasst: (a) ein T-Zellen-aktivierendes Mittel und einen Krebsimpfstoff; oder (b) ein T-Zellen-aktivierendes Mittel und einen IDO-Hemmer; wobei der unterstützende therapeutische Bestandteil möglicherweise weiterhin einen Hilfsstoff umfasst.

16. Kombination nach einem beliebigen der Ansprüche 1 bis 10 oder Kit nach einem beliebigen der Ansprüche 11 bis 14, wobei der unterstützende therapeutische Bestandteil ein T-Zellen-aktivierendes Mittel umfasst, das aus Agonisten oder Antikörpern für Folgendes ausgewählt ist: ICOS, GITR, MHC, CD80, CD86, Galectin 9 und LAG-3.

17. Kombination nach einem beliebigen der Ansprüche 1 bis 10 oder Kit nach einem beliebigen der Ansprüche 11 bis 14, wobei es sich bei dem T-Zellen-aktivierenden Mittel um einen Antikörper handelt, der aus den folgenden ausgewählt ist: (a) einem CD137-Agonisten; (b) einem CD40-Agonisten; (c) einem OX40-Agonisten; (d) einem PD-1 mAb; (e) einem PD-L1 mAb; (f) einem PD-L2 mAb; (g) einem CTLA-4 mAb; und (h) Kombinationen von (a) bis (g).

18. Kombination nach einem beliebigen der Ansprüche 1 bis 10 oder Kit nach einem beliebigen der Ansprüche 11 bis 14, wobei es sich bei dem T-Zellen-aktivierenden Mittel um einen Antikörper handelt und der Antikörper einen Antikörper umfasst, welcher aus den folgenden ausgewählt ist: (a) Tremelimumab; (b) Ipilimumab; (c) Nivolumab; (d) Lambrolizumab; (e) BMS-936559; (f) MEDI4736; (g) MPDL3280A; und (h) PF-05082566.

19. Kombination oder Kit gemäß einem beliebigen der vorhergehenden Anspruch zur Verwendung beim Behandeln einer Erkrankung, die aus den folgenden ausgewählt ist:
(a) einem myelodysplastischen Syndrom (MDS);
(b) einer Krebserkrankung;
(c) einer hämatologischen Störung; und
(d) einer Erkrankung, die mit einer anormalen Hämoglobinsynthese in Verbindung steht;
wobei die Verbindung der Formel I, oder das Salz derselben, gemäß der Begriffsbestimmung in einem beliebigen der Ansprüche 1 bis 7 vor der einen oder den mehreren unterstützenden therapeutischen Bestandteil(en) verabreicht wird.

20. Kombination oder Kit zur Verwendung gemäß Anspruch 19, wobei der eine oder die mehreren therapeutische(n) Bestandteil(e) einen CTLA-4 mAb umfasst.

21. Kombination oder Kit zur Verwendung gemäß einem beliebigen der Ansprüche 19 bis 20, wobei es sich bei der hämatologischen Störung um eine Leukämie handelt, die insbesondere aus akuter myeloischer Leukämie (AML), akuter promyeloischer Leukämie, akuter lymphatischer Leukämie und chronischer myeloischer Leukämie ausgewählt ist.

22. Kombination oder Kit zur Verwendung gemäß einem beliebigen der Ansprüche 19 bis 20, wobei die Krebserkrankung aus Brustkrebs, Hautkrebs, Knochenkrebs, Prostatakrebs, Leberkrebs, Lungenkrebs, dem nichtkleinzelligen Bronchialkarzinom, dem nichtkleinzelligen Plattenepithel-Adenokarzinom der Lunge, Hirntumoren, Krebserkrankungen des Kehlkopfs, der Gallenblase, der Bauchspeicheldrüse, des Enddarms, der Nebenschilddrüse, der Schilddrüse, der Nebenniere, des Nervengewebes, im Kopf- und Halsbereich, des Dickdarms, des Magens, der Bronchien sowie Nierenkarzinomen, dem Basaliom, Plattenepithelkarzinomen sowohl der ulzerierenden als auch der papillären Art, metastatischen Hautkarzinomen, dem Osteosarkom, dem Ewing-Sarkom, dem Retikulumzellen-Sarkom, dem Myelom, Riesenzelltumoren, kleinzelligen Bronchialtumoren, Inselzelltumoren, primären Hirntumoren, akuten und chronischen Tumoren der Lymphozyten und Granulozyten, dem Haarzellentumor, einem Adenom, einer Hyperplasie, einem medullären Karzinom, dem Phäochromozytom, einem Neurom der Schleimhäute, einem Ganglioneurom des Darms, hyperplastischen Tumoren des Hornhautnervs, Tumoren mit marfanoidem Habitus, dem Wilms-Tumor, einem Seminom, Eierstocktumoren, einer platinresistenten Krebserkrankung der Eierstöcke, Leiomyom-Tumoren, Dysplasien und in-situ-Karzinomen des Gebärmutterhalses, einem Neuroblastom, dem Retinoblastom, einem Sarkom des Weichgewebes, einem bösartigen Karzinoid, Mycosis fungoides, dem Rhabdomyosarkom, dem Kaposi-Sarkom, dem osteogenen Sarkom, der bösartigen Hyperkalzämie, Nierenzelltumoren, Polycythaemia vera, einem Adenokarzinom, einem Glioblastom, einer Leukämie, einem Lymphom, einem Melanom, epidermoiden Karzinomen, Leberzellkarzinomen und einem festen Tumor ausgewählt ist.

23. Kombination oder Kit zur Verwendung gemäß einem beliebigen der Ansprüche 19 bis 20, wobei die Erkrankung, welche mit einer anormalen Hämoglobinsynthese in Verbindung steht, aus Sichelzellenanämie und β-Thalassämie ausgewählt ist.

24. Kombination oder Kit zur Verwendung gemäß einem beliebigen der Ansprüche 19 bis 20, wobei das MDS aus Niedrig-, Mittel- und Hochrisiko-MDS oder myeloproliferativen Neoplasien ausgewählt ist.

25. Kombination nach einem beliebigen der Ansprüche 1 bis 11 oder Kit nach einem beliebigen der Ansprüche 12 bis 15 zur Verwendung in der Therapie oder der Prophylaxe.

26. Kombination oder Kit zur Verwendung gemäß einem beliebigen der Ansprüche 19 bis 24, wobei diese das Verabreichen einer Verbindung der Formel (I) an einen Zielorganismus gemäß einem Dosierschema von jedem Tag über fünf Tage, unmittelbar gefolgt von zwei Tagen ohne Dosisgabe, gefolgt vom Verabreichen des einen oder der mehreren unterstützenden therapeutischen Bestandteile(s) umfasst.

27. Kombination oder Kit zur Verwendung gemäß Anspruch 26, wobei diese das Verabreichen einer Verbindung der Formel (I) an einen Zielorganismus gemäß einem Dosierschema von jedem Tag über fünf Tage, unmittelbar gefolgt von zwei Tagen ohne Dosisgabe, gefolgt vom Verabreichen eines unterstützenden therapeutischen Bestandteiles umfasst, wobei letzterer einen CTLA-4 mAb umfasst.

28. Kombination oder Kit gemäß einem beliebigen der Ansprüche 1 bis 18, wobei der eine oder die mehreren unterstützenden therapeutische(n) Bestandteil(e) weiterhin einen Hilfsstoff umfasst.

29. Kombination oder Kit gemäß Anspruch 28, wobei es sich bei dem Hilfsstoff um einen Liganden eines Pathogenerkennungs-Rezeptors (PRR) handelt.

30. Kombination oder Kit gemäß Anspruch 28, wobei der Hilfsstoff einen TLR-Liganden umfasst, vorzugsweise einen oder mehrere von TLR1, TLR2, TLR3, TLR4, TLR5, TLR6, TLR7, TLR8, TLR9, TLR10 und TLR11.

## Revendications

1. Combinaison comprenant les composants séparés suivants :
(i) un composé de Formule I ou l'un des sels pharmaceutiquement acceptables de celui-ci :
(groupement 5-azacytosine)-L-(groupement guanine) (I)
où L représente un pont phosphoré de Formule (II) : 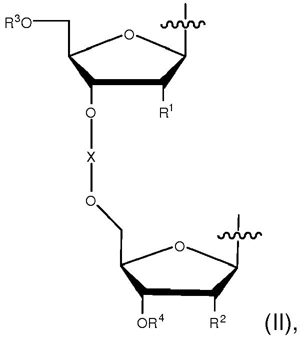 où chacun des radicaux R¹ et R² représente indépendamment H, OH, un groupement alkoxy, un groupement alkoxyalkoxy, un groupement acyloxy, un groupement carbonate, un groupement carbamate ou un atome d'halogène ; R³ représente H, ou R³ forme conjointement avec l'atome d'oxygène auquel R³ est lié un éther, un ester, un carbonate ou un carbamate ; R⁴ représente H, ou R⁴ forme conjointement avec l'atome d'oxygène auquel R⁴ est lié un éther, un ester, un carbonate ou un carbamate ; et X forme conjointement avec les atomes d'oxygène auxquels X est lié un phosphodiester, un diester de phosphorothioate, un diester de boranophosphate, ou un diester de méthylphosphonate ; et
(ii) un ou plusieurs composants thérapeutiques ancillaires, où ledit composant thérapeutique ancillaire est un agent d'activation de lymphocytes T ;
où ledit composé de Formule I ou l'un des sels pharmaceutiquement acceptables de celui-ci est destiné à une administration avant ledit composé thérapeutique ancillaire.

2. Combinaison selon la revendication 1, où chacun des radicaux R¹ et R² représente indépendamment H, OH, OMe, OEt, OCH₂CH₂OMe, OBn ou F, et préférentiellement, chacun des radicaux R¹ et R² représente H.

3. Combinaison selon la revendication 1 ou la revendication 2, où X forme conjointement avec les atomes d'oxygène auquel X est lié un phosphodiester.

4. Combinaison selon l'une quelconque des revendications précédentes, où le composé de Formule I est n'importe lequel des I-(1-44) : 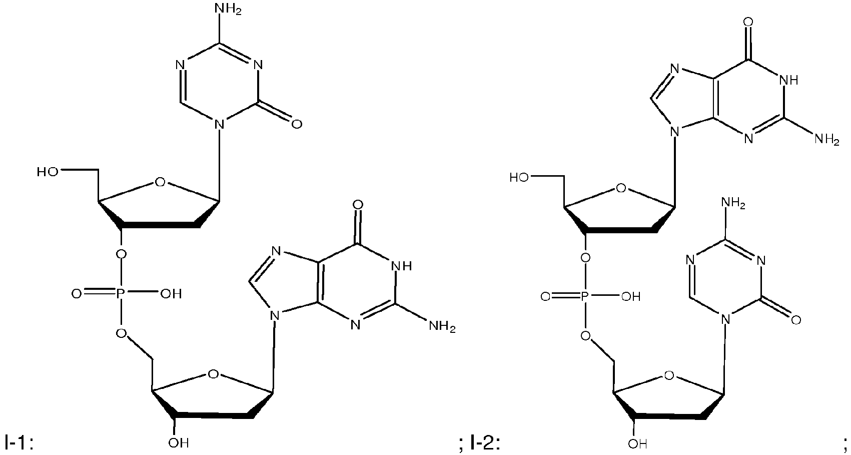 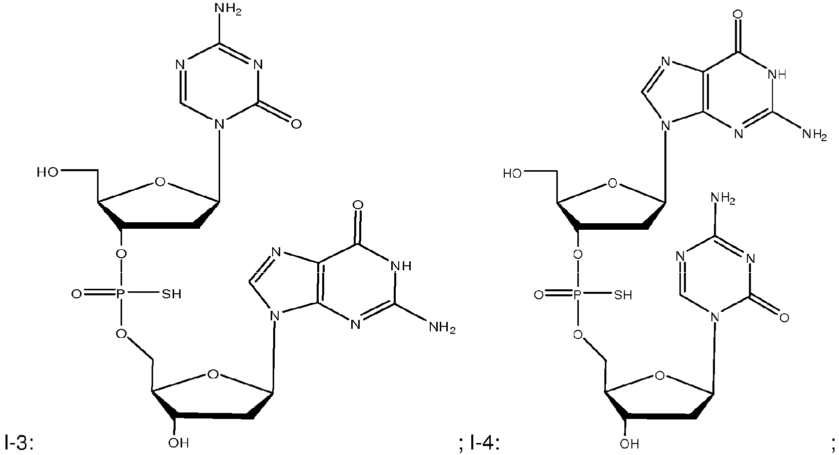 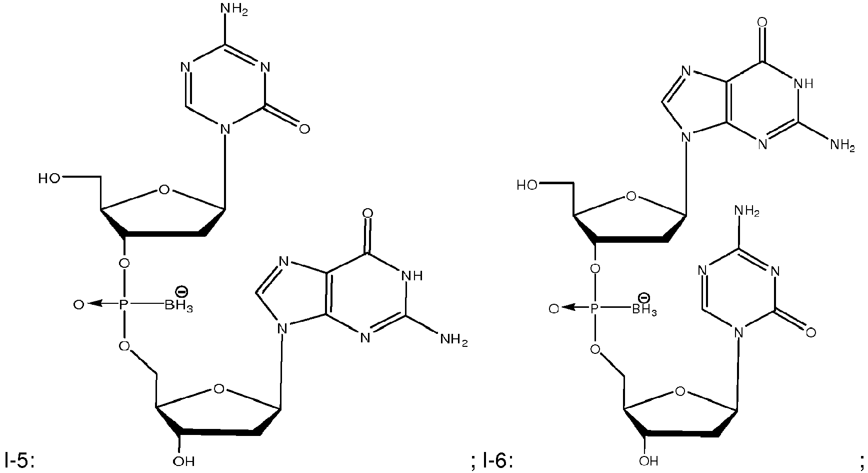 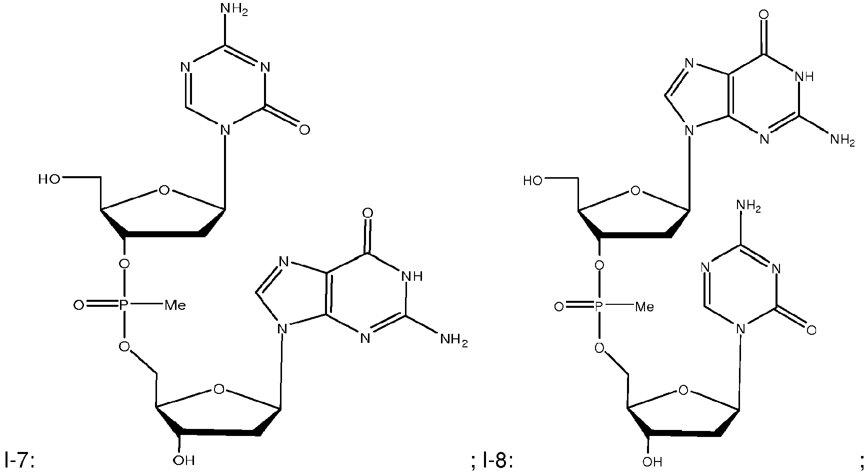 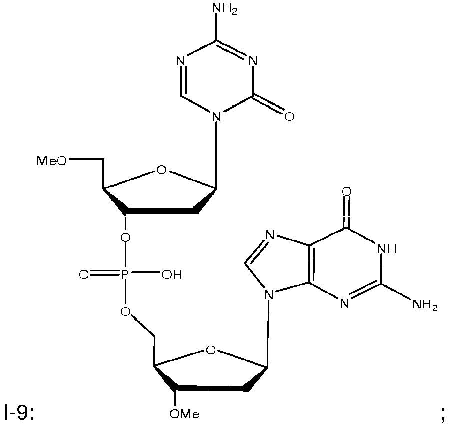 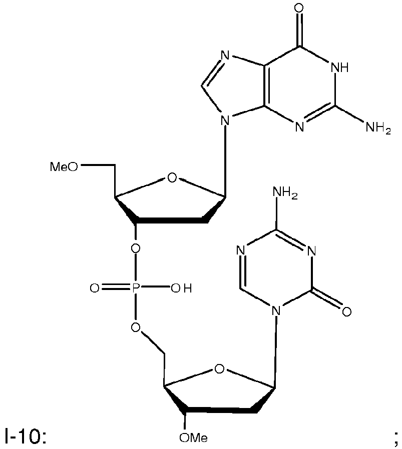 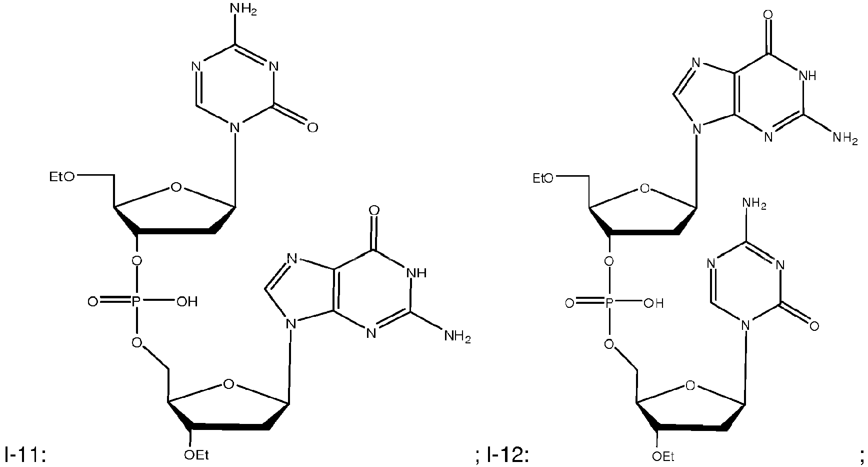 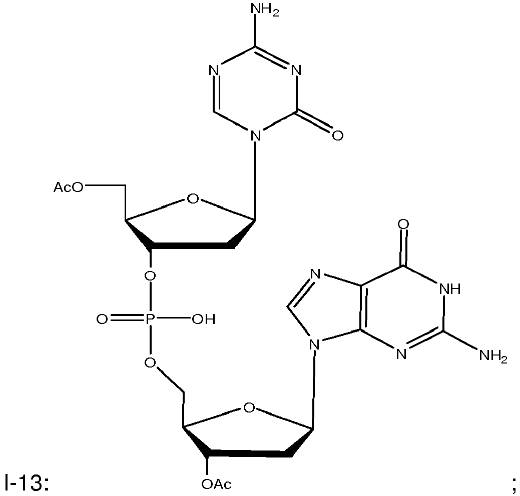 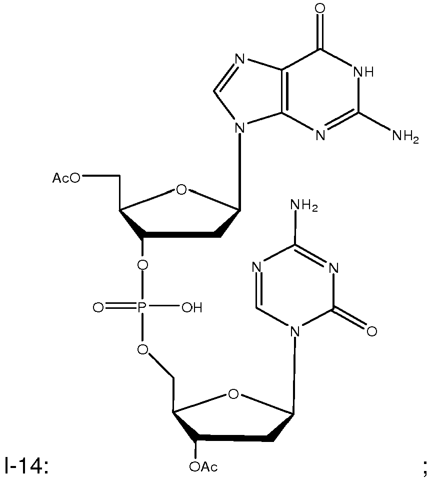 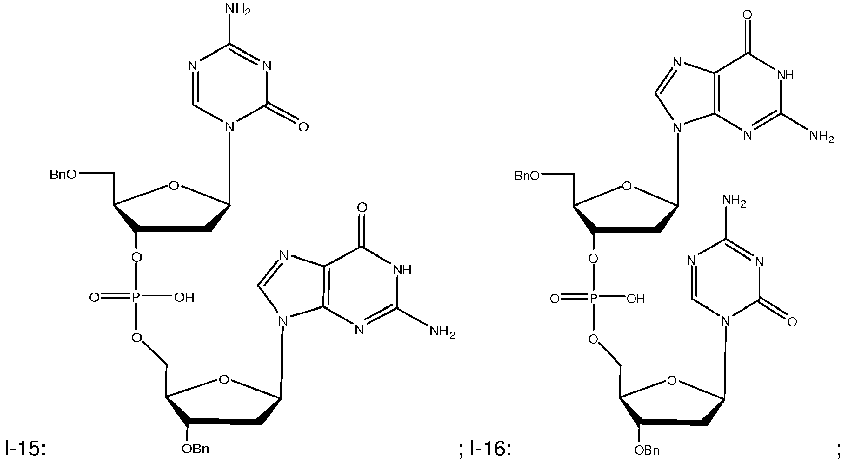 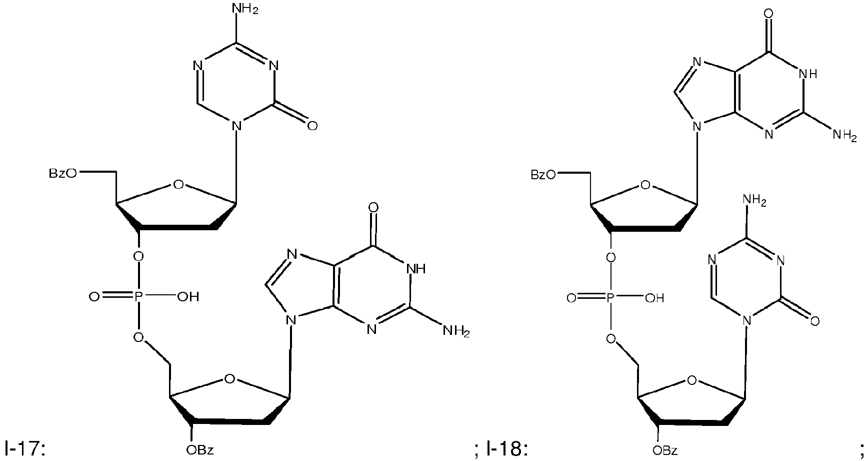 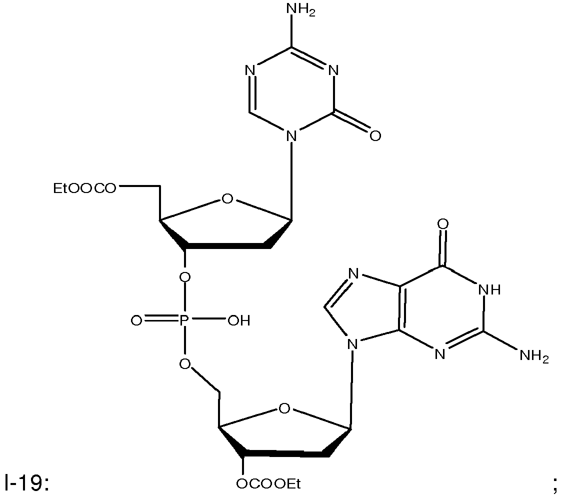 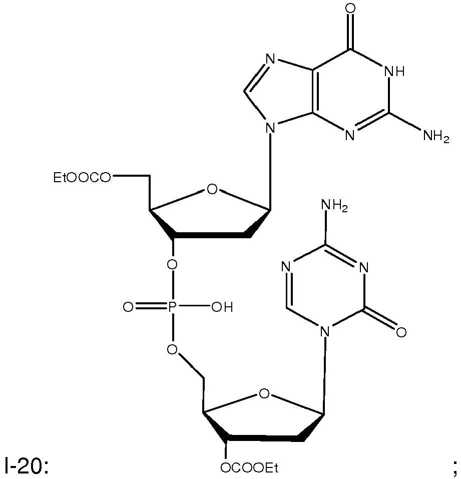 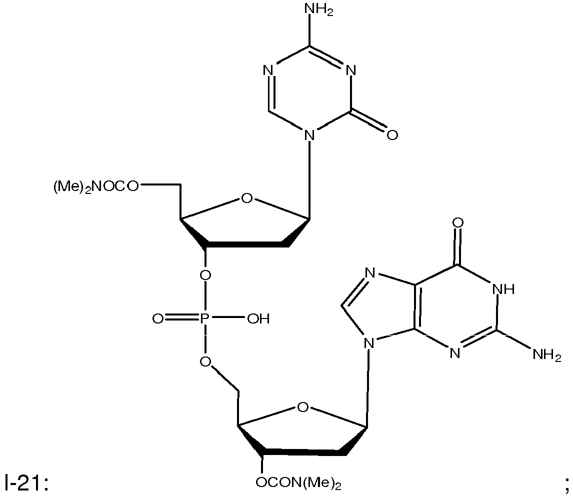 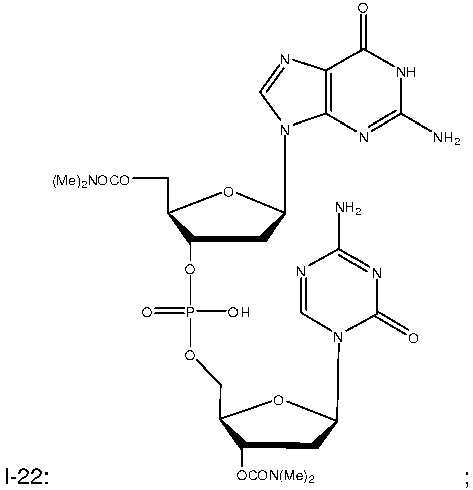 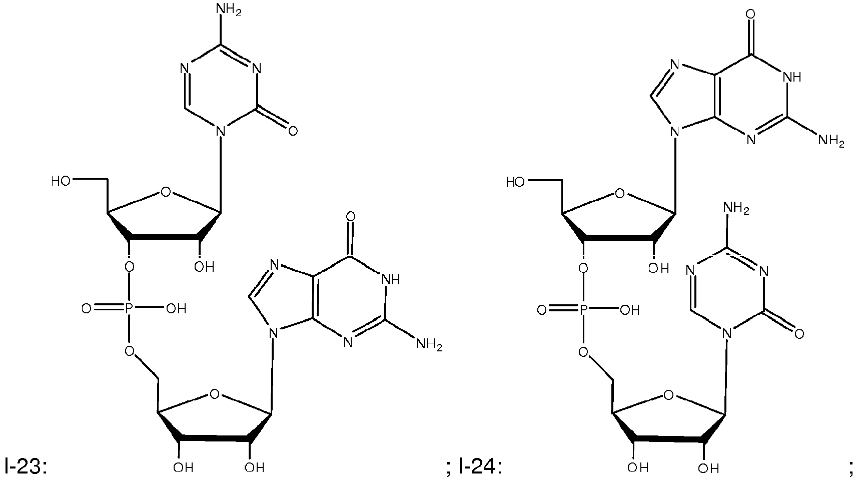 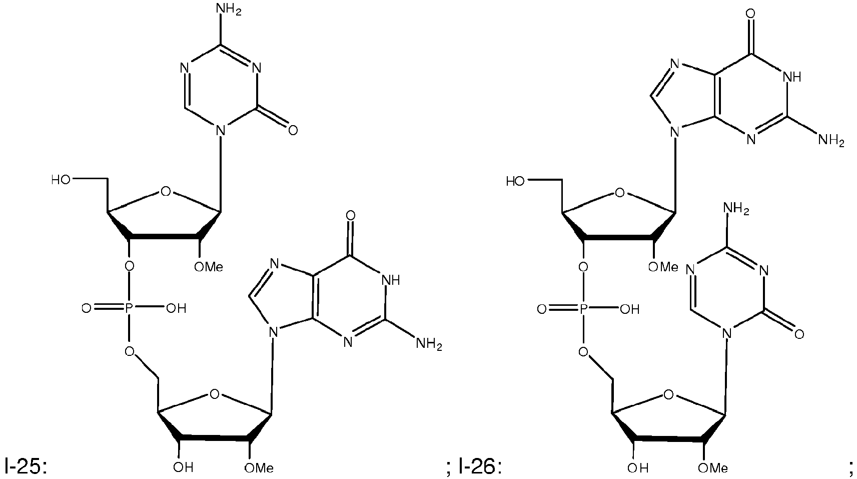 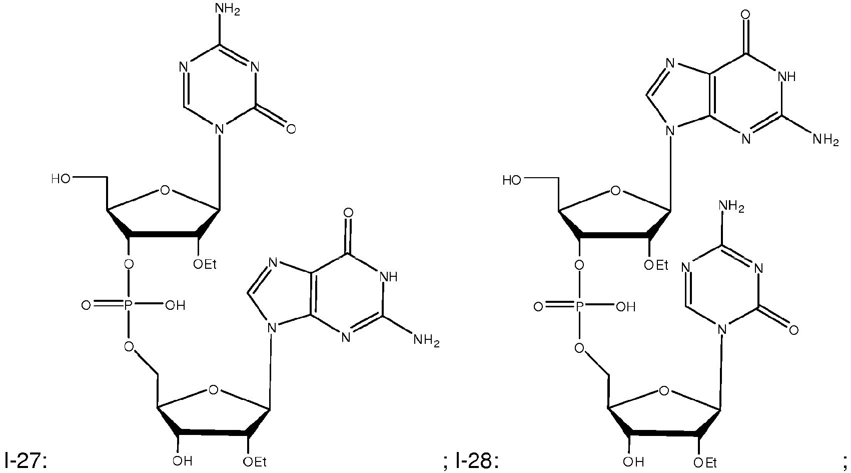 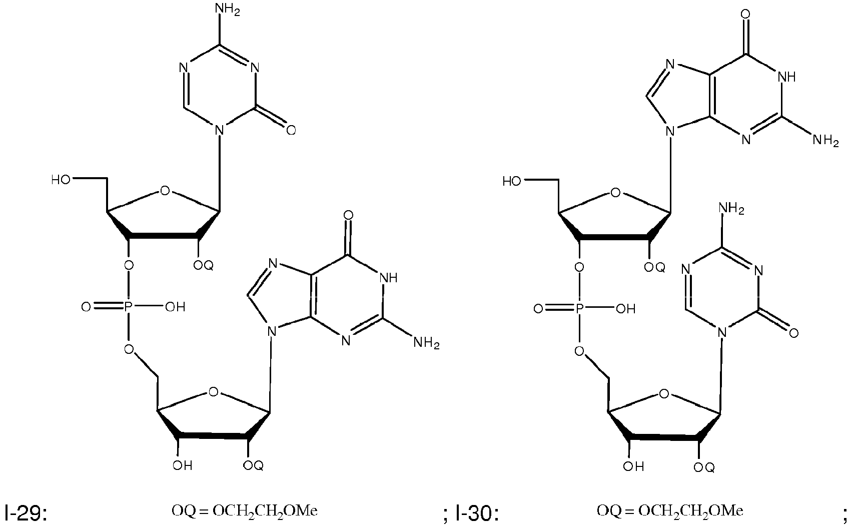 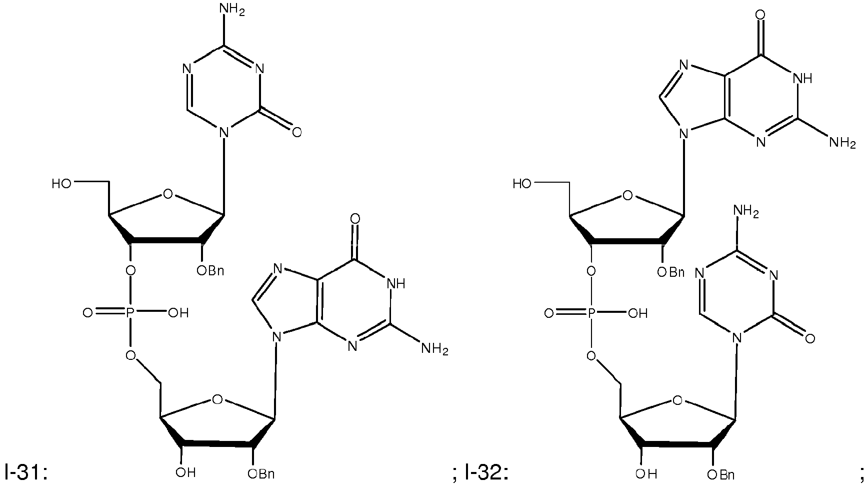 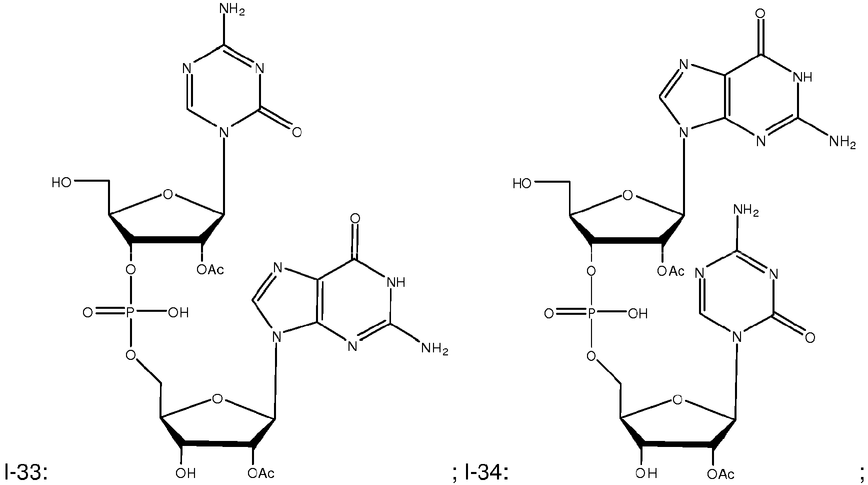 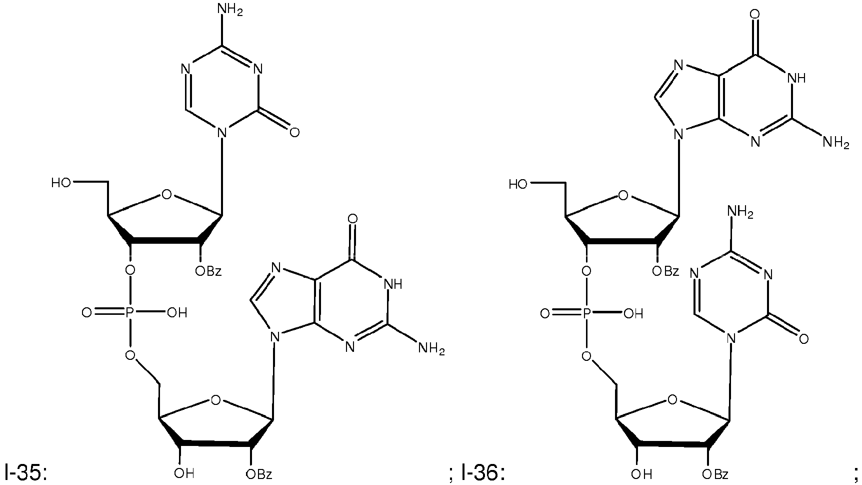 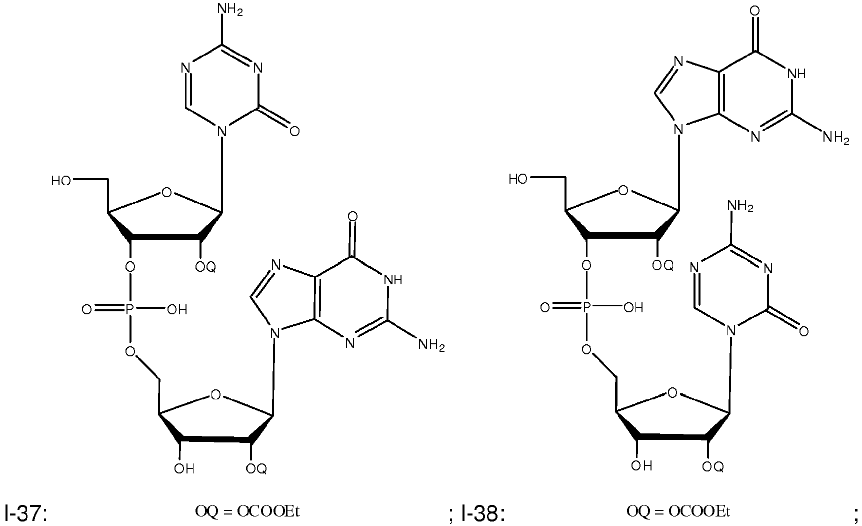 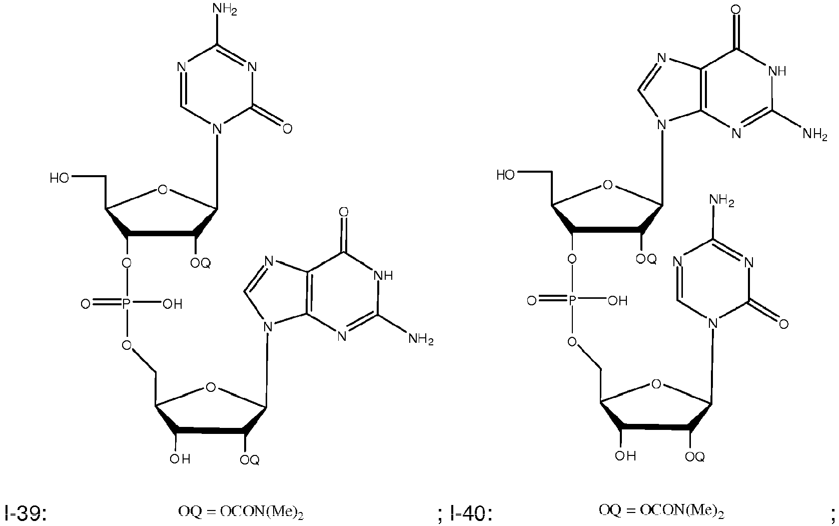 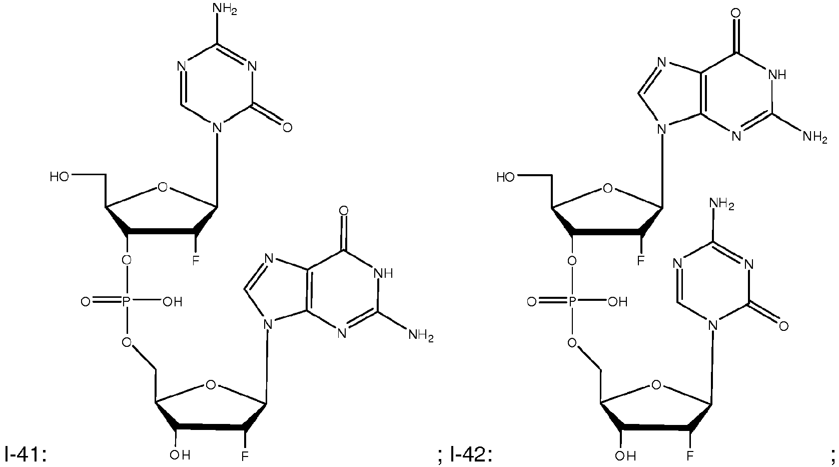 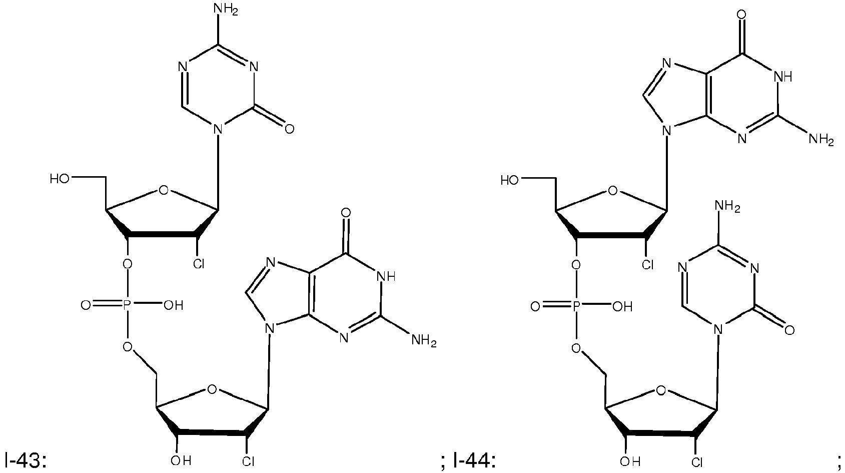 et les sels pharmaceutiquement acceptables de ceux-ci.

5. Combinaison selon l'une quelconque des revendications précédentes, où le composé de Formule I représente :

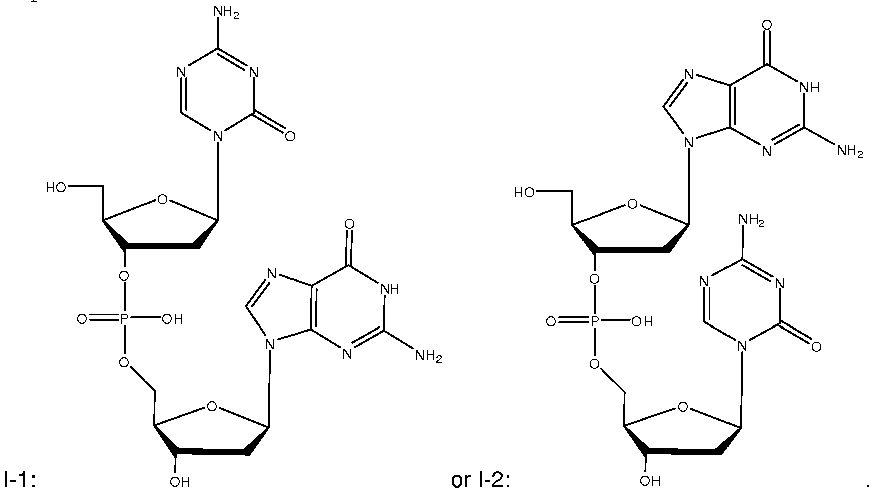

6. Combinaison selon l'une quelconque des revendications précédentes, où le composé de formule I répond à la formule : 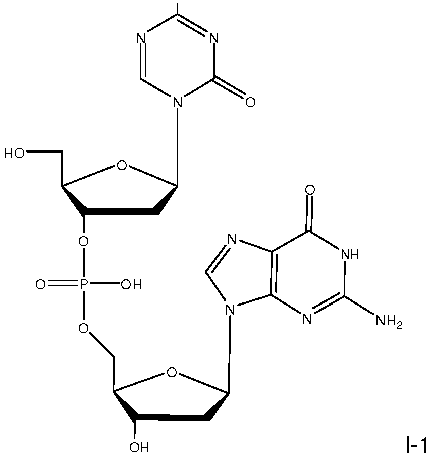 ou l'un des sels pharmaceutiquement acceptables de celui-ci.

7. Combinaison selon la revendication 6, où ledit sel est un sel de sodium.

8. Combinaison selon l'une quelconque des revendications précédentes, où le composé de Formule I ou le sel de celui-ci se présente sous la forme d'une formule, en étant dissous dans un solvant substantiellement anhydre comprenant 45 % à 85 % de propylène glycol ; 5 % à 45 % de glycérine ; et 0 % à 30 % d'éthanol.

9. Combinaison selon l'une quelconque des revendications précédentes, où le composé de Formule I ou le sel de celui-ci est présent dans la formule à une concentration de 80 mg/mL à 110 mg/mL.

10. Combinaison selon la revendication 8, où la formule est adaptée à une administration par injection sous-cutanée.

11. Kit comprenant :
(a) un premier récipient contenant le composé de Formule I ou l'un des sels de celui-ci selon l'une quelconque des revendications 1 à 7 ;
(b) un deuxième récipient contenant un solvant substantiellement anhydre selon la revendication 8 ; et
(c) un ou plusieurs composants thérapeutiques ancillaires selon la revendication 1 ; où ledit composé de Formule I est destiné à une administration avant lesdits un ou plusieurs composants thérapeutiques ancillaires.

12. Kit selon la revendication 11, où le composé de Formule I se présente sous la forme d'une poudre substantiellement anhydre, et est plus préférentiellement lyophilisé.

13. Kit selon l'une quelconque des revendications 11 à 12, où le premier récipient contient 80 mg à 110 mg dudit composé de Formule I ou d'un sel de celui-ci.

14. Kit selon l'une quelconque des revendications 11 à 13, comprenant en outre des instructions pour l'administration par injection sous-cutanée.

15. Combinaison selon l'une quelconque des revendications 1 à 10 ou kit selon l'une quelconque des revendications 11 à 14, où le composé thérapeutique ancillaire comprend : (a) un agent d'activation de lymphocytes T et d'un vaccin anticancéreux ; ou (b) un agent d'activation de lymphocytes T et un inhibiteur d'IDO ; éventuellement, où le composant thérapeutique ancillaire comprend en outre un adjuvant.

16. Combinaison selon l'une quelconque des revendications 1 à 10 ou kit selon l'une quelconque des revendications 11 à 14, où le composé thérapeutique ancillaire comprend un agent d'activation de lymphocytes T choisi parmi les agonistes ou les anticorps contre : ICOS, GITR, MHC, CD80, CD86, Galectine 9 et LAG-3.

17. Combinaison selon l'une quelconque des revendications 1 à 10 ou kit selon l'une quelconque des revendications 11 à 14, où l'agent d'activation de lymphocytes T est un anticorps choisi parmi : (a) un agoniste de CD137 ; (b) un agoniste de CD40 ; (c) un agoniste de OX40 ; (d) un PD-1 mAb ; (e) un PD-L1 mAb ; (f) un PD-L2 mAb ; (g) un CTLA-4 mAb ; et (h) des combinaisons de (a)-(g).

18. Combinaison selon l'une quelconque des revendications 1 à 10 ou kit selon l'une quelconque des revendications 11 à 14, où l'agent d'activation de lymphocytes T est un anticorps, et un anticorps comprend un anticorps choisi parmi : (a) Trémélimumab ; (b) Ipilimumab ; (c) Nivolumab ; (d) Lambrolizumab ; (e) BMS-936559 ; (f) MEDI4736 ; (g) MPDL3280A ; et (h) PF-05082566.

19. Combinaison ou kit selon l'une quelconque des revendications précédentes pour utilisation dans le traitement d'une maladie choisie parmi :
(a) un syndrome myélodysplasique (SMD) ;
(b) un cancer ;
(c) un trouble hématologique ; et
(d) une maladie associée à une synthèse anormale de l'hémoglobine ;
où le composé de Formule I ou le sel de celui-ci selon l'une quelconque des revendications 1 à 7 est administré avant le ou les composants thérapeutiques ancillaires.

20. Combinaison ou kit pour utilisation selon la revendication 19, où le ou les composants thérapeutiques ancillaires comprennent un CTLA-4 mAb.

21. Combinaison ou kit pour utilisation selon l'une quelconque des revendications 19 à 20, où le trouble hématologique est la leucémie, plus préférentiellement choisi parmi les suivantes : leucémie myéloïde aiguë (LMA), leucémie lymphocytaire aiguë, leucémie aiguë lymphoblastique et leucémie myéloïde chronique.

22. Combinaison ou kit pour utilisation selon l'une quelconque des revendications 19 à 20, où le cancer est choisi parmi les suivants : cancer du sein, cancer de la peau, cancer de l'os, cancer de la prostate, cancer du foie, cancer du poumon, cancer du poumon non à petites cellules, adénocarcinome pulmonaire non à petites cellules squameuses, cancer du cerveau, cancer du larynx, vésicule biliaire, pancréas, rectum, parathyroïdien, de la thyroïde, de la glande surrénale, du tissu neuronal, de la tête et du cou, du côlon, de l'estomac, des bronches et des reins, carcinome à cellules basales, carcinome à cellules squameuses de type ulcérant comme papillaire, carcinome métastatique de la peau, ostéosarcome, sarcome d'Ewing, sarcome des cellules du réticulum, myélome, tumeur à cellules géantes, tumeur pulmonaire à petites cellules, tumeur des cellules d'îlots, tumeur cérébrale primaire, tumeurs lymphocytaires et granulocytaires aiguës et chroniques, tumeur à tricholeucocytes, adénome, hyperplasie, carcinome médullaire, phéochromocytome, névrome muqueux, ganglioneuromes intestinaux, tumeur hyperplasique du nerf cornéen, tumeur du syndrome marfanoïde, tumeur de Wilm, séminome, tumeur ovarienne, cancer ovarien résistant au platine, tumeur léiomyomateuse, dysplasie du col de l'utérus et carcinome *in situ,* neuroblastome, rétinoblastome, sarcome des tissus mous, carcinoïde malin, mycosis fongoïde, rhabdomyosarcome, sarcome de Kaposi, sarcome ostéogène, hypercalcémie maligne, tumeur des cellules rénales, polyglobulie essentielle, adénocarcinome, glioblastome multiforme, leucémies, lymphomes, mélanomes, carcinomes épidermoïdes, carcinomes hépatocellulaires et tumeur solide.

23. Combinaison ou kit pour utilisation selon l'une quelconque des revendications 19 à 20, où la maladie associée à une synthèse anormale de l'hémoglobine est choisie parmi la drépanocytose et la β-thalassémie.

24. Combinaison ou kit pour utilisation selon l'une quelconque des revendications 19 à 20, où le SMD est choisi parmi les SMD à risque faible, intermédiaire et élevé et des néoplasies myéloprolifératives.

25. Combinaison selon l'une quelconque des revendications 1 à 11 ou kit selon l'une quelconque des revendications 12 à 15 pour utilisation dans un traitement prophylactique ou thérapeutique.

26. Combinaison ou kit pour utilisation selon l'une quelconque des revendications 19 à 24, comprenant l'administration d'un composé de formule (I) à un sujet selon une posologie d'une fois par jour pendant 5 jours, immédiatement suivie par deux jours sans administration, suivis par l'administration du ou des composants thérapeutiques ancillaires.

27. Combinaison ou kit pour utilisation selon la revendication 26, comprenant l'administration d'un composé de formule (I) à un sujet selon une posologie d'une fois par jour pendant 5 jours, immédiatement suivie par deux jours sans administration, suivis par l'administration d'un ou de plusieurs composants thérapeutiques ancillaires comprenant un CTLA-4 mAb.

28. Combinaison ou kit selon l'une quelconque des revendications 1 à 18, où le ou les composants thérapeutiques ancillaires comprennent en outre un adjuvant.

29. Combinaison ou kit selon la revendication 28, où ledit adjuvant est un ligand de récepteur de reconnaissance de pathogène (PRR).

30. Combinaison ou kit selon la revendication 28, où ledit adjuvant comprend un ligand TLR, préférentiellement un ou plusieurs des éléments du groupe constitué par TLR1, TLR2, TLR3, TLR4, TLR5, TLR6, TLR7, TLR8, TLR9, TLR10 et TLR11.
